(19) Europäisches Patentamt
European Patent Office
Office européen des brevets

(11) **EP 2 084 154 B1**

(12) **EUROPÄISCHE PATENTSCHRIFT**

(45) Veröffentlichungstag und Bekanntmachung des Hinweises auf die Patenterteilung:
**21.11.2012 Patentblatt 2012/47**

(21) Anmeldenummer: **07801837.1**

(22) Anmeldetag: **23.08.2007**

(51) Int Cl.:
***C07D 413/14*** *(2006.01)* ***A61P 7/02*** *(2006.01)*
***A61K 31/422*** *(2006.01)*

(86) Internationale Anmeldenummer:
**PCT/EP2007/007408**

(87) Internationale Veröffentlichungsnummer:
**WO 2008/022786 (28.02.2008 Gazette 2008/09)**

(54) **AMINOACYL-PRODRUG DERIVATE UND ARZNEIMITTEL ZUR BEHANDLUNG VON THROMBOEMBOLISCHEN ERKRANKUNGEN**

AMINOACYL PRODRUG DERIVATIVES AND MEDICAMENTS FOR TREATMENT OF THROMBOEMBOLIC DISORDERS

DÉRIVÉ PROMÉDICAMENT AMINOACYLÉ ET MÉDICAMENT POUR LE TRAITEMENT DE MALADIES THROMBOEMBOLIQUES

(84) Benannte Vertragsstaaten:
**AT BE BG CH CY CZ DE DK EE ES FI FR GB GR HU IE IS IT LI LT LU LV MC MT NL PL PT RO SE SI SK TR**
Benannte Erstreckungsstaaten:
**AL BA HR MK RS**

(30) Priorität: **24.08.2006 DE 102006039589**

(43) Veröffentlichungstag der Anmeldung:
**05.08.2009 Patentblatt 2009/32**

(73) Patentinhaber: **Bayer Intellectual Property GmbH**
**40789 Monheim (DE)**

(72) Erfinder:
- **LERCHEN, Hans-Georg**
  **51375 Leverkusen (DE)**
- **KRENZ, Ursula**
  **42799 Leichlingen (DE)**
- **SCHLEMMER, Karl-Heinz**
  **42113 Wuppertal (DE)**
- **PERZBORN, Elisabeth**
  **42327 Wuppertal (DE)**

(74) Vertreter: **Bayer Intellectual Property GmbH**
**Creative Campus Monheim**
**Alfred-Nobel-Straße 10**
**40789 Monheim (DE)**

(56) Entgegenhaltungen:
**WO-A-01/00622 WO-A-2005/028473**

- **ROEHRIG S ET AL: "Discovery of the Novel Antithrombotic Agent 5-Chloro-N-(((5S)-2-oxo-3 [4-(3-oxomorpholin-4-yl)phenyl]-1,3-oxazol idin-5-yl)methyl)thiophene 2-carboxamide (BAY 59-7939): An Oral, Direct Factor Xa Inhibitor" JOURNAL OF MEDICINAL CHEMISTRY, AMERICAN CHEMICAL SOCIETY. WASHINGTON, US, Bd. 48, 22. September 2005 (2005-09-22), Seiten 5900-5908, XP002418821 ISSN: 0022-2623 in der Anmeldung erwähnt**
- **KAHNS A H ET AL: "N ACYL DERIVATIVES AS PRODRUG FORMS FOR AMIDES CHEMICAL STABILITY AND ENZYMATIC HYDROLYSIS OF VARIOUS N ACYL AND N ALKOXYCARBONYL AMIDE DERIVATIVES" INTERNATIONAL JOURNAL OF PHARMACEUTICS (KIDLINGTON), Bd. 71, Nr. 1-2, 1991, Seiten 31-44, XP002440260 ISSN: 0378-5173**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).



Printed by Jouve, 75001 PARIS (FR)

**Beschreibung**

Die vorliegende Anmeldung betrifft Prodrug-Derivate von 5-Chlor-*N*-({(5*S*)-2-oxo-3-[4-(3-oxo-morpholin-4-yl) phenyl]-1,3-oxazolidin-5-yl}methyl)thiophen-2-carboxamid, Verfahren zu ihrer Herstellung, ihre Verwendung zur Behandlung und/oder Prophylaxe von Krankheiten sowie ihre Verwendung zur Herstellung von Arzneimitteln zur Behandlung und/oder Prophylaxe von Krankheiten, insbesondere von thromboembolischen Erkrankungen.

Prodrugs sind Derivate eines Wirkstoffs, die *in vivo* eine ein- oder mehrstufige Biotransformation enzymatischer und/oder chemischer Art durchlaufen, bevor der eigentliche Wirkstoff freigesetzt wird. Ein Prodrug-Rest wird in der Regel genutzt, um das Eigenschaftsprofil des zu Grunde liegenden Wirkstoffs zu verbessern [P. Ettmayer et al., J. Med. Chem. 47, 2393 (2004)]. Um ein optimales Wirkprofil zu erreichen, muss dabei das Design des Prodrug-Restes ebenso wie der angestrebte Freisetzungsmechanismus sehr genau auf den individuellen Wirkstoff, die Indikation, den Wirkort und die Applikationsroute abgestimmt werden. Eine große Zahl von Arzneimitteln wird als Prodrugs verabreicht, die gegenüber dem zu Grunde liegenden Wirkstoff eine verbesserte Bioverfügbarkeit aufweisen, beispielsweise erzielt durch eine Verbesserung des physikochemischen Profils, speziell der Löslichkeit, der aktiven oder passiven Absoiptionseigenschaften oder der gewebsspezifischen Verteilung. Aus der umfangreichen Literatur über Prodrugs sei beispielhaft genannt: H. Bundgaard (Ed.), Design of Prodrugs: Bioreversible derivatives for various functional groups and chemical entities, Elsevier Science Publishers B.V., 1985.

5-Chlor-*N*-{((5*S*)-2-oxo-3-[4-3-oxomorpholin-4-yl)phenyl]-1,3-oxazolidin-5-yl}methyl)thiophen-2-carboxamid [BAY 59-7939, Verbindung (A)] ist ein oral wirksamer, direkter Inhibitor der Serin-Protease Faktor Xa, welche eine essentielle Funktion bei der Regulation der Blutgerinnung ausübt. Die Verbindung befindet sich gegenwärtig in vertiefter klinischer Prüfung als möglicher neuer Arzneimittelwirkstoff für die Prävention und Therapie thromboembolischer Erkrankungen [S. Roehrig et al., J. Med. Chem. 48, 5900 (2005)].

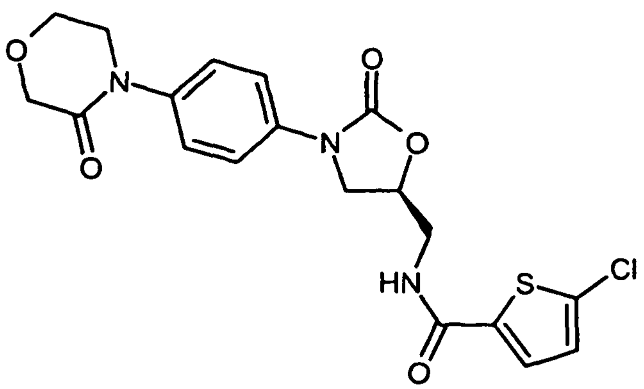

(A)

Verbindung (A) weist jedoch nur eine begrenzte Löslichkeit in Wasser und physiologischen Medien auf, was beispielsweise eine intravenöse Applikation des Wirkstoffs erschwert. Aufgabe der vorliegenden Erfindung war daher die Identifizierung von Derivaten oder Prodrugs von Verbindung (A), die eine verbesserte Löslichkeit in den genannten Medien besitzen und gleichzeitig nach Applikation eine kontrollierte Freisetzung des Wirkstoffs (A) im Körper des Patienten erlauben.

In WO 2005/028473 werden Acyloxymethylcarbamat-Prodrugs von Oxazolidinonen beschrieben, die einer Erhöhung der oralen Bioverfügbarkeit dienen. In WO 01/00622 werden Acyl-Prodrugs von Carbamat-Inhibitoren der Inosin-5'-monophosphat-Dehydrogenase offenbart. Eine weitere Art von Amid-Prodrugs für Oxazolidinone, die über einen mehrstufigen Aktivierungsmechanismus den zu Grunde liegenden Wirkstoff freisetzen, ist in WO 03/006440 beschrieben. In A. H. Kahns et al., Int. J. Pharmaceutics 71, 1991, 31-43 werden N-Acyl Derivate als Prodrug-Formen für Amide beschrieben.

Gegenstand der vorliegenden Erfindung sind Verbindungen der allgemeinen Formel (I)

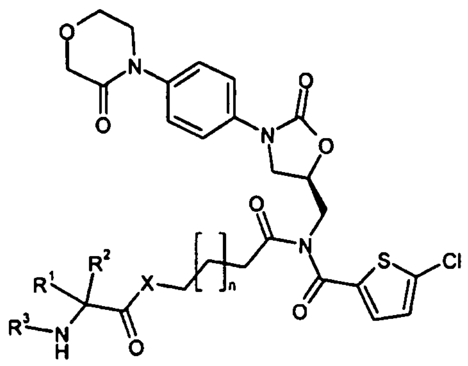

(I),

in welcher

n für die Zahl 1 oder 2 steht,

X für ein Sauerstoffatom, Schwefelatom oder NH steht,

$R^1$ für die Seitengruppe einer natürlichen $\alpha$-Aminosäure oder ihrer Homologe oder Isomere steht,

$R^2$ für Wasserstoff oder Methyl steht,

$R^3$ für Wasserstoff steht,

oder

$R^1$ und $R^3$ sind über eine $(CH_2)_3$- oder $(CH_2)_4$-Gruppe verknüpft und bilden zusammen mit dem Stickstoff- bzw. Kohlenstoffatom, an das sie gebunden sind einen 5- bzw. 6-Ring,

sowie ihre Salze, Solvate und Solvate der Salze.

Erfindungsgemäße Verbindungen sind die Verbindungen der Formel (I) und deren Salze, Solvate und Solvate der Salze, die von Formel (I) umfassten Verbindungen der nachfolgend genannten Formeln und deren Salze, Solvate und Solvate der Salze sowie die von Formel (I) umfassten, nachfolgend als Ausführungsbeispiele genannten Verbindungen und deren Salze, Solvate und Solvate der Salze, soweit es sich bei den von Formel (I) umfassten, nachfolgend genannten Verbindungen nicht bereits um Salze, Solvate und Solvate der Salze handelt.

Die erfindungsgemäßen Verbindungen können in Abhängigkeit von ihrer Struktur in stereoisomeren Formen (Enantiomere, Diastereomere) existieren. Die Erfindung umfasst deshalb die Enantiomeren oder Diastereomeren und ihre jeweiligen Mischungen. Aus solchen Mischungen von Enantiomeren und/oder Diastereomeren lassen sich die stereoisomer einheitlichen Bestandteile in bekannter Weise isolieren.

Sofern die erfindungsgemäßen Verbindungen in tautomeren Formen vorkommen können, umfasst die vorliegende Erfindung sämtliche tautomere Formen.

Als Salze sind im Rahmen der vorliegenden Erfindung physiologisch unbedenkliche Salze der erfindungsgemäßen Verbindungen bevorzugt. Umfasst sind auch Salze, die für pharmazeutische Anwendungen selbst nicht geeignet sind, jedoch beispielsweise für die Isolierung oder Reinigung der erfindungsgemäßen Verbindungen verwendet werden können.

Physiologisch unbedenkliche Salze der erfindungsgemäßen Verbindungen umfassen Säureadditionssalze von Mineralsäuren, Carbonsäuren und Sulfonsäuren, z.B. Salze der Chlorwasserstoffsäure, Bromwasserstoffsäure, Schwefelsäure, Phosphorsäure, Methansulfonsäure, Ethansulfonsäure, Toluolsulfonsäure, Benzolsulfonsäwe, Naphthalindisulfonsäure, Essigsäure, Trifluoressigsäure, Propionsäure, Milchsäure, Weinsäure, Äpfelsäure, Zitronensäure, Fumarsäure, Maleinsäure und Benzoesäure.

Als Solvate werden im Rahmen der Erfindung solche Formen der erfindungsgemäßen Verbindungen bezeichnet, welche in festem oder flüssigem Zustand durch Koordination mit Lösungsmittelmolekülen einen Komplex bilden. Hydrate sind eine spezielle Form der Solvate, bei denen die Koordination mit Wasser erfolgt. Als Solvate sind im Rahmen der vorliegenden Erfindung Hydrate bevorzugt.

Im Rahmen der vorliegenden Erfindung haben die Substituenten, soweit nicht anders spezifiziert, die folgende Bedeutung:

Die Seitengruppe einer $\alpha$-Aminosäure in der Bedeutung von $R^1$ umfasst sowohl die Seitengruppen der natürlich

vorkommenden α-Aminosäuren als auch die Seitengruppen von Homologen und Isomeren dieser α-Aminosäuren. Die α-Aminosäure kann hierbei sowohl in der L- als auch in der D-Konfiguration oder auch als Gemisch der L- und D-Form vorliegen. Als Seitengruppen seien beispielhaft genannt: Wasserstoff (Glycin), Methyl (Alanin), Propan-2-yl (Valin), Propan-1-yl (Norvalin), 2-Methylpropan-1-yl (Leucin), 1-Methylpropan-1-yl (Isoleucin), Butan-1-yl (Norleucin), Phenyl (2-Phenylglycin), Benzyl (Phenylalanin), p-Hydroxybenzyl (Tyrosin), Indol-3-ylmethyl (Tryptophan), Imidazol-4-ylmethyl (Histidin), Hydroxymethyl (Serin), 2-Hydroxyethyl (Homoserin), 1-Hydroxyethyl (Threonin), Mercaptomethyl (Cystein), Methylthiomethyl (S-Methylcystein), 2-Mercaptoethyl (Homocystein), 2-Methylthioethyl (Methionin), Carbamoylmethyl (Asparagin), 2-Carbamoylethyl (Glutamin), Carboxymethyl (Asparaginsäure), 2-Carboxyethyl (Glutaminsäure), 4-Aminobutan-1-yl (Lysin), 4-Amino-3-hydroxybutan-1-yl (Hydroxylysin), 3-Aminopropan-1-yl (Ornithin), 3-Guanidinopropan-1-yl (Arginin), 3-Ureidopropan-1-yl (Citrullin). Bevorzugte α-Aminosäure-Seitengruppen in der Bedeutung von $R^2$ sind Wasserstoff (Glycin), Methyl (Alanin), Propan-2-yl (Valin), Propan-1-yl (Norvalin), Imidazol-4-ylmethyl (Histidin), Hydroxymethyl (Serin), 1-Hydroxyethyl (Threonin), Carbamoylmethyl (Asparagin), 2-Carbamoylethyl (Glutamin), 4-Aminobutan-1-yl (Lysin), 3-Aminopropan-1-yl (Ornithin), 3-Guanidino-propan-1-yl (Arginin). Bevorzugt ist die L-Konfiguration.

Wenn Reste in den erfindungsgemäßen Verbindungen substituiert sind, können die Reste, soweit nicht anders spezifiziert, ein- oder mehrfach substituiert sein. Im Rahmen der vorliegenden Erfindung gilt, dass für alle Reste, die mehrfach auftreten, deren Bedeutung unabhängig voneinander ist. Eine Substitution mit einem oder zwei gleichen oder verschiedenen Substituenten ist bevorzugt. Ganz besonders bevorzugt ist die Substitution mit einem Substituenten.

Bevorzugt sind Verbindungen der Formel (I), in welcher

n für die Zahl 1 oder 2 steht,

X für ein Sauerstoffatom, Schwefelatom oder NH steht,

$R^1$ für Wasserstoff, Methyl, Propan-2-yl, Propan-1-yl, 2-Methylpropan-1-yl, Imidazol-4-yl-methyl, Hydroxymethyl, 1-Hydroxyethyl, Carboxymethyl, 2-Carboxyethyl, Carbamoylmethyl, 2-Carbamoylethyl, 4-Aminobutan-1-yl, 3-Aminopropan-1-yl, 3-Guanidinopropan-1-yl, Benzyl oder 4-Hydroxybenzyl steht,

$R^2$ für Wasserstoff oder Methyl steht,

$R^3$ für Wasserstoff steht,

oder

$R^1$ und $R^3$ sind über eine $(CH_2)_3$- oder $(CH_2)_4$-Gruppe verknüpft und bilden zusammen mit dem Stickstoff- bzw. Kohlenstoffatom, an das sie gebunden sind einen 5- bzw. 6-Ring,

sowie ihre Salze, Solvate und Solvate der Salze.

Bevorzugt sind auch Verbindungen der Formel (I), in welcher

n für die Zahl 1 oder 2 steht,

X für NH steht,

$R^1$ für Wasserstoff, Methyl, Propan-2-yl, 2-Methylpropan-1-yl, Imidazol-4-ylmethyl, Hydroxymethyl, 1-Hydroxyethyl, Carboxymethyl, 2-Carboxyethyl, Carbamoylmethyl, 2-Carbamoylethyl, 4-Aminobutan-1-yl, Benzyl oder 4-Hydroxybenzyl steht,

$R^2$ für Wasserstoff steht,

$R^3$ für Wasserstoff steht,

sowie ihre Salze, Solvate und Solvate der Salze.

Bevorzugt sind auch Verbindungen der Formel (I), in welcher n für die Zahl 2 steht.

Bevorzugt sind auch Verbindungen der Formel (I), in welcher **X** für NH steht.

Bevorzugt sind auch Verbindungen der Formel (I), in welcher $R^1$ für Wasserstoff, Methyl, Propan-2-yl, 2-Methylpropan-1-yl, Imidazol-4-ylmethyl, Hydroxymethyl, 1-Hydroxyethyl, Carboxymethyl, 2-Carboxyethyl, Carbamoylmethyl, 2-Carbamoylethyl, 4-Aminobutan-1-yl, 3-Guanidinopropan-1-yl, Benzyl oder 4-Hydroxybenzyl steht.

Bevorzugt sind auch Verbindungen der Formel (I), in welcher $R^1$ für Wasserstoff, Methyl, Propan-2-yl, 2-Methylpropan-1-yl, Imidazol-4-ylmethyl, Hydroxymethyl, 1-Hydroxyethyl, Carboxymethyl, 2-Carboxyethyl, Carbamoylmethyl, 2-Carbamoylethyl, 4-Aminobutan-1-yl, Benzyl oder 4-Hydroxybenzyl steht.

Bevorzugt sind auch Verbindungen der Formel (I), in welcher $R^2$ für Wasserstoff steht.

Bevorzugt sind auch Verbindungen der Formel (I), in welcher $R^3$ für Wasserstoff steht.

Weiterer Gegenstand der Erfindung ist ein Verfahren zur Herstellung der Verbindungen der Formel (I), dadurch gekennzeichnet, dass man entweder

[A] die Verbindung der Formel

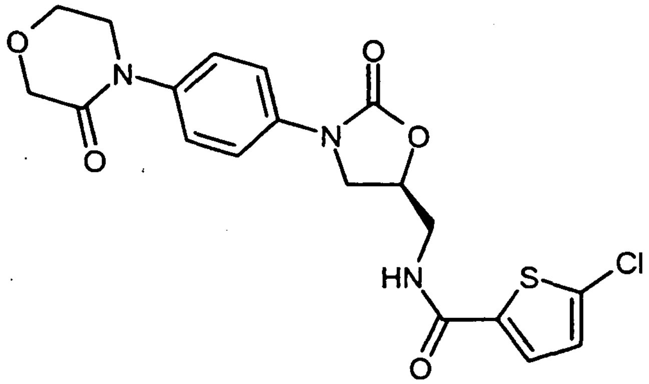

(A)

zunächst in einem inerten Lösungsmittel in Gegenwart einer Base mit einer Verbindung der Formel

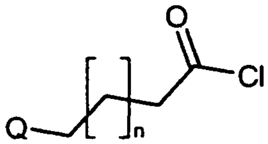

(II),

in welcher n die oben angegebene Bedeutung hat, und

Q für eine Abgangsgruppe wie beispielsweise Chlor, Brom oder Iod steht,

in eine Verbindung der Formel

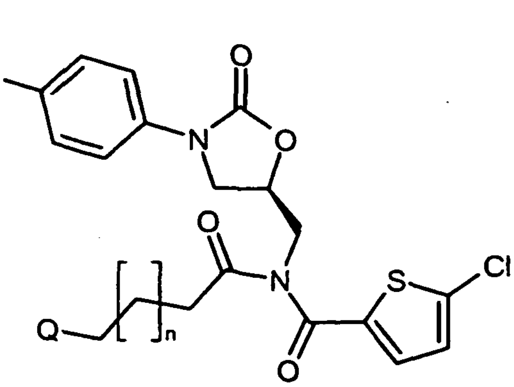

(III),

in welcher n und Q die oben angegebene Bedeutung haben,
überführt, diese dann nach Verfahren

[A1] in einem inerten Lösungsmittel mit dem Caesiumsalz einer $\alpha$-Aminocarbonsäure oder einer $\alpha$-Aminothiocarbonsäure der Formel

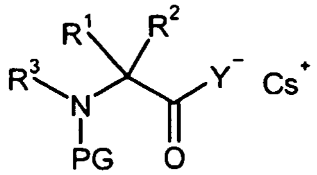

(IV),

in welcher $R^1$, $R^2$ und $R^3$ die oben angegebene Bedeutung haben,

PG für eine Amino-Schutzgruppe wie beispielsweise tert.-Butoxycarbonyl (Boc) oder Benzyloxycarbonyl (Z) steht

und

Y für O oder S steht,

zu einer Verbindung der Formel

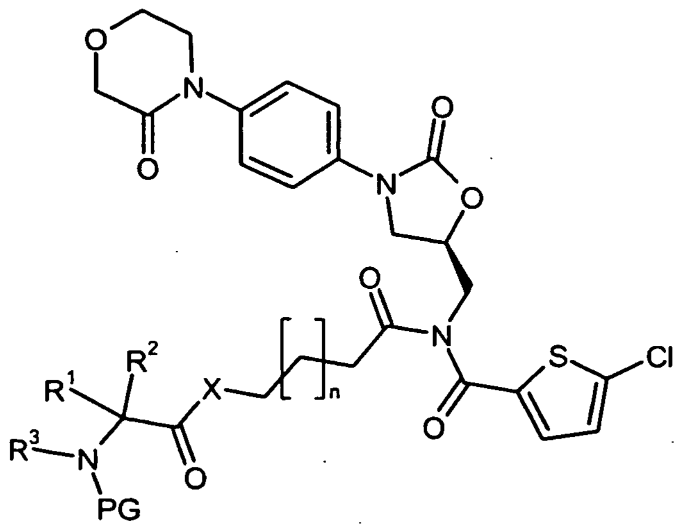

(V),

in welcher n, $R^1$, $R^2$, $R^3$ und PG die oben angegebene Bedeutung haben, und

X für O oder S steht,

umsetzt und nachfolgend die Schutzgruppe PG nach üblichen Methoden unter Erhalt einer Verbindung der Formel

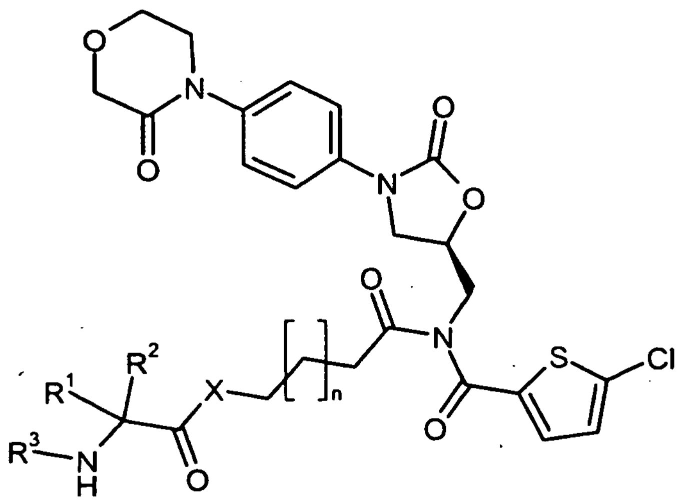

(I-A),

in welcher n, $R^1$, $R^2$ und $R^3$ die oben angegebene Bedeutung haben, und

X für O oder S steht,

entfernt
oder

[A2] in einem inerten Lösungsmittel in Gegenwart einer Base mit einer α-Aminothiocarbonsäure der Formel

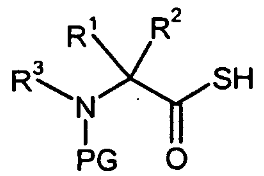

(VI),

in welcher $R^1$, $R^2$ und $R^3$ die oben angegebene Bedeutung haben,

PG für eine Amino-Schutzgruppe wie beispielsweise tert.-Butoxycarbonyl (Boc) oder Benzyloxycarbonyl (Z) steht,

zu einer Verbindung der Formel

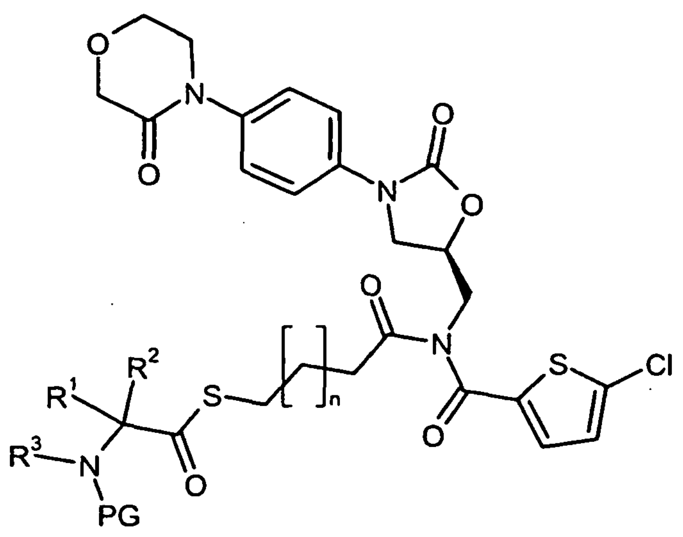

(V-A),

in welcher n, $R^1$, $R^2$, $R^3$ und PG die oben angegebene Bedeutung haben, umsetzt und nachfolgend die Schutzgruppe PG nach üblichen Methoden unter Erhalt einer Verbindung der Formel

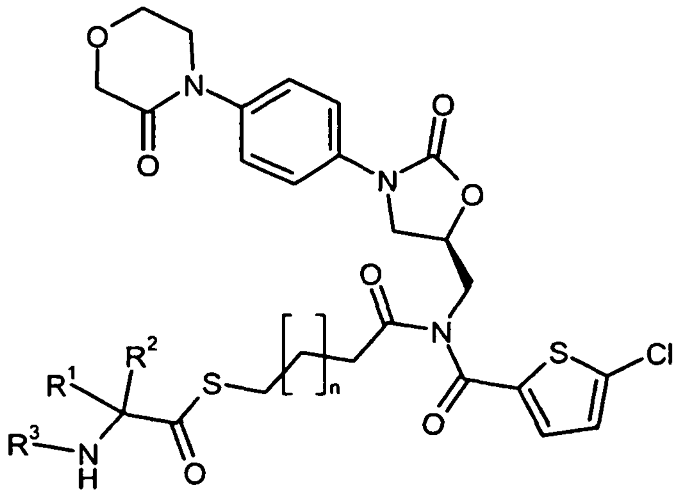

(I-A),

in welcher n, $R^1$, $R^2$ und $R^3$ die oben angegebene Bedeutung haben, entfernt
oder

[B] Verbindung (A) in einem inerten Lösungsmittel in Gegenwart einer Base mit einer Verbindung der Formel

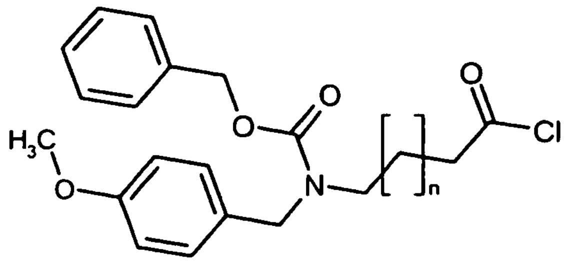

(VII),

in welcher n die oben angegebene Bedeutung hat,
zu einer Verbindung der Formel

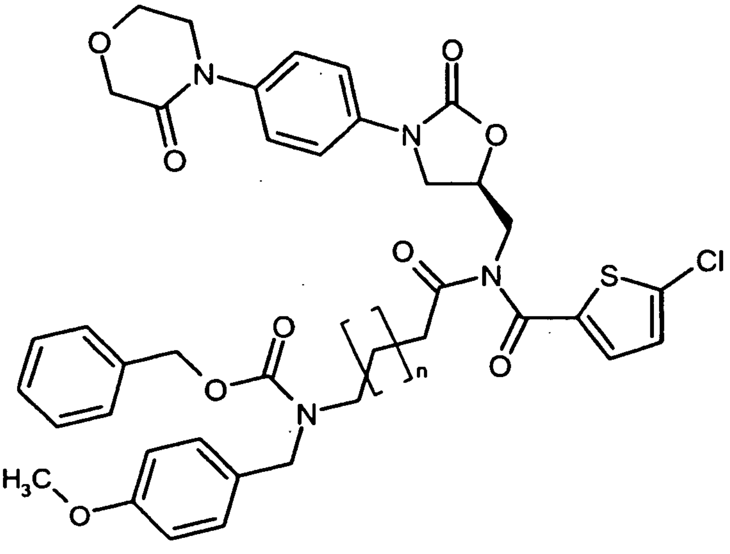

(VIII),

in welcher n die oben angegebene Bedeutung hat,

umsetzt, anschließend die Schutzgruppen nach üblichen Methoden unter Erhalt einer Verbindung der Formel

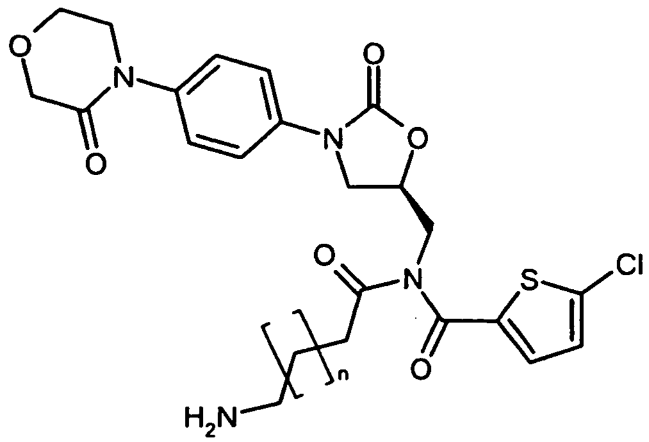

(IX),

in welcher n die oben angegebene Bedeutung hat, entfernt und dann in Gegenwart einer Base mit einer Verbindung der Formel

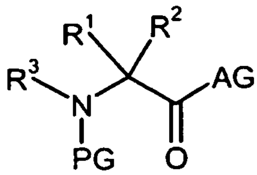

(X),

in welcher $R^1$, $R^2$ und $R^3$ die oben angegebene Bedeutung haben,

AG für Hydroxy oder Halogen, bevorzugt Chlor oder Brom, steht oder zusammen mit der Carbonylgruppe einen Aktivester, bevorzugt einen N-Hydoxysuccinimidester, oder ein gemischtes Anhydrid, bevorzugt einen Kohlensäurealkylester, besonders bevorzugt einen Kohlensäureethylester, bildet, und

PG für eine Amino-Schutzgruppe wie beispielsweise tert.-Butoxycarbonyl (Boc) oder Benzyloxycarbonyl (Z) steht,

unter Erhalt einer Verbindung der Formel

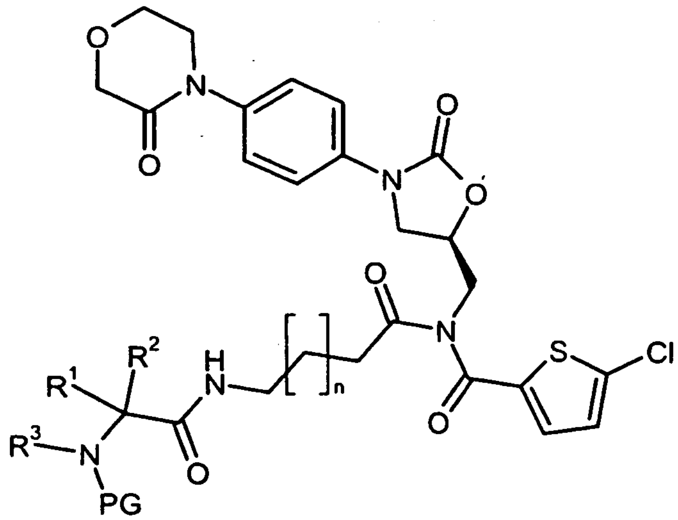

(XI)

in welcher n, $R^1$, $R^2$, $R^3$ und PG die oben angegebene Bedeutung haben, umsetzt,
und anschließend die Schutzgruppe PG nach üblichen Methoden unter Erhalt einer Verbindung der Formel

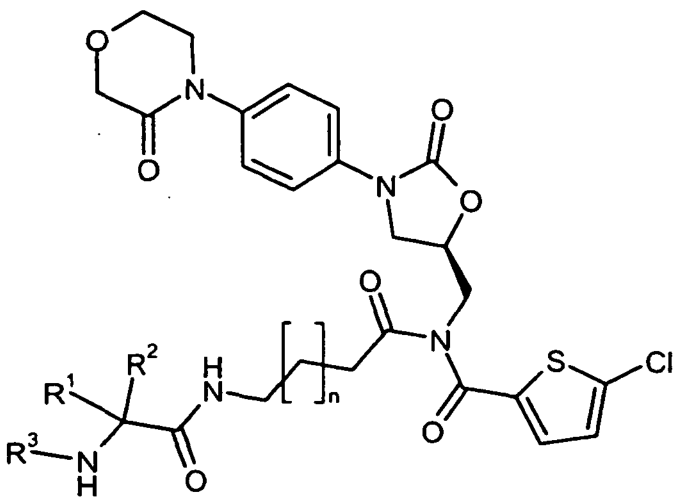

(I-B),

in welcher n, $R^1$, $R^2$ und $R^3$ die oben angegebene Bedeutung haben, entfernt und die jeweils resultierenden Verbindungen der Formel (I-A) bzw. (I-B) gegebenenfalls mit den entsprechenden (i) Lösungsmitteln und/oder (ii) Säuren in ihre Solvate, Salze und/oder Solvate der Salze überführt.

Die Verbindungen der Formel (I-A), (I-B) und (IX) können auch in Form ihrer Salze vorliegen. Diese Salze können gegebenenfalls mit den entsprechenden (i) Lösungsmitteln und/oder (ii) Basen in die freie Base überführt werden.

In dem Rest $R^1$ gegebenenfalls vorhandene funktionelle Gruppen können bei den zuvor beschriebenen Reaktionssequenzen, falls zweckmäßig oder erforderlich, auch in temporär geschützter Form vorliegen. Die Einführung und Entfernung solcher Schutzgruppen wie auch der Schutzgruppe PG erfolgt hierbei nach üblichen, aus der Peptidchemie bekannten Methoden [siehe z.B. T.W. Greene und P.G.M. Wuts, Protective Groups in Organic Synthesis, Wiley, New York, 1999; M. Bodanszky und A. Bodanszky, The Practice of Peptide Synthesis, Springer-Verlag, Berlin, 1984].

Solche in $R^1$ gegebenenfalls vorhandene Schutzgruppen können hierbei gleichzeitig mit der Abspaltung von PG oder in einem separaten Reaktionsschritt vor oder nach der Abspaltung von PG entfernt werden.

Als Amino-Schutzgruppe PG wird bei den obigen Verfahren bevorzugt tert.-Butoxycarbonyl (Boc) oder Benzyloxycarbonyl (Z) verwendet. Die Abspaltung dieser Schutzgruppen sowie die Abspaltung der Schutzgruppen in dem Verfahrensschritt (VIII) → (X) wird nach üblichen Methoden, vorzugsweise durch Reaktion mit einer starken Säure wie Chlorwasserstoff, Bromwasserstoff oder Trifluoressigsäure in einem inerten Lösungsmittel wie Dioxan, Dichlormethan oder Essigsäure durchgeführt.

Als inerte Lösungsmittel werden in den Verfahrensschritten (A) + (II) → (III) und (A) + (VII) → (VIII) vorzugsweise Tetrahydrofuran, *N,N*-Dimethylformamid oder Dimethylsulfoxid verwendet; besonders bevorzugt ist *N,N*-Dimethylformamid. Als Base ist bei diesen Reaktionen insbesondere Natriumhydrid geeignet. Die genannten Umsetzungen werden im Allgemeinen in einem Temperaturbereich von 0°C bis +40°C bei Normaldruck durchgeführt.

Als inerte Lösungsmittel werden in den Verfahrensschritten (III) + (VI) → (V-A) und (IX) + (X) → (XI) vorzugsweise Tetrahydrofuran, *N,N*-Dimethylformamid oder Dimethylsulfoxid verwendet; besonders bevorzugt ist *N,N*-Dimethylformamid. Als Base ist bei diesen Reaktionen insbesondere Ethyldiisopropylamin geeignet. Die genannten Umsetzungen werden im Allgemeinen in einem Temperaturbereich von 0°C bis +40°C bei Normaldruck durchgeführt.

Der Verfahrensschritt (IH) + (IV) → (V) erfolgt bevorzugt in *N,N*-Dimethylformamid als Lösungsmittel. Im Allgemeinen wird die Reaktion in einem Temperaturbereich von 0°C bis +50°C, bevorzugt bei +20°C bis +50°C, bei Normaldruck durchgeführt. Die Umsetzung kann auch vorteilhaft unter Ultraschall-Behandlung ausgerührt werden.

Die Verbindungen der Formeln (II), (IV), (VI), (VII) und (X) sind kommerziell erhältlich, literaturbekannt oder können nach literaturüblichen Verfahren hergestellt werden. Die Herstellung der Verbindungen (A) ist in S. Roehrig et al., J. Med. Chem. 48, 5900 (2005) beschrieben.

Die Herstellung der erfindungsgemäßen Verbindungen kann durch das folgende Syntheseschema veranschaulicht werden:

Schema

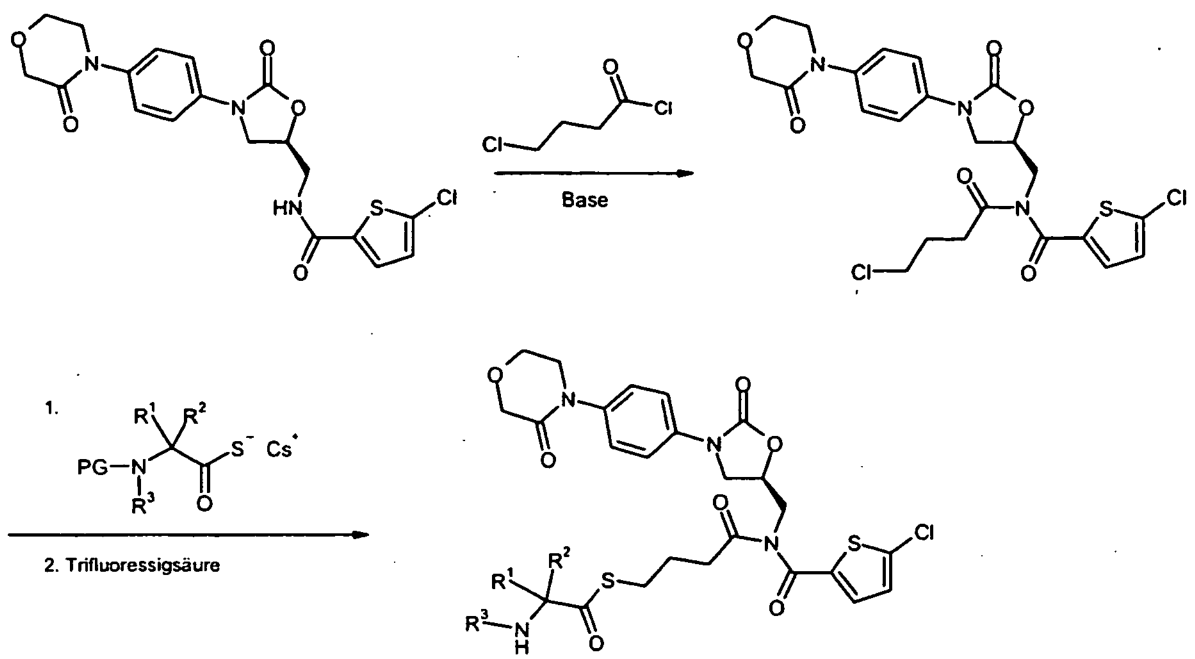


Die erfindungsgemäßen Verbindungen und ihre Salze stellen nützliche Prodrugs der Wirkstoff-Verbindung (A) dar. Sie weisen einerseits eine gute Stabilität zum Beispiel bei pH 4 auf und zeigen andererseits eine effiziente Konversion zur Wirkstoff-Verbindung (A) *in vivo*. Darüber hinaus besitzen die erfindungsgemäßen Verbindungen eine gute Löslichkeit in Wasser und anderen physiologisch verträglichen Medien, was sie für die therapeutische Anwendung insbesondere bei intravenöser Applikation geeignet macht.

Weiterer Gegenstand der vorliegenden Erfindung ist der Einsatz der erfindungsgemäßen Verbindungen zur Behandlung und/oder Prophylaxe von Erkrankungen, vorzugsweise von thromboembolischen Erkrankungen und/oder thromboembolischen Komplikationen.

Zu den "thromboembolischen Erkrankungen" im Sinne der vorliegenden Erfindung zählen insbesondere Erkrankungen wie Herzinfarkt mit ST-Segment-Erhöhung (STEMI) und ohne ST-Segment-Erhöhung (non-STEMI), stabile Angina Pectoris, instabile Angina Pectoris, Reokklusionen und Restenosen nach Koronarinterventionen wie Angioplastie oder aortokoronarem Bypass, periphere arterielle Verschlusskrankheiten, Lungenembolien, tiefe venöse Thrombosen und Nierenvenenthrombosen, transitorische ischämische Attacken sowie thrombotischer und thromboembolischer Hirnschlag.

Die Substanzen eignen sich daher auch zur Prävention und Behandlung von kardiogenen Thromboembolien, wie beispielsweise Hirn-Ischärnien, Schlaganfall und systemischen Thromboembolien und Ischämien, bei Patienten mit akuten, intermittierenden oder persistierenden Herzarrhythmien, wie beispielsweise Vorhofflimmern, und solchen, die sich einer Kardioversion unterziehen, ferner bei Patienten mit Herzklappen-Erkrankungen oder mit künstlichen Herzklappen. Darüber hinaus sind die erfindungsgemäßen Verbindungen zur Behandlung der disseminierten intravasalen Gerinnung (DIC) geeignet.

Thromboembolische Komplikationen treten ferner auf bei mikroangiopathischen hämolytischen Anämien, extrakorporalen Blutkreisläufen, wie Hämodialyse, sowie Herzklappenprothesen.

Außerdem kommen die erfindungsgemäßen Verbindungen auch für die Prophylaxe und/oder Behandlung von atherosklerotischen Gefäßerkrankungen und entzündlichen Erkrankungen wie rheumatische Erkrankungen des Bewegungsapparats in Betracht, darüber hinaus ebenso für die Prophylaxe und/oder Behandlung der Alzheimer'schen Erkrankung. Außerdem können die erfindungsgemäßen Verbindungen zur Inhibition des Tumorwachstums und der Metastasenbildung, bei Mikroangiopathien, altersbedingter Makula-Degeneration, diabetischer Retinopathie, diabetischer Nephropathie und anderen mikrovaskulären Erkrankungen sowie zur Prävention und Behandlung thromboembolischer Komplikationen, wie beispielsweise venöser Thromboembolien, bei Tumorpatienten, insbesondere solchen, die sich größeren chirurgischen Eingriffen oder einer Chemo- oder Radiotherapie unterziehen, eingesetzt werden.

Weiterer Gegenstand der vorliegenden Erfindung ist die Verwendung der erfindungsgemäßen Verbindungen zur Behandlung und/oder Prophylaxe von Erkrankungen, insbesondere der zuvor genannten Erkrankungen.

Weiterer Gegenstand der vorliegenden Erfindung ist die Verwendung der erfindungsgemäßen Verbindungen zur Herstellung eines Arzneimittels zur Behandlung und/oder Prophylaxe von Erkrankungen, insbesondere der zuvor

genannten Erkrankungen.

Weiterer Gegenstand der vorliegenden Erfindung ist ein Verfahren zur Behandlung und/oder Prophylaxe von Erkrankungen, insbesondere der zuvor genannten Erkrankungen, unter Verwendung der erfindungsgemäßen Verbindungen.

Weiterer Gegenstand der vorliegenden Erfindung sind Arzneimittel, enthaltend eine erfindungsgemäße Verbindung und einen oder mehrere weitere Wirkstoffe, insbesondere zur Behandlung und/oder Prophylaxe der zuvor genannten Erkrankungen. Als geeignete Kombinationswirkstoffe seien beispielhaft und vorzugsweise genannt:

- Lipidsenker, insbesondere HMG-CoA-(3-Hydroxy-3-methylglutaryl-Coenzym A)-Reduktase-Inhibitoren;
- Koronartherapeutika/Vasodilatatoren, insbesondere ACE-(Angiotensin-Converting-Enzyme)-Inhibitoren; AII-(Angiotensin II)-Rezeptor-Antagonisten; β-Adrenozeptor-Antagonisten; alpha-1-Adrenozeptor-Antagonisten; Diuretika; Calciumkanal-Blocker; Substanzen, die eine Erhöhung von cyclischem Guanosinmonophosphat (cGMP) bewirken, wie beispielsweise Stimulatoren der löslichen Guanylatcyclase;
- Plasminögen-Aktivatoren (Thrombolytika/Fibrinolytika) und die Thrombolyse/Fibrinolyse steigernde Verbindungen wie Inhibitoren des Plasminogen-Aktivator-Inhibitors (PAI-Inhibitoren) oder Inhibitoren des Thrombin-aktivierten Fibrinolyse-Inhibitors (TAFI-Inhibitoren);
- antikoagulatorisch wirksame Substanzen (Antikoagulantien);
- plättchenaggregationshemmende Substanzen (Plättchenaggregationshemmer, Thrombozytenaggregationshemmer);
- Fibrinogen-Rezeptor-Antagonisten (Glycoprotein-IIb/IIIa-Antagonisten);
- sowie Antiarrhythmika.

Weiterer Gegenstand der vorliegenden Erfindung sind Arzneimittel, die mindestens eine erfindungsgemäße Verbindung, üblicherweise zusammen mit einem oder mehreren inerten, nicht-toxischen, pharmazeutisch geeigneten Hilfsstoffen enthalten, sowie deren Verwendung zu den zuvor genannten Zwecken.

Die erfindungsgemäßen Verbindungen können systemisch und/oder lokal wirken. Zu diesem Zweck können sie auf geeignete Weise appliziert werden, wie z.B. oral, parenteral, pulmonal oder nasal. Für diese Applikationswege können die erfindungsgemäßen Verbindungen in geeigneten Applikationsformen verabreicht werden.

Für die orale Applikation eignen sich nach dem Stand der Technik funktionierende, die erfindungsgemäßen Verbindungen schnell und/oder modifiziert abgebende Applikationsformen, die die erfindungsgemäßen Verbindungen in kristalliner und/oder amorphisierter und/oder gelöster Form enthalten, wie z.B. Tabletten (nicht-überzogene oder überzogene Tabletten, beispielsweise mit magensaftresistenten oder sich verzögert auflösenden oder unlöslichen Überzügen, die die Freisetzung der erfindungsgemäßen Verbindung kontrollieren), in der Mundhöhle schnell zerfallende Tabletten oder Filme/Oblaten, Filme/Lyophylisate, Kapseln (beispielsweise Hart- oder Weichgelatinekapseln), Dragees, Granulate, Pellets, Pulver, Emulsionen, Suspensionen, Aerosole oder Lösungen.

Die parenterale Applikation kann unter Umgehung eines Resorptionsschrittes geschehen (z.B. intravenös, intraarteriell, intrakardial, intraspinal oder intralumbal) oder unter Einschaltung einer Resorption (z.B. intramuskulär, subcutan, intracutan, percutan oder intraperitoneal). Für die parenterale Applikation eignen sich als Applikationsformen u.a. Injektions- und Infusionszubereitungen in Form von Lösungen, Suspensionen, Emulsionen, Lyophilisaten oder sterilen Pulvern.

Für die sonstigen Applikationswege eignen sich z.B. inhalative Arzneiformen wie Pulverinhalatoren oder Nebulizer, oder nasal applizierbare Arzneiformen wie Tropfen, Lösungen oder Sprays.

Bevorzugt ist die parenterale Applikation, insbesondere die intravenöse Applikation.

Die erfindungsgemäßen Verbindungen können in die angeführten Applikationsformen überführt werden. Dies kann in an sich bekannter Weise durch Mischen mit inerten, nichttoxischen, pharmazeutisch geeigneten Hilfsstoffen geschehen. Zu diesen Hilfsstoffen zählen u.a. Trägerstoffe (beispielsweise mikrokristalline Cellulose, Lactose, Mannitol), Lösungsmittel (z.B. flüssige Polyethylenglycole), Emulgatoren und Dispergier- oder Netzmittel (beispielsweise Natriumdodecylsulfat, Polyoxysorbitanoleat), Bindemittel (beispielsweise Polyvinylpyrrolidon), synthetische und natürliche Polymere (beispielsweise Albumin), Stabilisatoren (z.B. Antioxidantien wie beispielsweise Ascorbinsäure), Farbstoffe (z.B. anorganische Pigmente wie beispielsweise Eisenoxide) und Geschmacks- und/oder Geruchskorrigentien.

Im Allgemeinen hat es sich als vorteilhaft erwiesen, bei parenteraler Applikation Mengen von etwa 0.001 bis 1 mg/kg, vorzugsweise etwa 0.01 bis 0.5 mg/kg Körpergewicht zur Erzielung wirksamer Ergebnisse zu verabreichen.

Bei oraler Applikation beträgt die Dosierung etwa 0.01 bis 100 mg/kg, vorzugsweise etwa 0.01 bis 20 mg/kg und ganz besonders bevorzugt 0.1 bis 10 mg/kg Körpergewicht.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Die nachfolgenden Ausführungsbeispiele erläutern die Erfindung. Die Erfindung ist nicht auf die Beispiele beschränkt.

Die Prozentangaben in den folgenden Tests und Beispielen sind, sofern nicht anders angegeben, Gewichtsprozente; Teile sind Gewichtsteile. Lösungsmittelverhältnisse, Verdünnungsverhältnisse und Konzentrationsangaben von flüssig/flüssig-Lösungen beziehen sich jeweils auf das Volumen.

**A. <u>Beispiele</u>**

**<u>Abkürzungen und Akronyme:</u>**

| | |
|---|---|
| abs. | absolut |
| Boc | *tert*.-Butoxycarbonyl |
| DMF | *N,N*-Dimethylformamid |
| DMSO | Dimethylsulfoxid |
| d. Th. | der Theorie (bei Ausbeute) |
| h | Stunde(n) |
| HPLC | Hochdruck-, Hochleistungsflüssigchromatographie |
| LC-MS | Flüssigchromatographie-gekoppelte Massenspektrometrie |
| min | Minute(n) |
| MS | Massenspektrometrie |
| NMR | Kernresonanzspektrometrie |
| Pd/C | Palladium auf Aktivkohle |
| quant. | quantitativ (bei Ausbeute) |
| RT | Raumtemperatur |
| $R_t$ | Retentionszeit (bei HPLC) |
| UM | Ultraviolett-Spektrometrie |
| v/v | Volumen-zu-Volumen-Verhältnis (einer Lösung) |
| Z | Benzyloxycarbonyl |

**<u>LC-MS- und HPLC-Methoden:</u>**

<u>Methode la (präparative HPLC</u> Säule: VP 250/21 Nukleodw 100-5 C18 ec, Macherey & Nagel Nr. 762002; Eluent A: Wasser / 0.01% Trifluoressigsäure, Eluent B: Acetonitril / 0.01 % Trifluoressigsäure; Gradient: 0 min 0% B → 20 min 20% B → 40 min 20% B → 60 min 30% B → 80 min 30% B → 90 min 100% B → 132 min 100% B; Fluss: 5 ml/min; Temperatur: RT; UV-Detektion: 210 nm.

<u>Methode 1b (präparative HPLC)</u>: Säule: SymmetryPrep ™ C18 7μM; 19 x 300 mm; Waters; Eluent A: Wasser / 0.01% Trifluoressigsäure, Eluent B: Acetonitril / 0.01% Trifluoressigsäure; Gradient: 0 min 0% B → 20 min 20% B → 40 min 20% B → 60 min 30% B → 80 min 30% B → 90 min 100% B → 132 min 100% B; Fluss: 5 ml/min; Temperatur: RT; UV-Detektion: 210 nm.

<u>Methode 2 (analytische HPLC):</u> Säule: XTerra 3.9 x 150 WAT 186000478; Eluent A: 10 ml 70%-ige Perchlorsäure in 2.5 Liter Wasser, Eluent B: Acetonitril; Gradient: 0.0 min 20% B → 1 min 20% B → 4 min 90% B → 9 min 90% B; Temperatur: RT; Fluss: 1 ml/min. In der Variante Methode 2a wird die Säule bei einer Temperatur von 40°C eluiert.

<u>Methode 3 (LC-MS)</u>: Instrument: Micromass ZQ; Gerätetyp HPLC: HP 1100 Series; UV DAD; Säule: Phenomenex Gemini 3μ 30 mm x 3.00 mm; Eluent A: 1 1 Wasser + 0.5 ml 50%ige Ameisensäure, Eluent B: 1 1 Acetonitril + 0.5 ml 50%ige Ameisensäure; Gradient: 0.0 min 90%A → 2.5 min 30%A → 3.0 min 5%A → 4.5 min 5%A; Fluss: 0.0 min 1 ml/min, 2.5 min/3.0 min/4.5 min. 2 ml/min; Temperatur: 50°C; UV-Detektion: 210 nm.

<u>Methode 4 (LC-MS)</u>: Instrument: Micromass ZQ mit HPLC HP 1100 Series; UV DAD; Säule: Phenomenex Synergi 2μ Hydro-RP Mercury 20 mm x 4 mm; Eluent A: 1 1 Wasser + 0.5 ml 50%-ige Ameisensäure, Eluent B: 1 1

Acetonitril + 0.5 ml 50%-ige Ameisensäure; Gradient: 0.0 min 90% A → 2.5 min 30% A → 3.0 min 5% A → 4.5 min 5% A; Fluss: 0.0 min 1 ml/min → 2.5 min/3.0 min/4.5 min 2 ml/min; Temperatur: 50°C; UV-Detektion: 210 nm.

Methode 5 (LC-MS): Instrument: Micromass Quattro LCZ mit HPLC Agilent Series 1100; Säule: Phenomenex Synergi 2μ Hydro-RP Mercury 20 mm x 4 mm; Eluent A: 1 1 Wasser + 0.5 ml 50%-ige Ameisensäure, Eluent B: 1 1 Acetonitril + 0.5 ml 50%-ige Ameisensäure; Gradient: 0.0 min 90% A → 2.5 min 30% A → 3.0 min 5% A → 4.5 min 5% A; Fluss: 0.0 min 1 ml/min → 2.5 min/3.0 min/4.5 min 2 ml/min; Temperatur: 50°C; UV-Detektion: 208-400 nm.

Methode 6 (LC-MS): Gerätetyp MS: Micromass ZQ; Gerätetyp HPLC: Waters Alliance 2795; Säule: Phenomenex Synergi 2μ Hydro-RP Mercury 20 mm x 4 mm; Eluent A: 1 1 Wasser + 0.5 ml 50%-ige Ameisensäure, Eluent B: 1 1 Acetonitril + 0.5 ml 50%-ige Ameisensäure; Gradient: 0.0 min 90% A → 2.5 min 30% A → 3.0 min 5% A → 4.5 min 5% A; Fluss: 0.0 min 1 ml/min → 2.5 min/3.0 min/4.5 min 2 ml/min; Temperatur: 50°C; UV-Detektion: 210 nm.

Methode 7 (chirale HPLC, analytisch): Chirale Silicagel-Phase (250 mm x 4.6 mm) basierend auf Poly(*N*-methacryloyl-L-leucin-dicyclopropylmethylamid); Eluent: Isohexan/ Essigsäure-ethylester 35:65 (v/v); Temperatur: 24°C; Fluss: 2 ml/min; UV-Detektion: 270 nm.

Methode 8 (chirale HPLC, analytisch): Chirale Silicagel-Phase (250 mm x 4.6 mm) basierend auf Poly(*N*-methacryloyl-L-leucin-*tert*.-butylamid); Eluent: Isohexan/Essigsäweethylester 35:65 (v/v); Temperatur: 24°C; Fluss: 2 ml/min; UV-Detektion: 270 nm.

Methode 9 (chirale HPLC, analytisch): Chirale Silicagel-Phase (250 mm x 4.6 mm) basierend auf Poly(N-methacryloyl-L-leucin-*tert*.-butylamid); Eluent: Isohexan/Essigsäureethylester 65:35 (v/v); Temperatur: 24°C; Fluss: 2 ml/min; UV-Detektion: 270 nm.

Methode 10 (chirale HPLC, präparativ): Chirale Silicagel-Phase (670 mm x 40 mm) basierend auf Poly(*N*-methacryloyl-L-leucin-dicyclopropylmethylamid); Eluent: Isohexan/Essigsäureethylester 25:75 (v/v); Temperatur: 24°C; Fluss: 80 ml/min; UV-Detektion: 270 nm.

Methode 11 (chirale HPLC, präparativ): Chirale Silicagel-Phase (670 mm x 40 mm) basierend auf Poly(N-methacryloyl-L-leucin-*tert*.-butylamid); Eluent: Isohexan/Essigsäureethylester 65:35 (v/v); Temperatur: 24°C; Fluss: 50 ml/min; UV-Detektion: 260 nm.

Methode 12 (LC-MS): Instrument: Micromass Quattro LCZ mit HPLC Agilent Serie 1100; Säule: Phenomenex Onyx Monolithic C18, 100 mm x 3 mm. Eluent A: 1 1 Wasser + 0.5 ml 50%ige Ameisensäure, Eluent B: 1 1 Acetonitril + 0.5 ml 50%ige Ameisensäure; Gradient: 0.0 min 90%A → 2 min 65%A → 4.5 min 5%A → 6 min 5%A; Fluss: 2 ml/min; Ofen: 40°C; UV-Detektion: 208-400 nm.

Methode 13 (LC-MS): Instrument: Micromass Platform LCZ mit HPLC Agilent Serie 1100; Säule: Thermo Hypersil GOLD 3μ 20 mm x 4 mm; Eluent A: 1 1 Wasser + 0.5 ml 50%ige Ameisensäure, Eluent B: 1 1 Acetonitril + 0.5 ml 50%ige Ameisensäure; Gradient: 0.0 min 100%A → 0.2 min 100%A → 2.9 min 30%A → 3.1 min 10%A → 5.5 min 10%A; Ofen: 50°C; Fluss: 0.8 ml/min; UV-Detektion: 210 nm.

Methode 14 (LC-MS): Gerätetyp MS: Micromass ZQ; Gerätetyp HPLC: Waters Alliance 2795; Säule: Merck Chromolith SpeedROD RP-18e 100 mm x 4.6 mm; Eluent A: 1 1 Wasser + 0.5 ml 50%ige Ameisensäure; Eluent B: 1 1 Acetonitril + 0.5 ml 50%ige Ameisensäure; Gradient: 0.0 min 10%B → 7.0 min 95%B → 9.0 min 95%B; Ofen: 35°C; Fluss: 0.0 min 1.0 ml/min → 7.0 min 2.0 ml/min → 9.0 min 2.0 ml/min; UV-Detektion: 210 nm.

**NMR-Spektrometrie:**

NMR-Messungen werden bei einer Protonenfrequenz von 400.13 MHz oder 500.13 MHz durchgeführt. Die Proben wurden üblicherweise in DMSO-$d_6$ gelöst; Temperatur: 302 K.

**Ausgangsverbindungen:**

Als Ausgangsmaterial wird 5-Chlor-*N*-({(5*S*)-2-oxo-3-[4-(3-oxomorpholin-4-yl)phenyl]-1;3-oxa-zolidin-5-yl}methyl)thiophen-2-carboxamid [Verbindung (A)] verwendet, dessen Herstellung an anderer Stelle beschrieben ist [S. Roechrig et al., J. Med. Chem 48, 5900 (2005)]

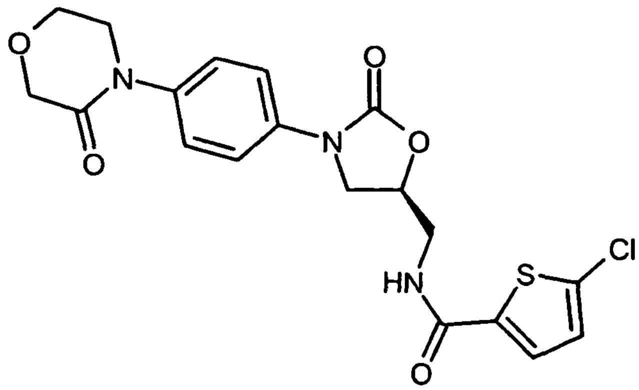

(A)

**Beispiel 1A**

5-Chlor-*N*-(4-chlorbutanoyl)-*N*-({(5*S*)-2-oxo-3-[4-(3-oxomorpholin-4-yl)phenyl]-1,3-oxazolidin-5-yl} methyl)thiophen-2-carboxamid

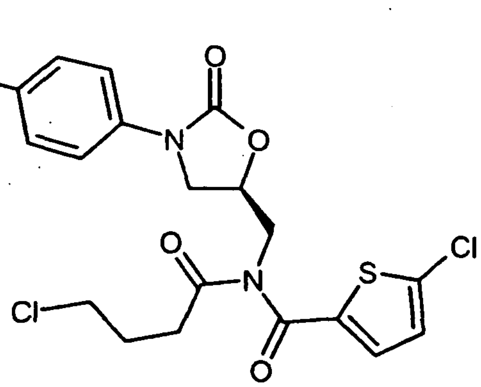

1 g (2.3 mmol) 5-Chlor-*N*-({(5*S*)-2-oxo-3-[4-(3-oxomorpholin-4-yl)phenyl]-1,3-oxazolidin-5-yl}-methyl)thiophen-2-carboxamid [Verbindung (A)] wird unter Argon in 100 ml abs. DMF gelöst. Man setzt 110 mg (4.6 mmol) Natriumhydrid (98%ig) zu und lässt 20 min bei RT rühren. Dann werden 4.37 g (30.97 mmol) Chlorbutanoylchlorid zugesetzt, wobei die Reaktionstemperatur auf RT gehalten wird. Man rührt 16 h bei RT und setzt dann unter Kühlung langsam 25 ml Wasser zu. Anschließend werden 300 ml Essigsäureethylester zugesetzt und weitere 50 ml Wasser. Die Phasen werden getrennt und die Essigsäureethylesterphase im Vakuum eingeengt. Der Rückstand wird mit Essigsäureethylester verrührt und filtriert. Die Mutterlauge wird eingeengt und der Rückstand durch Flash-Chromatographie an Kieselgel mit Toluol/Ethanol 5:1 als Eluent gereinigt.

Die entsprechenden Fraktionen, die die Zielverbindung enthalten, sowie diejenigen, die eine nach Enolisierung entstehende bis-acylierte Verbindung enthalten, werden vereinigt und das Lösungsmittel entfernt. Der Rückstand wird mit einer gesättigten Lösung aus Chlorwasserstoff in Dichlormethan versetzt und über Nacht bei RT gerührt. Dann wird im Vakuum eingeengt und der Rückstand erneut durch Flash-Chromatographie an Kieselgel mit Toluol/Ethanol 6:1 als Eluent gereinigt. Die entsprechenden Fraktionen werden eingeengt und der Rückstand aus Dioxan lyophilisiert. Dabei werden 94 mg (7.5 % d. Th.) der Zielverbindung erhalten.
HPLC (Methode 2): $R_t$ = 5.23 min;
LC-MS (Methode 6): $R_t$ = 2.13 min; m/z = 540 $(M+H)^+$.

**Beispiel 2A**

5-Chlor-*N*-(4-chlorpentanoyl)-*N*-({(5*S*)-2-oxo-3-[4-(3-oxomorpholin-4-yl)phenyl]-1,3-oxazolidin-5-yl}methyl)thiophen-2-carboxamid

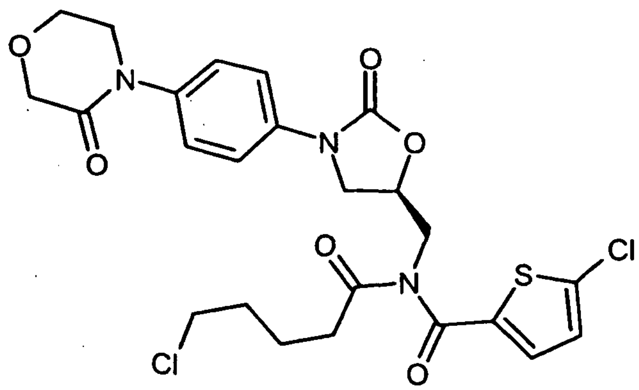

Die Herstellung erfolgt in Analogie zu Beispiel 1A ausgehend von 3 g (6.88 mmol) der Verbindung (A) und 5-Chlor-pentanoylchlorid. Man erhält 1008 mg (26% d. Th.) der Zielverbindung.
HPLC (Methode 2): $R_t$ = 5.35 min;
LC-MS (Methode 6): $R_t$ = 2.22 min; m/z = 554 $(M+H)^+$.

**Beispiel 3A**

N-(4-Aminobutanoyl)-5-chlor-N-({(5S)-2-oxo-3-[4-(3-oxomorpholin-4-yl)phenyl]-1,3-oxazolidin-5-yl}methyl)thiophen-2-carboxamid-Hydrochlorid

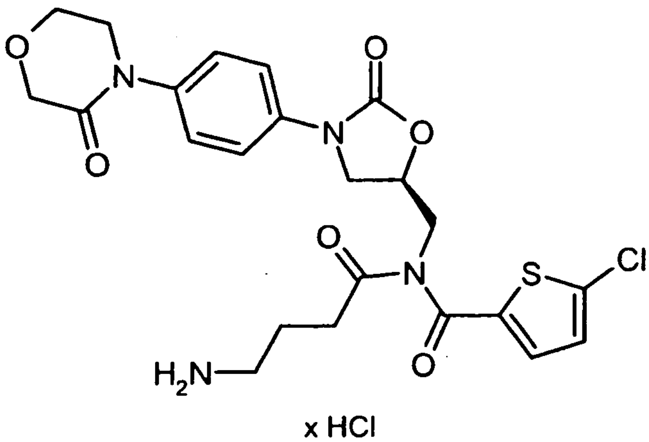

*Stufe a):*

1.33 g (3.06 mmol) der Verbindung (A) werden in 75 ml abs. DMF gelöst, mit 220 mg (9.2 mmol) Natriumhydrid (98%ig) versetzt, und die Mischung 30 min bei RT gerührt. Dann gibt man 11.5 g (30.6 mmol) des Beispiels 5A, gelöst in 10 ml abs. DMF, hinzu. Man rührt weitere 15 min bei RT und versetzt den Ansatz dann mit 20 ml Wasser. Danach wird eingeengt und der Rückstand in 300 ml Essigsäureethylester aufgenommen. Man schüttelt dreimal mit 300 ml einer 10%-igen Natriumcarbonat-Lösung aus. Die organische Phase wird abgetrennt, eingeengt und in 50 ml Dichlormethan aufgenommen. Man setzt dann 25 ml Diethylether zu. Nach kurzem Verrühren werden ungelöste Rückstände abfiltriert und die Dichlormethan-Phase eingeengt. Der Rückstand wird durch Flash-Chromatographie an Kieselgel mit Essigsäureethylester/Toluol 5:1 als Eluent gereinigt. Die entsprechenden Fraktionen, die ein bis-acyliertes Nebenprodukt mit der Masse M=1113, das nach Enolisierung der Mono-Acylverbindung entsteht, enthalten, werden eingeengt. Anschließend wird der Rückstand 2 h lang mit 10 ml einer gesättigten Lösung von Chlorwasserstoff in Dichlormethan verrührt, wobei der initial entstandene Enolester gespalten wird. Anschließend wird eingeengt und der verbleibende Rückstand durch Flash-Chromatographie an Kieselgel mit Essigsäureethylester/Toluol 5:1 als Eluent gereinigt. Die entsprechenden Fraktionen werden eingeengt und man erhält 151 mg (7% d. Th.) der an der Aminogruppe voll geschützten Verbindung.
HPLC (Methode 2): $R_t$ = 5.83 min.
LC-MS (Methode 6): $R_t$ = 2.61 min; m/z = 775 $(M+H)^+$.

*Stufe b):*

151 mg (0.2 mmol) dieser geschützten Verbindung werden in 8 ml wasserfreier Trifluoressigsäure über Nacht bei RT gerührt. Der Ansatz wird im Hochvakuum eingeengt, wobei die Temperatur auf etwa 20°C gehalten wird. Der Rückstand wird in 100 ml auf pH 3 eingestellter Salzsäure aufgenommen und mit 75 ml Dichlormethan und anschließend zweimal mit Essigsäureethylester ausgeschüttelt. Die wässrige Phase wird eingeengt und der Rückstand aus Salzsäure pH 3 lyophilisiert. Man erhält 70 mg (64% d. Th.) der Zielverbindung.
HPLC (Methode 2): $R_t$ = 4.13 min;
LC-MS (Methode 5): $R_t$ = 1.38 min; m/z = 521 $(M+H)^+$.

**Beispiel 4A**

N-(5-Aminopentanoyl)-5-chlor-N-({(5S)-2-oxo-3-[4-(3-oxomorpholin-4-yl)phenyl]-1,3-oxazolidin-5-yl}methyl)thiophen-2-carboxamid-Hydrochlorid

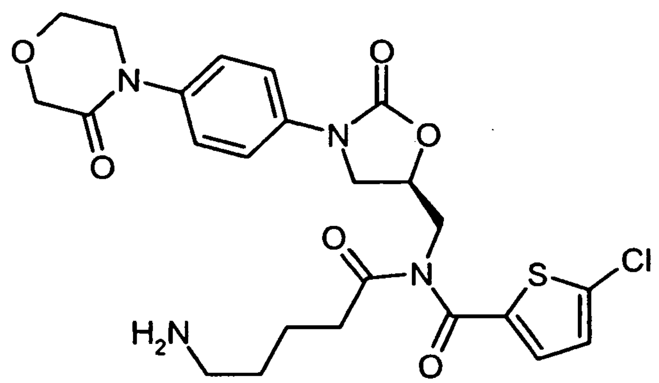

x HCl

*Stufe a):*

2.83 g (6.5 mmol) der Verbindung (A) werden unter Argon in 100 ml abs. DMF gelöst, mit 468 mg (19.5 mmol) Natriumhydrid versetzt, und die Mischung 30 min bei RT gerührt. Dann gibt man 7.6 g (19.5 mmol) des Beispiels 10A, gelöst in 10 ml DMF, hinzu. Man rührt weitere 15 min bei RT und versetzt den Ansatz dann langsam mit 20 ml Wasser. Danach wird eingeengt und der Rückstand 1 h lang mit 150 ml einer gesättigten Lösung von Chlorwasserstoff in Dichlormethan verrührt, wobei die initial nach Enolisierung entstandene Bis-Acylverbindung mit der Masse M=1141 gespalten wird. Anschließend wird eingeengt und der Rückstand in 700 ml Essigsäureethylester aufgenommen. Man schüttelt zweimal mit je 200 ml einer 10%-igen Natriumcarbonat-Lösung aus. Die organische Phase wird abgetrennt, eingeengt und in 30 ml Essigsäureethylester aufgenommen und dann mit 30 ml Diethylether versetzt. Nach kurzem Verrühren werden ungelöste Rückstände abfiltriert und die organische Phase eingeengt. Der Rückstand wird durch Flash-Chromatogaphie an Kieselgel mit Essigsäweethylester/Toluol 4:1 als Eluent gereinigt. Die entsprechenden Fraktionen werden eingeengt und der Rückstand in 10 ml Essigsäureethylester aufgenommen. Man gibt 100 ml kalten Diethylether hinzu und lässt 30 min bei 0°C stehen. Man filtriert ab und behandelt den Rückstand nochmals mit 100 ml Diethylether. Nach erneutem Abfiltrieren wird der Filterrückstand gesammelt und getrocknet. Man erhält 1 g (20% d. Th.) der an der Aminogruppe voll geschützten Verbindung.
HPLC (Methode 2): $R_t$ = 5.92 min.

LC-MS (Methode 6): $R_t$ = 2.68 min; m/z = 789 $(M+H)^+$.

*Stufe b):*

1 g (1.3 mmol) dieser geschützten Verbindung werden in 70 ml wasserfreier Trifluoressigsäure 6 h lang im Ultraschallbad behandelt. Der Ansatz wird im Hochvakuum eingeengt, wobei die Temperatur auf etwa 20°C gehalten wird. Der Rückstand wird in 350 ml auf pH eingestellter Salzsäure aufgenommen und nach 15 Minuten Verrühren bei RT mit 100 ml Dichlormethan ausgeschüttelt. Anschließend wird mit 100 ml Essigsäureethylester ausgeschüttelt. Die wässrige Phase wird abgetrennt, dann zur Entfernung restlichen Essigsäweethylesters kurz im Hochvakuum andestilliert und schließlich lyophilisiert. Man erhält 586 mg (81% d. Th.) der Zielverbindung.
HPLC (Methode 2): $R_t$ = 4.2 min;
LC-MS (Methode 6): $R_t$ = 1.17 min; m/z = 535 $(M+H)^+$.

**Beispiel 5A**

Benzyl-(4-chlor-4-oxobutyl)(4-methoxybenzyl)carbamat

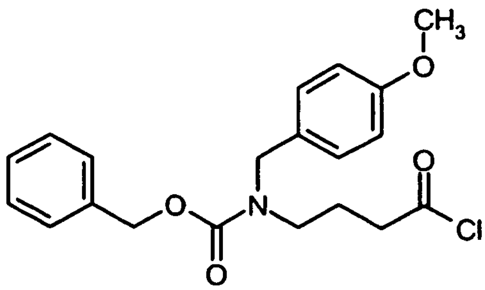

Die Herstellung erfolgt in Analogie zu Beispiel 10A ausgehend von 4-Amino-buttersäure.

**Beispiel 6A**

(2S)-2-[(tert.-Butoxycarbonyl)amino]-3-methylbutanthio-S-säure

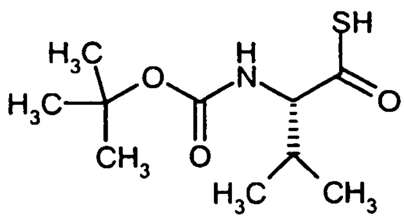

Die Titelverbindung wird aus Boc-Valin analog literaturbekannter Vorschrift [R. Michelot et al., Bioorg. Med. Chem. 1996, 4, 2201) hergestellt.

**Beispiel 7A**

[(tert-Butoxycarbonyl)amino]ethanthio S-säure

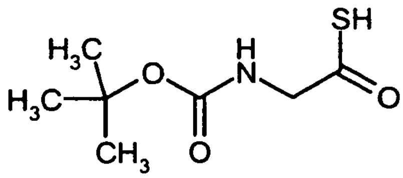

Die Titelverbindung wird aus Boc-Glycin analog literaturbekannter Vorschrift [R. Michelot et al., Bioorg. Med. Chem. 1996, 4, 2201) hergestellt.

**Beispiel 8A**

(2S)-2,6-Bis[(tert-butoxycarbonyl)amino)hexanthio S-säure

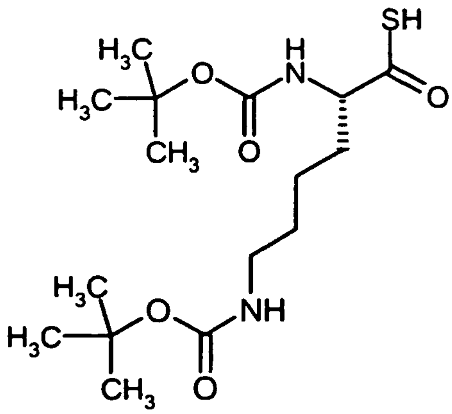

Die Titelverbindung wird aus Bis-Boc-Lysin analog literaturbekannter Vorschrift [R. Michelot et al., Bioorg. Med. Chem. 1996, 4, 2201) hergestellt.

**Beispiel 9A**

(2S)-2-[(tert-Butoxycarbonyl)amino]propanthio S-säure

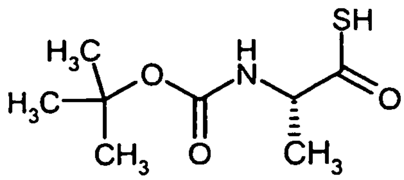

Die Titelverbindung wird aus Boc-Alanin analog literaturbekannter Vorschrift [R. Michelot et al., Bioorg. Med. Chem. 1996, 4, 2201) hergestellt.

**Beispiel 10A**

Benzyl-(5-chlor-5-oxopentyl)(4-methoxybenzyl)carbamat

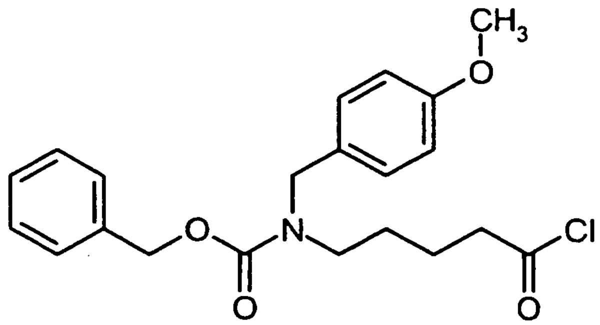

10 g (85.4 mmol) 5-Amino-valeriansäure, 17.4 g (128 mmol) p-Anisaldehyd und 10.3 g (85.4 mmol) Magnesiumsulfat werden in 330 ml Ethanol aufgenommen und 1 h unter Rückfluss erhitzt. Man filtriert ab, wäscht mit Ethanol nach und versetzt anschließend die Lösung portionsweise innerhalb von 15 min mit 1.94 g (51.2 mmol) Natriumborhydrid. Man setzt zunächst 10 ml Wasser zu und dann 128 ml einer 2M Natronlauge. Nach 5 min wird mit 300 ml Wasser verdünnt und anschließend dreimal mit je 200 ml Essigsäureethylester ausgeschüttelt. Die wässrige Phase wird mit 4M Salzsäure auf pH 2 eingestellt und im Vakuum eingeengt. Der Rückstand wird durch Flash-Chromatographie an Kieselgel mit Acetonitril/Wasser/Essigsäure 5:1:0.1 als Eluent gereinigt. Die entsprechenden Fraktionen werden eingeengt und mit Essigsäureethylester und Diethylether verrührt. Der Rückstand wird dann abgesaugt und im Hochvakuum getrocknet. Man erhält 9.1 g (45% d. Th.) der p-Methoxybenzyl-geschützten Aminosäure.

Diese wird in 1.6 1 Dioxan/Wasser 1:1 aufgenommen, mit Natronlauge auf pH 10 eingestellt und anschließend tropfenweise mit 12.97 g (76 mmol) Benzyl-chlorcarbonat versetzt. Nach 15 min Rühren bei RT wird Dioxan im Vakuum entfernt und die verbleibende Lösung mit 2M Salzsäure auf pH 2 eingestellt. Man extrahiert mit Essigsäureethylester und wäscht die organische Phase anschließend zweimal mit Wasser. Die organische Phase wird dann eingeengt und der Rückstand im Hochvakuum getrocknet. Anschließend erfolgt eine Reinigung durch Flash-Chromatographie an Kieselgel mit Acetonitril als Eluent. Die entsprechenden Fraktionen werden eingeengt und der Rückstand im Hochvakuum getrocknet. Man erhält 5.6 g (38% d. Th.) der geschützten Aminosäure.
LC-MS (Methode 3): $R_t$ = 2.47 min; m/z = 372 $(M+H)^+$.
5.6 g (15 mmol) 5-{[(Benzyloxy)carbonyl](4-methoxybenzyl)amino}valeriansäure werden in 60 ml Dichlormethan gelöst und mit 2.2 ml Thionylchlorid versetzt. Man erhitzt die Mischung 30 min lang unter Rückfluss. Danach wird im Vakuum eingeengt, der Rückstand erneut mit Dichlormethan versetzt und abermals eingeengt. Zurück bleibt ein viskoses Öl, das im Hochvakuum getrocknet wird. Man erhält 5.7 g (98% d. Th.) der Zielverbindung, welche ohne weitere Aufreinigung und Charakterisierung weiter umgesetzt wird.

**Ausführungsbeispiele:**

Allgemeine Vorschrift 1 zur Herstellung von Caesium-Salzen von Carbonsäuren oder geeignet geschützten Aminosäure-Derivaten:

1 mmol der entsprechenden Carbonsäure wird in einer Mischung aus 10 ml Dioxan und 10 ml Wasser gelöst und mit 0.5 mmol Caesiumcarbonat versetzt. Anschließend wird lyophilisiert.

Allgemeine Vorschrift 2 zur Herstellung von urethan-geschützten N-Carboxy-anhydriden von geeignet geschützten Aminosäure-Derivaten:

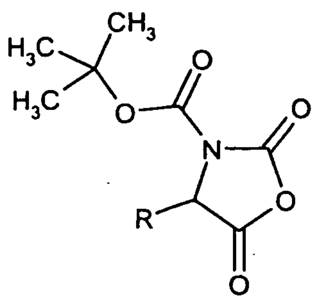

Urethangeschützte N-Carboxyanhydride von Aminsosäure-Derivaten sind entweder kommerziell erhältlich oder können nach Literaturvorschriften hergestellt werden: M. Johnston et al. J.Org.Chem. 1985, 50, 2200; W.D. Fuller et al. J.Am.Chem.Soc. 1990, 112, 7414; S. Mobasheri et al. J.Org.Chem. 1992, 57, 2755.

Allgemeine Vorschrift 3 zur Herstellung von N-Hydroxysuccinimid Estern von geeignet geschützten Aminosäure-Derivaten:

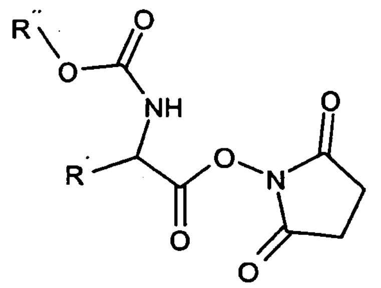

N-Hydroxysuccinimid Ester von Aminsosäure-Derivaten sind entweder kommerziell erhältlich oder können nach Standardmethoden der Peptidchemie hergestellt werden.

**Beispiel 1**

2-[[(5-Chlor-2-thienyl)carbonyl]({(5S)-2-oxo-3-[4-(3-oxomorpholin-4-yl)phenyl]-1,3-oxazolidin-5-yl}methyl)amino]-4-oxobutyl-glycinat-Hydrochlorid

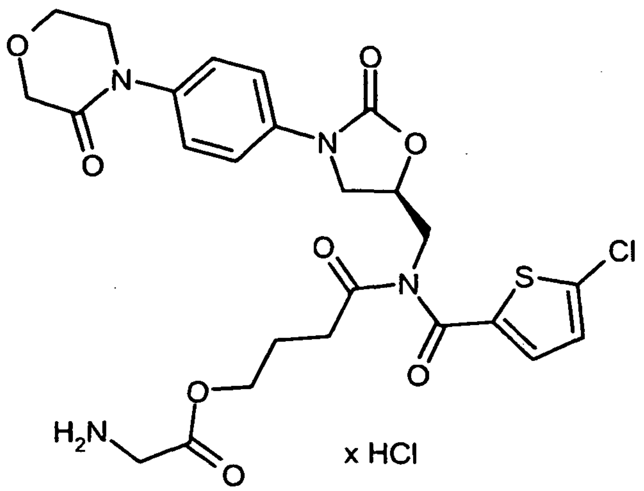

*Stufe a):*

14 mg (26 μmol) von Beispiel 1A werden mit 9.5 mg (31 μmol) des Caesiumsalzes von Boc-Glycin (hergestellt aus Boc-Glycin nach der Allgemeinen Vorschrift 1) in 5 ml DMF gelöst. Nach 16 h Rühren bei 50°C wird eingeengt und der Rückstand durch präparative HPLC (Methode 1a) aufgereinigt. Die entsprechenden Fraktionen werden eingeengt und im Hochvakuum getrocknet. Man erhält 8 mg (45% d. Th.) der geschützten Titelverbindung.
HPLC (Methode 2): $R_t$ = 5.18 min;
LC-MS (Methode 3): $R_t$ = 2.38 min; m/z = 679 $(M+H)^+$.

*Stufe b*):

7 mg (11 μmol) der in Stufe a) erhaltenen, noch verunreinigten geschützten Zwischenstufe werden mit 1 ml einer 22%-igen Lösung von Chlorwasserstoff in Dioxan versetzt. Nach 30 min wird das Gemisch im Vakuum bei 25°C oder darunter eingeengt. Der Rückstand wird durch präparative HPLC (Methode 1a) aufgereinigt. Die entsprechenden Fraktionen werden eingeengt und anschließend aus Dioxan lyophilisiert. Man erhält 0.6 mg (8% d. Th.) der Titelverbindung.
HPLC (Methode 2): R, = 4.2 min;

LC-MS (Methode 3): $R_t$ = 1.33 min; m/z = 579 $(M+H)^+$.

**Beispiel 2**

2-[[(5-Chlor-2-thienyl)carbonyl]({(5*S*)-2-oxo-3-[4-(3-oxomorpholin-4-yl)phenyl]-1,3-oxazolidin-5-yl}methyl)amino]-5-oxopentyl-glycinat-Hydrochlorid

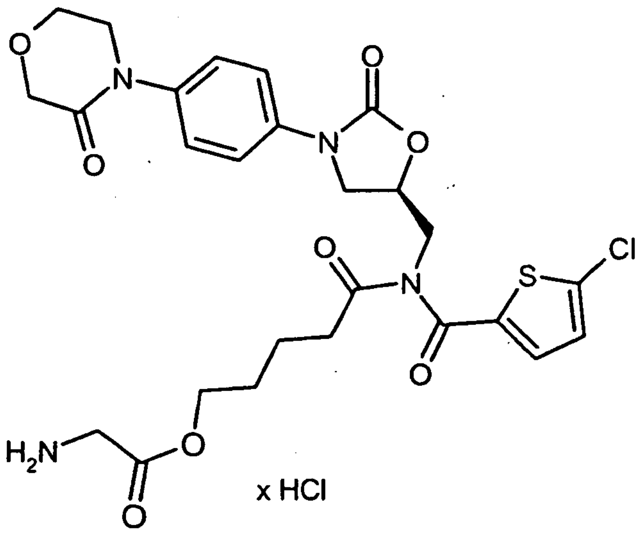

*Stufe a):*

59 mg (106 μmol) von Beispiel 2A werden mit 43 mg (138.4 μmol) des Caesiumsalzes von Boc-Glycin (hergestellt aus Boc-Glycin nach der Allgemeinen Vorschrift 1) in 10 ml DMF gelöst. Nach 16 h Rühren bei 50°C wird eingeengt und der Rückstand durch präparative HPLC (Methode 1a) aufgereinigt. Die entsprechenden Fraktionen werden eingeengt und im Hochvakuum getrocknet. Man erhält 26 mg (35% d. Th.) der geschützten Titelverbindung.
HPLC (Methode 2): $R_t$ = 5.27 min;
LC-MS (Methode 6): $R_t$ = 2.23 min; m/z = 693 $(M+H)^+$.

*Stufe b*):

12 mg (17 μmol) der in Stufe a) erhaltenen, geschützten Zwischenstufe werden mit 3 ml einer 22%-igen Lösung von Chlorwasserstoff in Dioxan versetzt. Nach 30 min wird das Gemisch im Vakuum bei 25°C oder darunter eingeengt. Der Rückstand wird durch präparative HPLC (Methode 1a) aufgereinigt. Die entsprechenden Fraktionen werden eingeengt und anschließend aus Salzsäure pH 4 lyophilisiert. Man erhält 7.2 mg (66% d. Th.) der Titelverbindung.
HPLC (Methode 2): $R_t$ = 4.32 min;
LC-MS (Methode 5): $R_t$ = 1.48 min; m/z = 593 $(M+H)^+$.

**Beispiel 3**

2-[[(5-Chlor-2-thienyl)carbonyl]({(5*S*)-2-oxo-3-[4-(3-oxomorpholin-4-yl)phenyl]-1,3-oxazolidin-5-yl}methyl)amino]-5-oxopentyl-L-valinat-Hydrochlorid

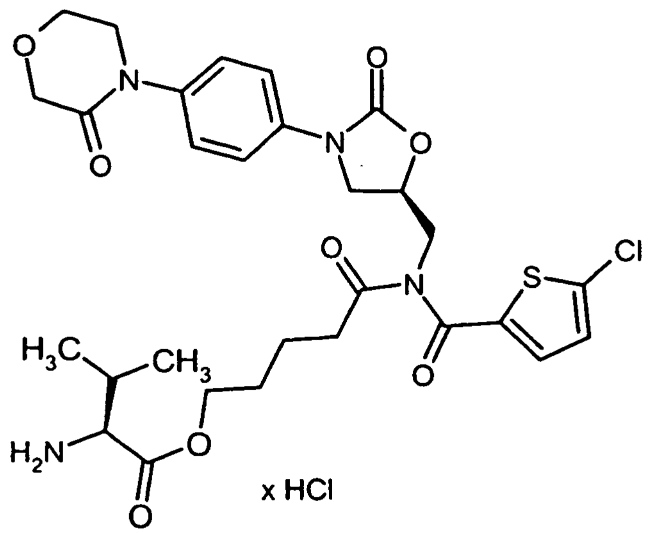

*Stufe a):*

50 mg (90 μmol) von Beispiel 2A werden mit 41 mg (117 μmol) des Caesiumsalzes von Boc-Valin (hergestellt aus Boc-Valin nach der Allgemeinen Vorschrift 1) in 10 ml DMF gelöst. Nach 42 h Rühren bei 50°C wird eingeengt und der Rückstand durch präparative HPLC (Methode 1a) aufgereinigt. Die entsprechenden Fraktionen werden eingeengt und im Hochvakuum getrocknet. Man erhält 26 mg (39% d. Th.) der geschützten Titelverbindung.
HPLC (Methode 2): $R_t$ = 5.71 min;
LC-MS (Methode 6): $R_t$ = 2.56 min; m/z = 733 $(M-H)^+$.

*Stufe b*):

26 mg (35 μmol) der in Stufe a) erhaltenen, geschützten Zwischenstufe werden in 5 ml Dichlormethan aufgenommen und mit 2 ml wasserfreier Trifluoressigsäure versetzt. Nach 30 min wird im Vakuum bei 25°C oder darunter eingeengt. Der Rückstand wird mit Acetonitril verrührt und das Lösungsmittel anschließend entfernt. Der Rückstand wird aus Salzsäure pH 3 lyophilisiert. Man erhält 24 mg (quant.) der Titelverbindung.
HPLC (Methode 2): $R_t$ = 4.5 min;
LC-MS (Methode 3): $R_t$ = 1.52 min; m/z = 635 $(M+H)^+$.
$^1$H-NMR (500MHz, DMSO-$d_6$): δ = 0.95 (2d, 6H), 1.65 (m, 4H), 2.15 (m, 1H), 2.6 (m, 2H), 3.7 (t, 2H), 3.8 (dd, 1H), 3.9 (d, 1H), 3.95 (t, 2H), 4.1-4.3 (m, 7H), 4.9 (m, 1H), 7.3 (d, 1H), 7.4 (d, 2H), 7.5 (d, 2H), 7.6 (d, 1H), 8.3 (m, 3H).

<u>**Beispiel 4**</u>

S-(5-{[(5-Chlor-2-thienyl)carbonyl]({(5S)-2-oxo-3-[4-(3-oxomorpholin-4-yl)phenyl]-1,3-oxazolidin-5-yl}methyl)amino}-5-oxopentyl)(2S)-2-amino-3-methylbutanthioat-Hydrochlorid

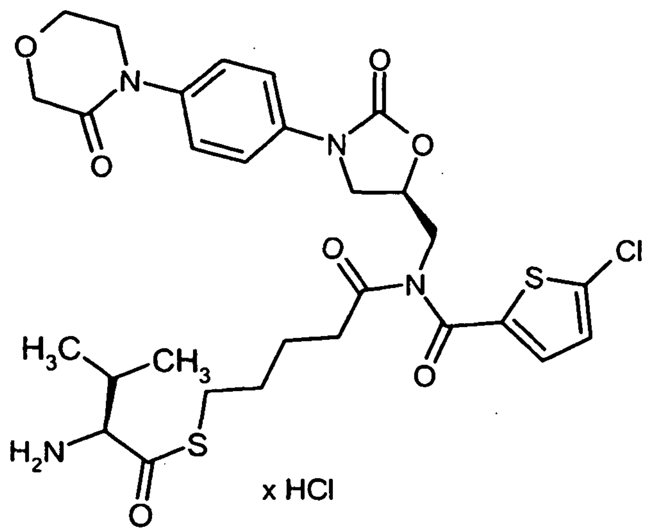

*Stufe a):*

50 mg (90 µmol) von Beispiel 2A werden mit 42 mg (180 µmol) von Beispiel 6A in 10 ml DMF gelöst. Man setzt 16 µl Ethyldiisopropylamin zu und rührt 16 h bei 60°C. Anschließend wird eingeengt und der Rückstand durch präparative HPLC (Methode 1b) aufgereinigt. Die entsprechenden Fraktionen werden eingeengt und im Hochvakuum getrocknet. Man erhält 17 mg (25% d. Th.) der geschützten Titelverbindung.
HPLC (Methode 2): $R_t$ = 5.56 min;

*Stufe b*):

17 mg (23 µmol) der in Stufe a) erhaltenen, geschützten Zwischenstufe werden mit 3 ml wasserfreier Trifluoressigsäure versetzt. Nach 15 min wird im Vakuum bei 25°C oder darunter eingeengt. Der Rückstand wird in Salzsäure, die auf pH 3 eingestellt wird, aufgenommen und zweimal mit wenig Dichlormethan und Essigsäureethylester ausgeschüttelt. Die wässrige Phase wird eingeengt und anschließend aus Salzsäure pH 3 lyophilisiert. Man erhält 7 mg (45% d. Th.) der Titelverbindung.
HPLC (Methode 2): $R_t$ = 4.65 min;
LC-MS (Methode 6): $R_t$ = 1.5 min; m/z = 651 $(M+H)^+$.
$^1H$-NMR (500MHz, DMSO-$d_6$): δ = 0.95 und 1.0 (2d, 6H), 1.5-1.7 (m, 4H), 2.15 (m, 1H), 2.55 (t, 2H), 3.0 (m, 2H), 3.7 (t, 2H), 3.8 (dd, 1H), 3.95 (t, 2H), 4.1-0.3 (m, 6H), 4.9 (m, 1H), 7.3 (d, 1H), 7.4 (d, 2H), 7.5 (d, 2H), 7.6 (d, 1H), 8.3 (m, 3H).

**Beispiel 5**

S-(5-{[(5-Chlor-2-thienyl)carbonyll({(5S)-2-oxo-3-[4-(3-oxomorpholin-4-yl)phenyl]-1,3-oxazolidin-5-yl}methyl)amino}-5-oxopentyl) aminoethanthioat-Hydrochlorid

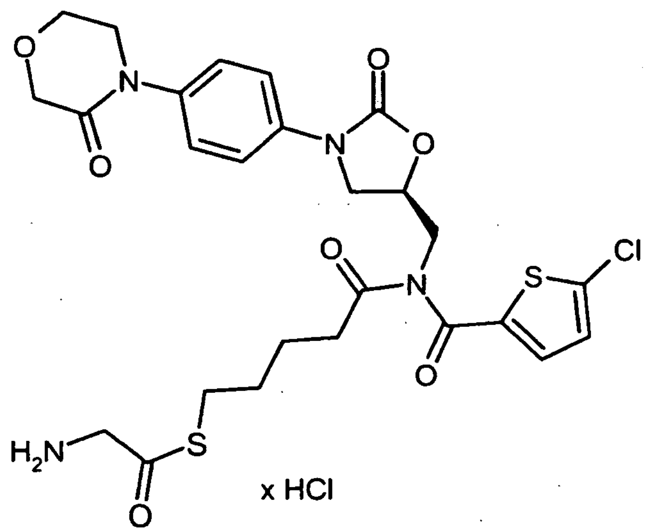

x HCl

*Stufe a):*

50 mg (90 μmol) von Beispiel 2A werden mit 52 mg (271 μmol) von Beispiel 7A in 15 ml DMF gelöst. Man setzt 16 μl Ethyldiisopropylamin zu und rührt 40 h bei 60°C. In diesem Zeitraum werden noch fünfmal jeweils 52 mg von Beispiel 7A nachgesetzt. Anschließend wird eingeengt. Der Rückstand wird in Essigsäureethylester aufgenommen und zweimal mit 10%iger Natriumcarbonatlösung ausgeschüttelt. Die organische Phase wird eingeengt und der Rückstand dann durch präparative HPLC (Methode 1a) aufgereinigt. Die entsprechenden Fraktionen mit der Zielverbindung enthalten noch Ausgangsmaterial. Sie werden im Vakuum vom Lösungsmittel befreit und so in die nächste Stufe eingesetzt. Man erhält 38 mg (59% d. Th.; Rohprodukt) der geschützten Titelverbindung.
HPLC (Methode 2): R, = 5.43 min;

*Stufe b*):

37 mg (52 μmol) der in Stufe a) erhaltenen, geschützten Zwischenstufe werden mit 3 ml wasserfreier Trifluoressigsäure versetzt. Nach 15 min wird im Vakuum bei 25°C oder darunter eingeengt. Der Rückstand wird durch präparative HPLC (Methode 1a) gereinigt. Hier erfolgt die Abtrennung von restlichem Ausgangsmaterial. Die entsprechenden Fraktionen werden eingeengt und anschließend aus Salzsäure pH 3 lyophilisiert. Man erhält 8 mg (24% d. Th.) der Titelverbindung.
HPLC (Methode 2): $R_t$ = 4.4 min;
LC-MS (Methode 12): $R_t$ = 2.1 min; m/z = 609 $(M+H)^+$.
$^1$H-NMR (500MHz, DMSO-$d_6$): δ = 1.5-1.7 (m, 4H), 2.55 (m, 2H), 3.0 (t, 2H), 3.7 (t, 2H), 3.8 (dd, 1H), 3.95 (t, 2H), 4.05-4.25 (m, 7H), 4.9 (m, 1H), 7.3 (d, 1H), 7.4 (d, 2H), 7.5 (d, 2H), 7.6 (d, 1H), 8.3 (m, 3H).

**Beispiel 6**

S-(5-{[(5-Chlor-2-thienyl)carbonyl]({(5S)-2-oxo-3-[4-(3-oxomorpholin-4-yl)phenyl]-1,3-oxazolidin-5-yl}methyl)amino}-5-oxopentyl) (2S)-2,6-diaminohexanthioat-Dihydrochlorid

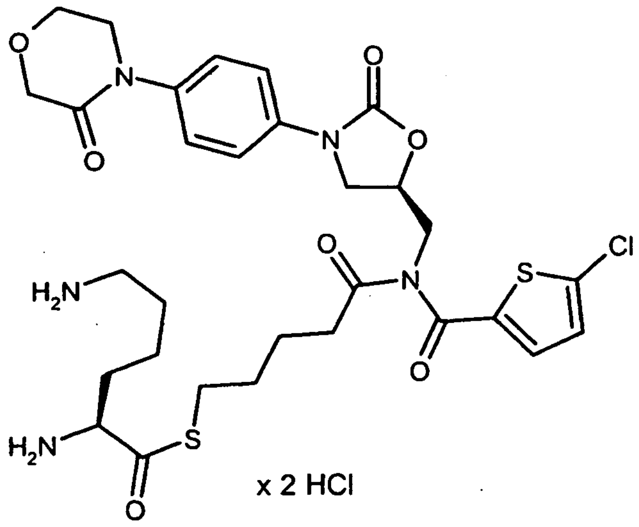

*Stufe a):*

50 mg (90 µmol) von Beispiel 2A werden mit 98 mg (271 µmol) von Beispiel 8A in 15 ml DMF gelöst. Man setzt 16 µl Ethyldiisopropylamin zu und rührt 40 h bei 60°C. In diesem Zeitraum werden noch fünfmal jeweils 98 mg von Beispiel 8A nachgesetzt. Anschließend wird eingeengt. Der Rückstand wird in Essigsäureethylester aufgenommen und zweimal mit 10%iger Natriumcarbonatlösung ausgeschüttelt. Die organische Phase wird eingeengt und der Rückstand dann durch präparative HPLC (Methode 1a) aufgereinigt. Die entsprechenden Fraktionen mit der Zielverbindung werden eingeengt und ein zweites Mal durch präparative HPLC (Methode 1a) aufgereinigt. Die Fraktionen, die die Zielverbindung rein enthalten, werden vereinigt und eingeengt. Man erhält 26 mg (33% d. Th.) der geschützten Titelverbindung.
HPLC (Methode 2): $R_t$ = 5.85 min;

*Stufe b*):

25 mg (28 µmol) der in Stufe a) erhaltenen, geschützten Zwischenstufe werden mit 5 ml wasserfreier Trifluoressigsäure versetzt. Nach 5 min wird im Vakuum bei 25°C oder darunter eingeengt. Der Rückstand wird durch präparative HPLC (Methode 1a) gereinigt. Die entsprechenden Fraktionen werden eingeengt und anschließend aus Salzsäure pH 3 lyophilisiert. Man erhält 10 mg (49% d. Th.) der Titelverbindung.
HPLC (Methode 2): $R_t$ = 4.2 min;
LC-MS (Methode 13): $R_t$ = 2.6 min; m/z = 680 $(M+H)^+$.
$^1$H-NMR (500MHz, DMSO-$d_6$): δ = 1.3-1.5 (m, 2H), 1.5-1.7 (m, 6H), 1.7-1.9 (m, 2H), 2.55 (m, 2H), 2.75 (m, 2H), 2.95 (t, 2H), 3.7 (t, 2H), 3.8 (dd, 1H), 3.95 (t, 2H), 4.1-4.3 (m, 6H), 4.9 (m, 1H), 7.3 (d, 1H), 7.4 (d, 2H), 7.5 (d, 2H), 7.6 (d, 1H), 7.85 (m, 3H), 8.5 (m, 3H).

**Beispiel 7**

S-(5-{[(5-Chlor-2-thienyl)carbonyl]({(5S)-2-oxo-3-[4-(3-oxomorpholin-4-yl)phenyl]-1,3-oxazolidin-5-yl}methyl)amino}-5-oxopentyl)(2S)-2-aminopropanthioat-Hydrochlorid

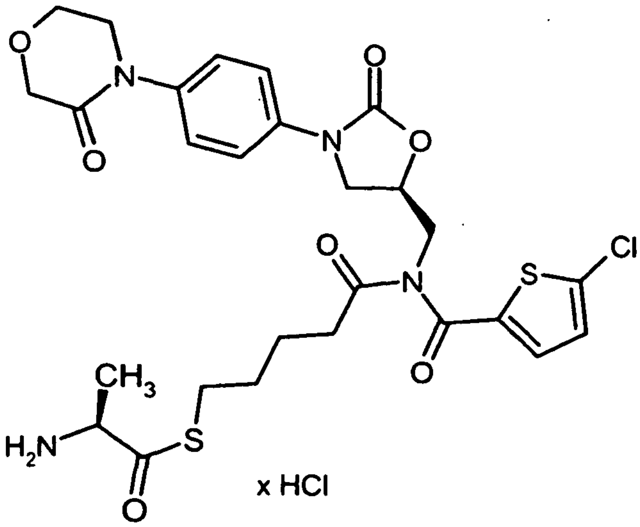

*Stufe a*):

50 mg (90 µmol) von Beispiel 2A werden mit 55 mg (270 µmol) von Beispiel 9A in 15 ml DMF gelöst. Man setzt 16 µl Ethyldiisopropylamin zu und rührt 40 h bei 60°C. In diesem Zeitraum werden noch fünfmal jeweils 55 mg von Beispiel 9A nachgesetzt. Anschließend wird eingeengt. Der Rückstand wird in Essigsäureethylester aufgenommen und zweimal mit 10%iger Natriumcarbonatlösung ausgeschüttelt. Die organische Phase wird eingeengt und der Rückstand dann durch präparative HPLC (Methode 1a) aufgereinigt. Die entsprechenden Fraktionen werden vereinigt und vom Lösungsmittel befreit. Man erhält 28 mg (43% d. Th.) der geschützten Titelverbindung.

*Stufe b*):

28 g (19 µmol) der in Stufe a) erhaltenen, geschützten Zwischenstufe werden mit 3 ml wasserfreier Trifluoressigsäure versetzt. Nach 15 min wird der Ansatz im Vakuum bei 25°C oder darunter eingeengt. Der Rückstand wird durch präparative HPLC (Methode 1a) gereinigt. Die entsprechenden Fraktionen werden eingeengt und anschließend aus Salzsäure pH 3 lyophilisiert. Man erhält 10 mg (81% d. Th.) der Titelverbindung.
HPLC (Methode 2): $R_t$ = 4.46 min;
LC-MS (Methode 14): $R_t$ = 3.37 min; m/z = 623 $(M+H)^+$.
$^1$H-NMR (500MHz, DMSO-$d_6$): $\delta$ = 1.45 (d, 3H), 1.5-1.7 (m, 4H), 2.55 (t, 2H), 2.95 (t, 2H), 3.7 (t, 2H), 3.8 (dd, 1H), 3.95 (t, 2H), 4.1-4.25 (m, 5H), 4.3 (q, 1H), 4.9 (m, 1H), 7.3 (d, 1H), 7.4 (d, 2H), 7.5 (d, 2H), 7.6 (d, 1H), 8.4 (m, 3H).

**Beispiel 8**

S-(5-{[(5-Chlor-2-thienyl)carbonyl]({(5S)-2-oxo-3-[4-(3-oxomorpholin-4-yl)phenyl]-1,3-oxazolidin-5-yl}methyl)amino}-4-oxobutyl) (2S)-2-amino-3-methylbutanthioat-Hydrochlorid

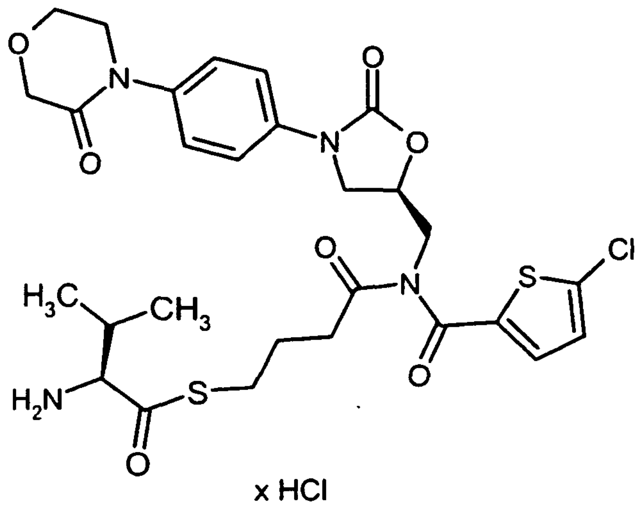

x HCl

*Stufe a):*

48 mg (89 µmol) von Beispiel 1 A werden mit 62 mg (266 µmol) von Beispiel 6A in 15 ml DMF gelöst. Man setzt 16 µl Ethyldiisopropylamin zu und rührt 40 h bei 60°C. In diesem Zeitraum werden noch fünfmal jeweils 62 mg von Beispiel 6A nachgesetzt. Anschließend wird eingeengt. Der Rückstand wird in Essigsäureethylester aufgenommen und zweimal mit 10%iger Natriumcarbonatlösung ausgeschüttelt. Die organische Phase wird eingeengt und der Rückstand dann durch präparative HPLC (Methode 1a) aufgereinigt. Die entsprechenden Fraktionen werden eingeengt und im Hochvakuum getrocknet. Man erhält 22 mg (34% d. Th.) der geschützten Titelverbindung.
HPLC (Methode 2): $R_t$ = 5.76 min;

*Stufe b*):

22 mg (30 µmol) der in Stufe a) erhaltenen, geschützten Zwischenstufe werden mit 3 ml wasserfreier Trifluoressigsäure versetzt. Nach 5 min wird im Vakuum bei 25°C oder darunter eingeengt. Der Rückstand wird durch präparative HPLC (Methode 1a) gereinigt. Die entsprechenden Fraktionen werden eingeengt und anschließend aus Salzsäure pH 3 lyophilisiert. Man erhält 11 mg (52% d. Th.) der Titelverbindung.
HPLC (Methode 2): $R_t$ = 4.55 min;
LC-MS (Methode 6): $R_t$ = 1.43 min; m/z = 637 $(M+H)^+$.
$^1$H-NMR (500MHz, DMSO-$d_6$): $\delta$ = 0.95 und 1.0 (2d, 6H), 1.8-1.9 (m, 2H), 2.2 (m, 1H), 2.65 (m, 2H), 3.0 (m, 2H), 3.7 (t, 2H), 3.8 (dd, 1H), 3.95 (t, 2H), 4.1-4.3 (m, 6H), 4.9 (m, 1H), 7.3 (d, 1H), 7.4 (d, 2H), 7.5 (d, 2H), 7.65 (d, 1H), 8.4 (m, 3H).

## Beispiel 9

5-Chlor-N-[4-(glycylamino)butanoyl]-N-({(5S)-2-oxo-3-[4-(3-oxomorpholin-4-yl)phenyl]-1,3-oxazolidin-5-yl}methyl)thiophen-2-carboxamid-Hydrochlorid

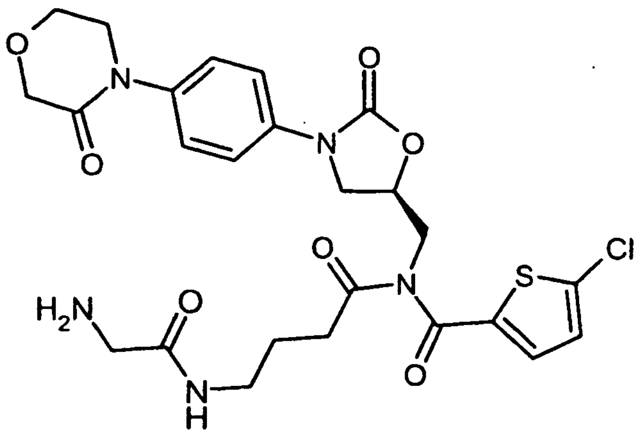

x HCl

*Stufe a):*

40 mg (72 μmol) von Beispiel 3A werden mit 17 mg (86 μmol) tert-Butyl-2,5-dioxo-1,3-oxazolidin-3-carboxylat in 5 ml DMF gelöst. Man setzt portionsweise 13 μl Ethyldiisopropylamin zu und rührt weitere 10 min bei RT. Anschließend wird eingeengt und der Rückstand dann durch präparative HPLC (Methode 1a) aufgereinigt. Die entsprechenden Fraktionen werden eingeengt und im Hochvakuum getrocknet. Man erhält 22 mg (44% d. Th.) der geschützten Titelverbindung.
HPLC (Methode 2): $R_t$ = 4.74 min;
LC-MS (Methode 5): $R_t$ = 2.07 min; m/z = 678 $(M+H)^+$.

*Stufe b*):

22 mg (32 μmol) der in Stufe a) erhaltenen, geschützten Zwischenstufe werden in 10 ml einer gesättigten Lösung von Chlorwasserstoff in Dioxan aufgenommen. Man setzt 1 ml Wasser zu und rührt 5 min bei RT. Nach 5 min wird im Vakuum bei 25°C oder darunter eingeengt. Der Rückstand wird durch präparative HPLC (Methode 1b) gereinigt. Die entsprechenden Fraktionen werden eingeengt und anschließend aus Salzsäure pH 3 lyophilisiert. Man erhält 4 mg (21% d. Th.) der Titelverbindung.
HPLC (Methode 2): $R_t$ = 4.14 min;
LC-MS (Methode 3): $R_t$ = 1.26 min; m/z = 578 $(M+H)^+$.

__**Beispiel 10**__

5-Chlor-N-[4-(glycylamino)pentanoyl]-N-({(5S)-2-oxo-3-[4-(3-oxomorpholin-4-yl)phenyl]-1,3-oxazolidin-5-yl}methyl) thiophen-2-carboxamide-Hydrochlorid

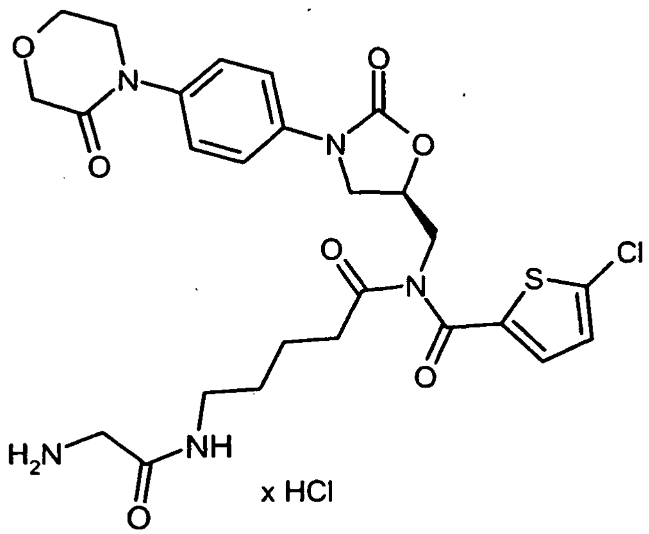

*Stufe a):*

35 mg (61 µmol) von Beispiel 4A werden mit 37 mg (184 µmol) tert-Butyl-2,5-dioxo-1,3-oxazolidin-3-carboxylat in 5 ml DMF gelöst. Man setzt portionsweise 12 µl Ethyldiisopropylamin zu und rührt weitere 10 min bei RT. Anschließend wird eingeengt und der Rückstand dann durch präparative HPLC (Methode 1a) aufgereinigt. Die entsprechenden Fraktionen werden eingeengt und im Hochvakuum getrocknet. Man erhält 15 mg (36% d. Th.) der geschützten Titelverbindung.
HPLC (Methode 2): $R_t$ = 4.85 min;
LC-MS (Methode 6): R, = 1.95 min; m/z = 692 $(M+H)^+$.

*Stufe b*):

15 mg (22 µmol) der in Stufe a) erhaltenen, geschützten Zwischenstufe werden in 3 ml einer gesättigten Lösung von Chlorwasserstoff in Dioxan aufgenommen und mit einem Tropfen Wasser versetzt. Nach 10 min Rühren bei RT wird im Vakuum bei 25°C oder darunter eingeengt. Der Rückstand wird in 30 ml wässriger Salzsäure (pH3) aufgenommen und zweimal mit Dichlormethan und zweimal mit Essigsäureethylester ausgeschüttelt. Die wässrige Phase wird eingeengt und anschließend aus Salzsäure pH 3 lyophilisiert. Man erhält 8 mg (58% d. Th.) der Titelverbindung.
HPLC (Methode 2): $R_t$ = 4.24 min;
LC-MS (Methode 3): $R_t$ = 1.36 min; m/z = 592 $(M+H)^+$.

**Beispiel 11**

N-(5-{[(5-Chlor-2-thienyl)carbonyl]({(5S)-2-oxo-3-[4-(3-oxomorpholin-4-yl)phenyl]-1,3-oxazolidin-5-yl}methyl)amino}-5-oxopentyl)-L-prolinamid-Hydrochlorid

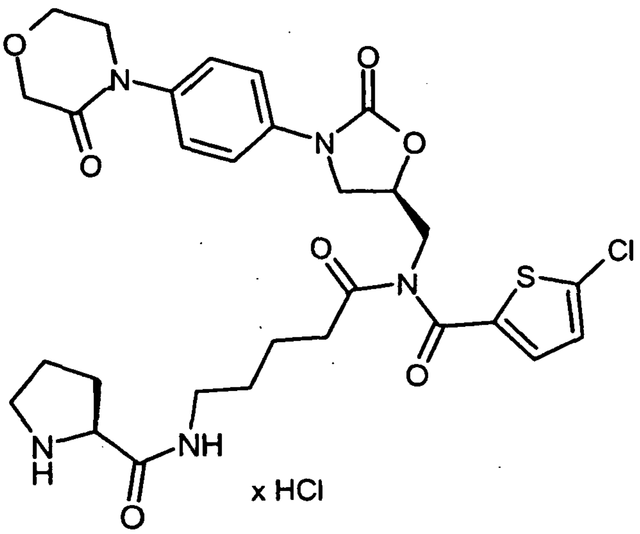

*Stufe a):*

50 mg (87 μmol) von Beispiel 4A werden mit 47 mg (175 μmol) Benzyl-(2S)-2-(chlorcarbonyl)pyrrolidin-1-carboxylat in 80 ml Dichlormethan vorgelegt. Man setzt in 3 Portionen innerhalb von 3 Minuten 263 μmol einer 0.1 M Lösung von Ethyldiisopropylamin gelöst in DMF zu und rührt weitere 10 min bei RT. Anschließend wird mit Essigsäure angesäuert und eingeengt. Der Rückstand wird in 2 ml DMF aufgenommen und dann durch präparative HPLC (Methode 1b) aufgereinigt. Die entsprechenden Fraktionen werden eingeengt und im Hochvakuum getrocknet. Man erhält 40 mg (60% d. Th.) der geschützten Titelverbindung.
HPLC (Methode 2): $R_t$ = 5.05 min;

*Stufe b):*

40 mg (52 μmol) der in Stufe a) erhaltenen, geschützten Zwischenstufe werden in 40 ml wasserfreier Trifluoressigsäure aufgenommen. Nach 16 h Rühren bei RT wird im Vakuum bei 25°C oder darunter eingeengt und der Rückstand durch präparative HPLC (Methode 1b) gereinigt. Die entsprechenden Fraktionen werden eingeengt und anschließend aus Salzsäure pH 3 lyophilisiert. Man erhält 16 mg (46% d. Th.) der Titelverbindung.
HPLC (Methode 2): $R_t$ = 4.28 min;
LC-MS (Methode 3): $R_t$ = 1.39 min; m/z = 632 $(M+H)^+$.
$^1$H-NMR (500MHz, DMSO-$d_6$): δ = 1.4 (m, 2H), 1.55 (m, 2H), 1.9 m (2H), 1.8 und 2.25 (2m, 2H), 2.55 (m, 2H), 3.0-3.3 (m, 4H), 3.7 (t, 2H), 3.8 (dd, 1H), 3.95 (t, 2H), 4.05-4.25 (m, 6H), 4.9 (m, 1H), 7.3 (d, 1H), 7.4 (d, 2H), 7.5 (d, 2H), 7.6 (d, 1H), 8.5 (m, 2H).

**Beispiel 12**

N-(5-{[(5-Chlor-2-thienyl)carbonyl]({(5S)-2-oxo-3-[4-(3-oxomorpholin-4-yl)phenyl]-1,3-oxazolidin-5-yl}methyl)amino}-5-oxopentyl)-L-histidinamid-Hydrochlorid

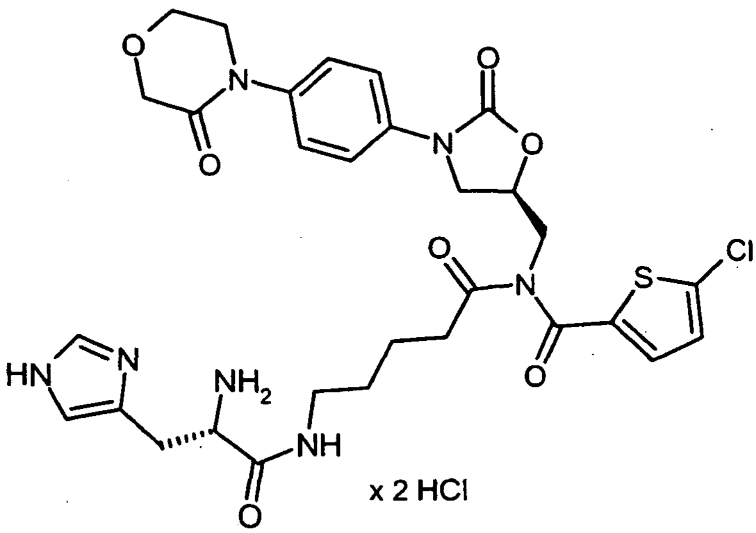

*Stufe a):*

199 mg (441 µmol) 2,5-Dioxopyrrolidin-1-yl-N,1-bis(tert-butoxycarbonyl)-L-histidinat werden zusammen mit 661 µl einer 0.1M Lösung von Ethyldiisopropylamin in DMF in 1 ml DMF vorgelegt. 42 mg (73 µmol) von Beispiel 4A gelöst in 2.5 ml DMF werden über eine Spritze tropfenweise über einen Zeitraum von 30 min zugesetzt. Nach 30 min Rühren bei RT wird eingeengt und der Rückstand durch Flash-Chromatographie zunächst mit Acetonitril und später mit Acetonitril/Wasser 10:1 als Eluent gereinigt. Die entsprechenden Fraktionen, die noch verunreinigte Zielverbindung enthalten, werden vereinigt und im Vakuum eingeengt. Der Rückstand wird dann nochmals durch präparative HPLC (Methode 1a) aufgereinigt. Die entsprechenden Fraktionen, die ein Gemisch aus dem bis-Boc-geschützten und dem mono-Boc-geschützten Beispiel enthalten, werden eingeengt und im Hochvakuum getrocknet. Man erhält 18 mg (28% d. Th.) der geschützten Titelverbindung.
HPLC (Methode 2): $R_t$ = 4.48 min; 4.92 min
LC-MS (Methode 3): $R_t$ = 1.60 min; m/z = 772 $(M+H)^+$; $R_t$ = 2.58 min; m/z = 872 $(M+H)^+$.

*Stufe b):*

18 mg des Gemischs aus der bis-Boc-geschützten und der mono-Boc-geschützten Zwischenstufe werden in 4 ml wasserfreier Trifluoressigsäure aufgenommen und 20 min bei RT gerührt. Dann wird im Vakuum bei 25°C oder darunter eingeengt und der Rückstand anschließend zweimal aus Salzsäure pH 3 lyophilisiert. Man erhält 15 mg (98% d. Th.) der Titelverbindung.
HPLC (Methode 2): $R_t$ = 4.12 min;
LC-MS (Methode 3): $R_t$ = 1.09 min; m/z = 672 $(M+H)^+$.
$^1$H-NMR (500MHz, DMSO-$d_6$): δ = 1.35 (m, 2H), 1.5 (m, 2H), 2.55 (m, 2H), 3.0-3.3 (m, 4H), 3.7 (t, 2H), 3.8 (dd, 1H), 3.95 (t, 2H), 4.1-4.3 (m, 6H), 4.95 (m, 1H), 7.3 (d, 1H), 7.4 (d, 2H), 7.45 (s, 1H), 7.5 (d, 2H), 7.65 (d, 1H), 8.5 (m, 3H), 8.7 (t, 1H), 9.0 (s, 1H).

**Beispiel 13**

N-(5-{[(5-Chlor-2-thienyl)carbonyl]({(5S)-2-oxo-3-[4-(3-oxomorpholin-4-yl)phenyl]-1,3-oxazolidin-5-yl}methyl)amino}-5-oxopentyl)-L-valinamid-Hydrochlorid

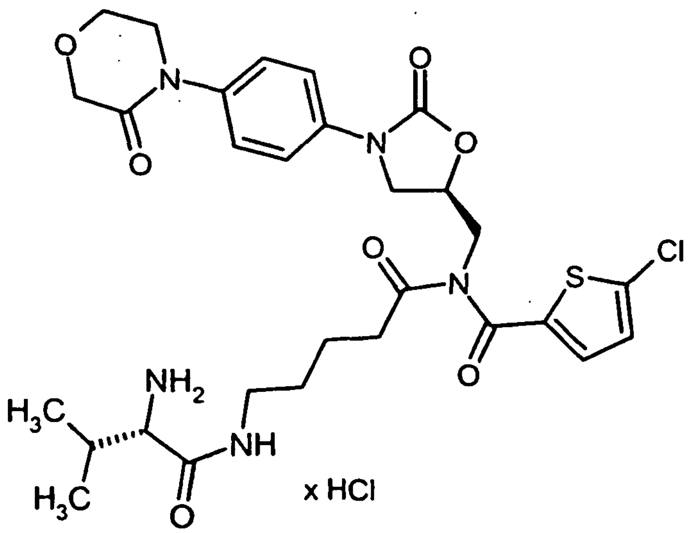

*Stufe a):*

50 mg (87 μmol) von Beispiel 4A werden zusammen mit 64 mg (262 μmol) tert-Butyl-(4S)-4-isopropyl-2,5-dioxo-1,3-oxazolidin-3-carboxylat in 20 ml Dichlormethan vorgelegt. Man setzt portionsweise 874 μl einer 0.1M Lösung von Ethyldiisopropylamin in DMF zu und rührt weitere 10 min bei RT. Anschließend wird mit Dichlormethan verdünnt und zweimal mit Wasser ausgeschüttelt. Die organische Phase wird eingeengt und der Rückstand dann durch präparative HPLC (Methode 1b) aufgereinigt. Die entsprechenden Fraktionen werden eingeengt und im Hochvakuum getrocknet. Man erhält 4.5 mg (7% d. Th.) der geschützten Titelverbindung.
HPLC (Methode 2): $R_t$ = 5.14 min;

*Stufe b):*

4.5 mg (6 μmol) der geschützten Verbindung werden in 2 ml wasserfreier Trifluoressigsäure aufgenommen und 15 min bei RT gerührt. Dann wird im Vakuum bei 25°C oder darunter eingeengt und der Rückstand in 20 ml verdünnter Salzsäure (pH 3) aufgenommen und zweimal mit Dichlormethan und einmal mit Essigsäureethylester ausgeschüttelt. Die wässrige Phase wird anschließend aus Salzsäure pH 3 lyophilisiert. Man erhält 3 mg (73% d. Th.) der Titelverbindung.
HPLC (Methode 2): $R_t$ = 4.36 min;
LC-MS (Methode 3): $R_t$ = 1.46 min; m/z = 634 $(M+M)^+$.

__Beispiel 14__

N-(5-{[(5-Chlor-2-thienyl)carbonyl]({(5S)-2-oxo-3-[4-(3-oxomorpholin-4-yl)phenyl]-1,3-oxazolidin-5-yl}methyl)amino}-5-oxopentyl)-L-lysinamid-Hydrochlorid

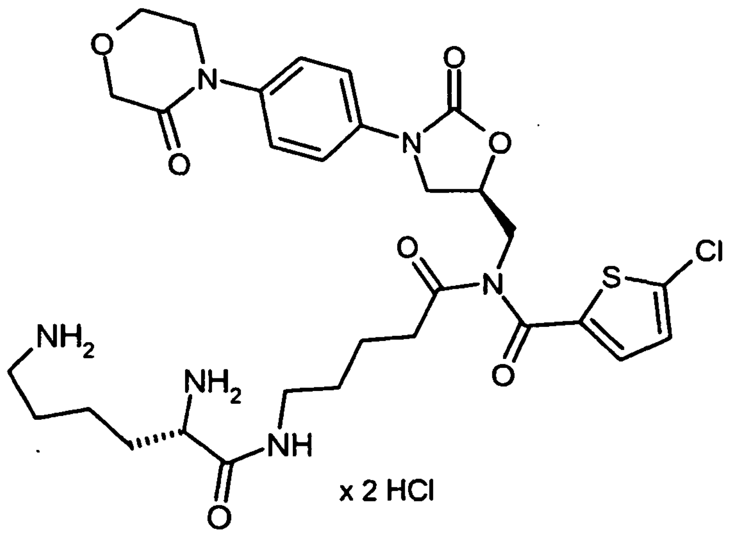

x 2 HCl

*Stufe a):*

39 mg (87 μmol) 2,5-Dioxopyrrolidin-1-yl-$N^2$,$N^6$-bis(tert-butoxycarbonyl)-L-lysinat werden zusammen mit 25 mg (44 μmol) von Beispiel 4A in 40 ml DMF gelöst und dann portionsweise mit 350 μl einer 0.1M Lösung von Ethyldiisopropylamin in DMF versetzt. Nach 10 min Rühren bei RT wird eingeengt. Der Rückstand wir in Essigsäureethylester aufgenommen und zweimal mit 10%iger Natriumcarbonatlösung ausgeschüttelt. Die organische Phase wird eingeengt und der Rückstand durch Flash-Chromatographie zunächst mit Essigsäureethylester und anschließend mit Toluol/Ethanol 1:1 als Eluent gereinigt. Die entsprechenden Fraktionen, die die noch verunreinigte Zielverbindung enthalten, werden vereinigt und im Vakuum eingeengt. Der Rückstand wird dann nochmals durch präparative HPLC (Methode 1a) aufgereinigt. Die entsprechenden Fraktionen werden eingeengt und im Hochvakuum getrocknet. Man erhält 5 mg (11 % d. Th.) der geschützten Titelverbindung.
HPLC (Methode 2): $R_t$ = 5.27 min
LC-MS (Methode 3): $R_t$ = 2.59 min; m/z = 863 $(M+H)^+$.

*Stufe b):*

5 mg des geschützten Beispiels werden in 2.5 ml wasserfreier Trifluoressigsäure aufgenommen und 20 min bei RT gerührt. Dann wird im Vakuum bei 25°C oder darunter eingeengt und der Rückstand anschließend in Salzsäure (pH 3) aufgenommen und zweimal mit Dichlormethan ausgeschüttelt. Die wässrige Phase wird abgetrennt und lyophilisiert. Man erhält 3.8 mg (89% d. Th.) der Titelverbindung.
HPLC (Methode 2): $R_t$ = 4.12 min;
LC-MS (Methode 3): $R_t$ = 1.02 min; m/z = 663 $(M+H)^+$.
$^1$H-NMR (500MHz, DMSO-$d_6$): δ = 1.3 (m, 2H), 1.4 (m, 2H), 1.5-1.6 (m, 4H), 1.7 (m, 2H), 2.55 (m, 2H), 2.75 (m, 2H), 3.1 (m, 2H), 3.7 (t, 2H), 3.8 (dd, 1H), 3.95 (t, 2H), 4.1-4.3 (m, 6H), 4.9 (m, 1H), 7.3 (d, 1H), 7.4 (d, 2H), 7.5 (d, 2H), 7.6 (d, 1H), 7.85 (m, 3H), 8.15 (m, 3H), 8.45 (t, 1H).

**Beispiel 15**

5-Chlor-N-({(5S)-2-oxo-3-[4-(3-oxomorpholin-4-yl)phenyl]-1,3-oxazolidin-5-yl}methyl)-N-[5-(L-threonylamino)pentanoyl]thiophen-2-carboxamid-Hydrochlorid

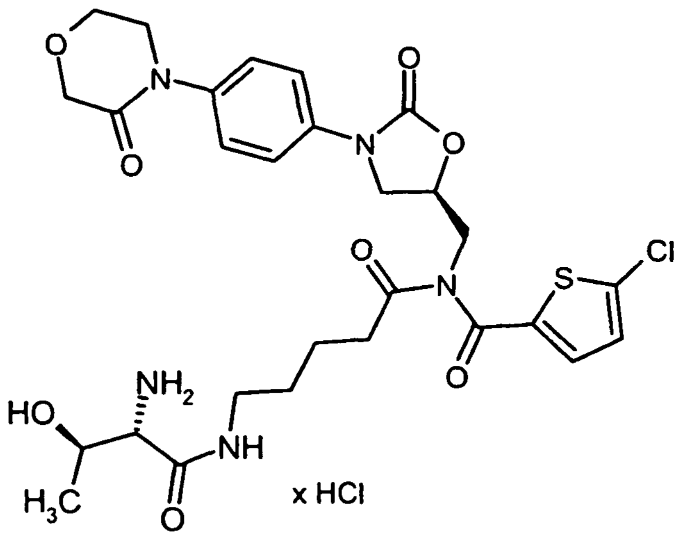

*Stufe a):*

277 mg (875 μmol) 2,5-Dioxopyrrolidin-1-yl-N-(tert-butoxycarbonyl)-L-threoninat werden zusammen mit 13.7 μl Ethyldiisopropylamin in 1 ml DMF vorgelegt. 50 mg (87 μmol) der Verbindung aus Beispiel 4A gelöst in 5 ml DMF werden tropfenweise über einen Zeitraum von 1 h zugesetzt. Nach 30 min Rühren bei RT wird eingeengt und der Rückstand durch Flash-Chromatographie zunächst mit Essigsäureethylester und später mit Toluol/Ethanol 1:1 als Eluent gereinigt. Die entsprechenden Fraktionen, die noch verunreinigte Zielverbindung enthalten, werden vereinigt und im Vakuum eingeengt. Der Rückstand wird dann nochmals durch präparative HPLC (Methode 1a) aufgereinigt. Die entsprechenden Fraktionen werden eingeengt und im Hochvakuum getrocknet. Man erhält 22 mg (34% d. Th.) der geschützten Titelverbindung.
HPLC (Methode 2a): $R_t$ = 4.8 min
LC-MS (Methode 12): $R_t$ = 3.13 min; m/z = 736 $(M+H)^+$.

*Stufe b):*

22 mg (30 μmol) der geschützten Verbindung werden in 5 ml wasserfreier Trifluoressigsäure aufgenommen und 20 min bei RT gerührt. Dann wird im Vakuum bei 25°C oder darunter eingeengt und der Rückstand anschließend in 30 ml Salzsäure (pH 3) aufgenommen und zweimal mit 30 ml Dichlormethan und einmal mit 30 ml Essigsäureethylester ausgeschüttelt. Die wässrige Phase wird abgetrennt und lyophilisiert. Man erhält 15 mg (75% d. Th.) der Titelverbindung.
HPLC (Methode 2): $R_t$ = 4.2 min;
LC-MS (Methode 3): $R_t$ = 1.39 min; m/z = 636 $(M+H)^+$.
$^1$H-NMR (500MHz, DMSO-$d_6$): δ = 1.1 (d, 3H), 1.4 (m, 2H), 1.6 (m, 2H), 2.6 (t, 2H), 3.0 und 3.15 (2m, 2H), 3.4 (m, 1H), 3.7 (t, 2H), 3.8 (m, 2H), 3.95 (t, 2H), 4.1-4.3 (m, 5H), 4.95 (m, 1H), 5.5 (d, 1H), 7.3 (d, 1H), 7.4 (d, 2H), 7.5 (d, 2H), 7.6 (d, 1H), 8.05 (m, 3 H), 8.4 (t, 1H).

**Beispiel 16**

N-(5-{[(5-Chlor-2-thienyl)carbonyl]({(5S)-2-oxo-3-[4-(3-oxomorpholin-4-yl)phenyl]-1,3-oxazolidin-5-yl} methyl)amino} -5-oxopentyl)-L-tyrosinamid-Hydrochlorid

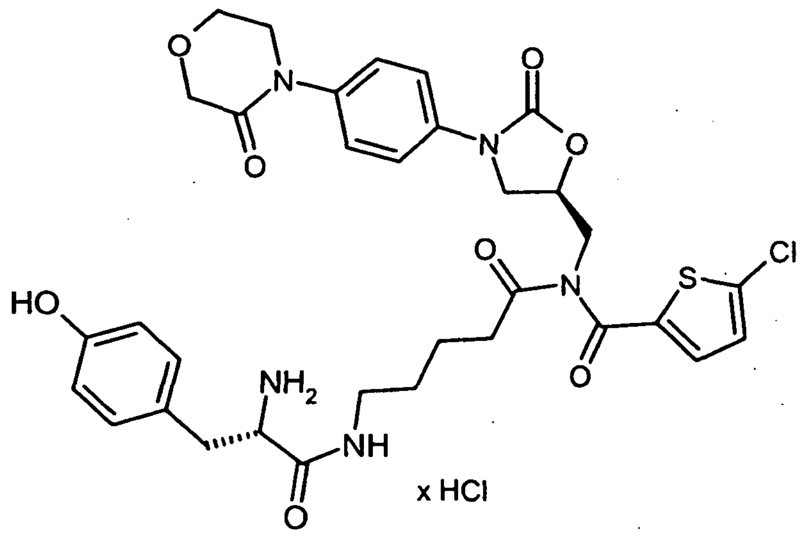

*Stufe a):*

331 mg (875 μmol) 2,5-Dioxopyrrolidin-1-yl-N-(tert-butoxycarbonyl)-L-tyrosinat werden zusammen mit 13.7 μl Ethyldiisopropylamin in 1 ml DMF vorgelegt. 50 mg (87 μmol) der Verbindung aus Beispiel 4A gelöst in 5 ml DMF werden tropfenweise über einen Zeitraum von 1 h zugesetzt. Nach 30 min Rühren bei RT wird eingeengt und der Rückstand durch Flash-Chromatographie zunächst mit Essigsäurethylester und später mit Toluol/Ethanol 1:1 als Eluent gereinigt. Die entsprechenden Fraktionen, die noch verunreinigte Zielverbindung enthalten, werden vereinigt und im Vakuum eingeengt. Der Rückstand wird dann nochmals durch präparative HPLC (Methode 1a) aufgereinigt. Die entsprechenden Fraktionen werden eingeengt und im Hochvakuum getrocknet. Man erhält 26 mg (37% d. Th.) der geschützten Titelverbindung.
HPLC (Methode 2a): $R_t$ = 5.0 min
LC-MS (Methode 3): $R_t$ = 2.38 min; m/z = 798 $(M+H)^+$.

*Stufe b):*

26 mg (33 μmol) der geschützten Verbindung werden in 5 ml wasserfreier Trifluoressigsäure aufgenommen und 10 min bei RT gerührt. Dann wird im Vakuum bei 25°C oder darunter eingeengt und der Rückstand anschließend in 60 ml Salzsäure (pH 3) aufgenommen. Nicht gelöste Bestandteile werden abfiltriert. Die wässrige Phase wird dann lyophilisiert. Man erhält 23 mg (96% d. Th.) der Titelverbindung.
HPLC (Methode 2): $R_t$ = 4.4 min;
LC-MS (Methode 12): $R_t$ = 2.09 min; m/z = 698 $(M+H)^+$.
$^1$H-NMR (500MHz, DMSO-$d_6$): δ = 1.3 (m, 2H), 1.5 (m, 2H), 2.8-3,2 (m, 4H), 3.7 (t, 2H), 3.8 (m, 2H), 3.95 (t, 2H), 4.1-4.3 (m, 5H), 4.9 (m, 1H), 6.7 (d, 2H), 7.0 (d, 2H), 7.3 (d, 1H), 7.4 (d, 2H), 7.5 (d, 2H), 7.65 (d, 1H), 8.1 (m, 3H), 8.3 (t, 1H), 9.4 (s, 1H).

**Beispiel 17**

$N^1$-(5-{[(5-Chlor-2-thienyl)carbonyl]({(5S)-2-oxo-3-[4-(3-oxomorpholin-4-yl)phenyl]-1,3-oxazolidin-5-yl}methyl)amino}-5-oxopentyl)-L-aspartamid-Hydrochlorid

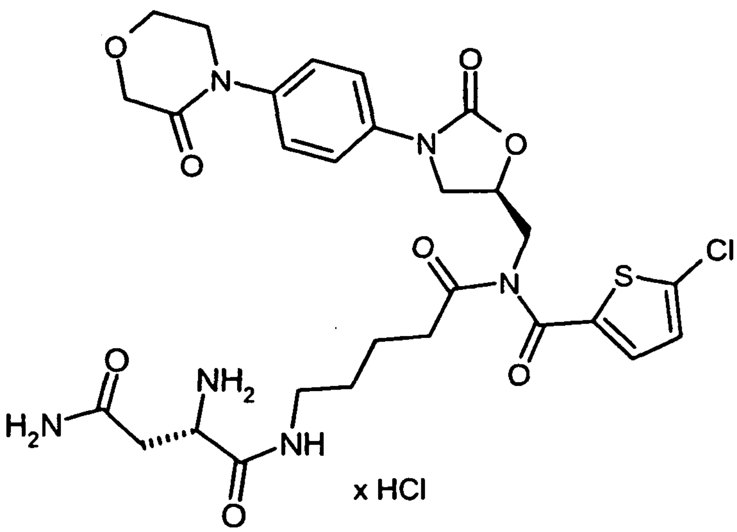

x HCl

*Stufe a):*

288 mg (875 μmol) 2,5-Dioxopyrrolidin-1-yl-$N^2$-(tert-butoxycarbonyl)-L-asparaginat werden zusammen mit 13.7 μl Ethyldiisopropylamin in 1 ml DMF vorgelegt. 50 mg (87 μmol) der Verbindung aus Beispiel 4A gelöst in 5 ml DMF werden tropfenweise über einen Zeitraum von 30 min zugesetzt. Nach weiteren 30 min Rühren bei RT wird eingeengt und der Rückstand durch präparative HPLC (Methode 1a) aufgereinigt. Die entsprechenden Fraktionen, die noch mit etwas Verbindung (A) verunreinigt sind, werden eingeengt und im Hochvakuum getrocknet. Man erhält 29 mg Rohprodukt der geschützten Titelverbindung, die ohne weitere Reinigung in die nächste Stufe eingesetzt werden.
HPLC (Methode 2): $R_t$ = 4.5 min
LC-MS (Methode 3): $R_t$ = 2.07 min; m/z = 749 $(M+H)^+$.

*Stufe b):*

26 mg des geschützten Rohproduktes aus Stufe a) werden in 5 ml wasserfreier Trifluoressigsäure aufgenommen und 10 min bei RT gerührt. Dann wird im Vakuum bei 25°C oder darunter eingeengt und der Rückstand anschließend in 50 ml Salzsäure (pH 3) aufgenommen. Nicht gelöste Bestandteile werden abfiltriert und die wässrige Phase eingeengt. Anschließend wird der Rückstand durch präparative HPLC (Methode 1a) aufgereinigt. Die entsprechenden Fraktionen werden eingeengt und im Hochvakuum getrocknet. Der Rückstand wird dann aus Salzsäure, die auf pH 3 eingestellt wurde, lyophilisiert. Man erhält 14 mg (53% d. Th.) der Titelverbindung.
HPLC (Methode 2a): $R_t$ = 4.1 min;
LC-MS (Methode 12): $R_t$ = 1.84 min; m/z = 649 $(M+H)^+$.
$^1$H-NMR (500MHz, DMSO-$d_6$): δ = 1.4 (m, 2H), 1.55 (m, 2H), 2.55 (m, 2H), 2.65 (m, 2H), 3.0-3.1 (m, 2H), 3.7 (t, 2H), 3.8 (dd, 1H), 3.95 (m, 3H), 4.1-4.3 (m, 5H), 4.9 (m, 1H), 7.2 (s, 1H), 7.3 (d, 1H), 7.4 (d, 2H), 7.5 (d, 2H), 7.6 (m, 2H), 8.0 (m, 3H), 8.3 (t, 1H).

**Beispiel 18**

N-(5-{[(5-Chlor-2-thienyl)carbonyl]({(5S)-2-oxo-3-[4-(3-oxomorpholin-4-yl)phenyl]-1,3-oxazolidin-5-yl} methyl)amino}-5-oxopentyl)-L-phenylalaninamid-Hydrochlorid

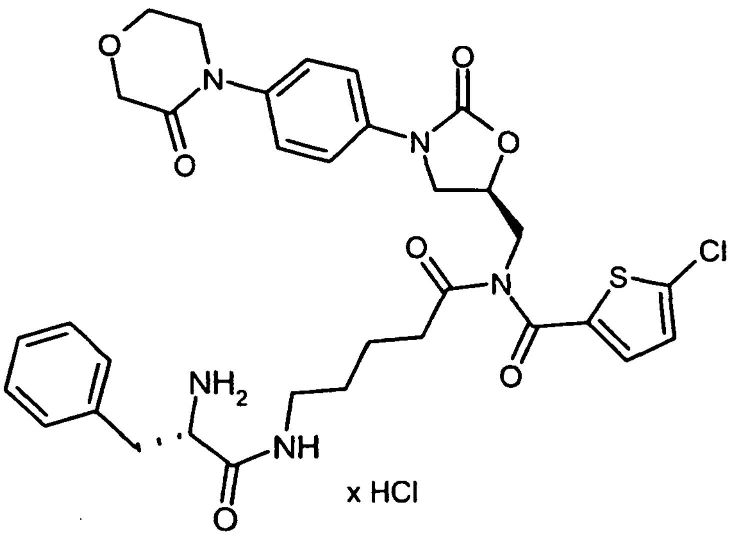

x HCl

*Stufe a):*

317 mg (875 μmol) 2,5-Dioxopyrrolidin-1-yl-N-(tert-butoxycarbonyl)-L-phenylalaninat werden zusammen mit 13.7 μl Ethyldiisopropylamin in 1 ml DMF vorgelegt. 50 mg (87 μmol) der Verbindung aus Beispiel 4A gelöst in 5 ml DMF werden tropfenweise über einen Zeitraum von 30 min zugesetzt. Nach 30 min Rühren bei RT wird eingeengt. Der Rückstand wird durch Flash-Chromatographie zunächst mit Dichlormethan/Essigsäurethylester Laufmitteln im Verhältnis 3:1, 2:1 und 1:1 eluiert. Dann wird mit reinem Essigsäureethylester und schließlich mit Ethanol als Laufmittel eluiert. Die entsprechenden Fraktionen, die noch verunreinigte Zielverbindung enthalten, werden vereinigt und im Vakuum eingeengt. Der Rückstand wird dann nochmals durch präparative HPLC (Methode 1a) aufgereinigt. Die entsprechenden Fraktionen werden eingeengt und im Hochvakuum getrocknet. Man erhält 34 mg (50% d. Th.) der geschützten Titelverbindung.
HPLC (Methode 2a): $R_t$ = 5.34 min
LC-MS (Methode 12): $R_t$ = 3.47 min; m/z = 782 $(M+H)^+$.

*Stufe b):*

33 mg (42 μmol) der geschützten Verbindung werden in Dichlormethan gelöst und mit 1.5 ml wasserfreier Trifluoressigsäure versetzt und 10 min bei RT gerührt. Dann wird im Vakuum bei 25°C oder darunter eingeengt und der Rückstand anschließend in 5 ml Salzsäure (pH 3) aufgenommen. Die wässrige Phase wird dann lyophilisiert. Man erhält 28 mg (93% d. Th.) der Titelverbindung.
HPLC (Methode 2): $R_t$ = 4.5 min;
LC-MS (Methode 12): $R_t$ = 2.08 min; m/z = 682 $(M+H)^+$.
$^1$H-NMR (400MHz, DMSO-$d_6$): δ = 1.25 (m, 2H), 1.5 (m, 2H), 2.9-3,2 (m, 4H), 3.7 (m, 2H), 3.8 (t, 2H), 3.9 (m, 1H), 4.0 (t, 2H), 4.1-4.3 (m, 5H), 4.9 (m, 1H), 7.2 (d, 2H), 7.2-7.35 (m, 4H), 7.4 (d, 2H), 7.5 (d, 2H), 7.65 (d, 1H), 8.2 (m, 2H), 8.3 (t, 1H).

**Beispiel 19**

N$^1$-(5-{[(5-Chlor-2-thienyl)carbonyl]({(5S)-2-oxo-3-[4-(3-oxomorpholin-4-yl)phenyl]-1,3-oxazolidin-5-yl}methyl)amino}-5-oxopentyl)-L-glutamamid-Hydrochlorid

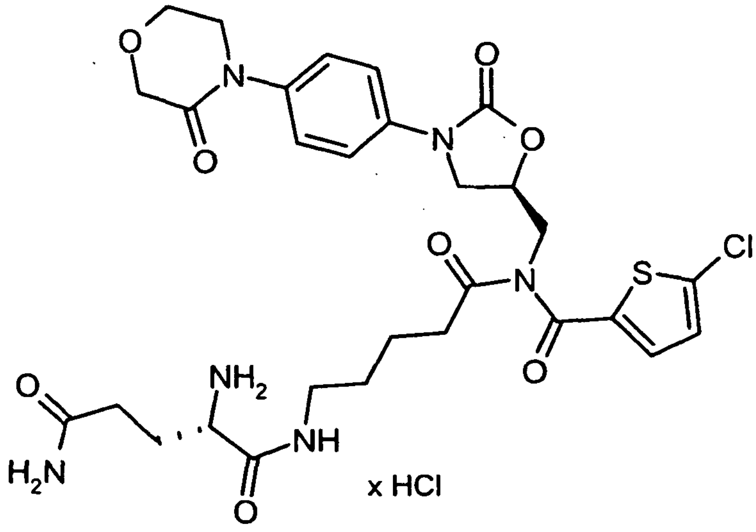

x HCl

*Stufe a):*

300 mg (875 μmol) 2,5-Dioxopyrrolidin-1-yl-$N^2$-(tert-butoxycarbonyl)-L-glutaminat werden zusammen mit 13.7 μl Ethyldiisopropylamin in 1 ml DMF vorgelegt. 50 mg (87 μmol) der Verbindung aus Beispiel 4A gelöst in 5 ml DMF werden tropfenweise über einen Zeitraum von 30 min zugesetzt. Nach 30 min Rühren bei RT wird eingeengt und der Rückstand durch Flash-Chromatographie mit Dichlormethan/Essigsäurethylester/Methanol zunächst im Verhältnis von 150:50:5, dann im Verhältnis von 150:50:10 und schließlich im Verhältnis von 150:50:20 als Eluent gereinigt. Die entsprechenden Fraktionen, die noch verunreinigte Zielverbindung enthalten, werden vereinigt und im Vakuum eingeengt. Der Rückstand wird dann nochmals durch präparative HPLC (Methode 1a) aufgereinigt. Die entsprechenden Fraktionen werden eingeengt und im Hochvakuum getrocknet. Man erhält 24 mg (34% d. Th.) der geschützten Titelverbindung.
HPLC (Methode 2a): $R_t$ = 4.57 min
LC-MS (Methode 12): $R_t$ = 2.97 min; m/z = 763 $(M+H)^+$.

*Stufe b):*

24 mg (35 μmol) der geschützten Verbindung werden in Dichlormethan gelöst und mit 2 ml wasserfreier Trifluoressigsäure versetzt und 10 min bei RT gerührt. Dann wird im Vakuum bei 25°C oder darunter eingeengt und der Rückstand anschließend in 15 ml Salzsäure (pH 3) aufgenommen. Man schüttelt zunächst zweimal mit Dichlormethan und dann einmal mit Essigsäureethylester aus. Die wässrige Phase wird anschließend lyophilisiert. Man erhält 14 mg (59% d. Th.) der Titelverbindung.
LC-MS (Methode 12): $R_t$ = 1.60 min; m/z = 663 $(M+H)^+$.
$^1$H-NMR (400MHz, DMSO-$d_6$): δ = 1.4 (m, 2H), 1.6 (m, 2H), 1.9 (q, 2H), 2.15 (m, 2H), 2.55 (m, 2H), 3.1 (m, 2H), 3.7 (t, 2H), 3.8 (dd, 1H), 4.0 (t, 2H), 4.1-4.3 (m, 5H), 4.9 (m, 1H), 6.9 (s, 1H), 7.3 (d, 1H), 7.4 (m, 3H), 7.5 (d, 2H), 7.65 (d, 1H), 8.1 (m, 3H), 8.4 (t, 1H).

**Beispiel 20**

N-(5-{[(5-Chlor-2-thienyl)carbonyl]({(5S)-2-oxo-3-[4-(3-oxomorpholin-4-yl)phenyl]-1,3-oxazolidin-5-yl} methyl)amino}-5-oxopentyl)-L-alpha-glutamin-Hydrochlorid

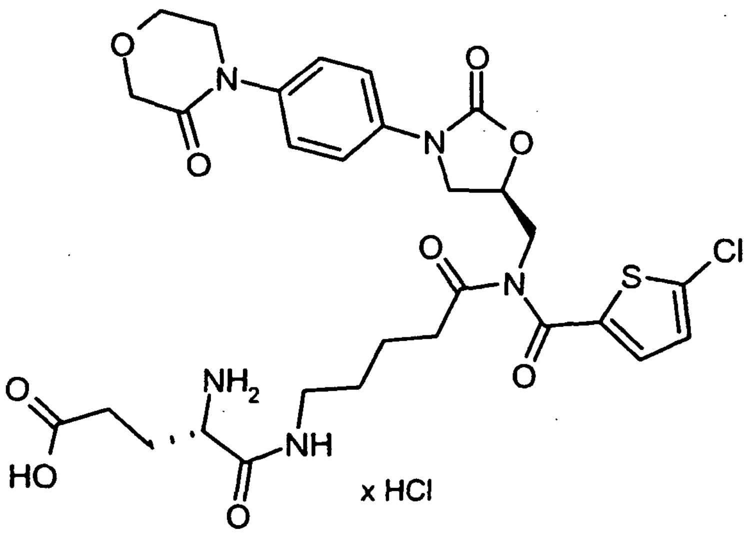

*Stufe a):*

350 mg (875 μmol) 5-tert-Butyl-1-(2,5-dioxopyrrolidin-1-yl)-N-(tert-butoxycarbonyl) -L-glutamat werden zusammen mit 13.7 μl Ethyldiisopropylamin in 1 ml DMF vorgelegt. 50 mg (87 μmol) der Verbindung aus Beispiel 4A gelöst in 5 ml DMF werden tropfenweise über einen Zeitraum von 30 min zugesetzt. Nach 30 min Rühren bei RT wird eingeengt und der Rückstand durch Flash-Chromatogaphie mit Dichlormethan/Essigsäurethylester/Methanol zunächst im Verhältnis von 150:50:5, dann im Verhältnis von 150:50:10 und schließlich im Verhältnis von 150:50:20 als Eluent gereinigt. Die entsprechenden Fraktionen, die noch verunreinigte Zielverbindung enthalten, werden vereinigt und im Vakuum eingeengt. Der Rückstand wird dann nochmals durch präparative HPLC (Methode 1a) aufgereinigt. Die entsprechenden Fraktionen werden eingeengt und im Hochvakuum getrocknet. Man erhält 35 mg (49% d. Th.) der geschützten Titelverbindung.
HPLC (Methode 2a): $R_t$ = 5.4 min
LC-MS (Methode 3): $R_t$ = 2.63 min; m/z = 820 $(M+H)^+$.

*Stufe b):*

35 mg (43 μmol) der geschützten Verbindung werden in Dichlormethan gelöst und mit 1.5 ml wasserfreier Trifluoressigsäure versetzt und 2 h bei RT gerührt. Dann wird im Vakuum bei 25°C oder darunter eingeengt und der Rückstand anschließend in 10 ml Salzsäure (pH 3) aufgenommen und lyophilisiert. Man erhält 29 mg (97% d. Th.) der Titelverbindung.
LC-MS (Methode 12): $R_t$ = 1.72 min; m/z = 664 $(M+H)^+$.
$^1$H-NMR (400MHz, DMSO-$d_6$): δ = 1.4 (m, 2H), 1.6 (m, 2H), 1.9 (q, 2H), 2.3 (m, 2H), 2.55 (m, 2H), 3.1 (m, 2H), 3.7 (t, 2H), 3.8 (dd, 1H), 4.0 (t, 2H), 4.1-4.3 (m, 5H), 4.9 (m, 1H), 7.3 (d, 1H), 7.4 (d, 2H), 7.5 (d, 2H), 7.6 (d, 1H), 8.1 (m, 3H), 8.45 (t, 1H).

**Beispiel 21**

5-Chlor-N-({(5S)-2-oxo-3-[4-(3-oxomorpholin-4-yl)phenyl]-1,3-oxazolidin-5-yl}methyl)-N-[5-(L-serylamino)pentanoyl] thiophen-2-carboxamid-Hydrochlorid

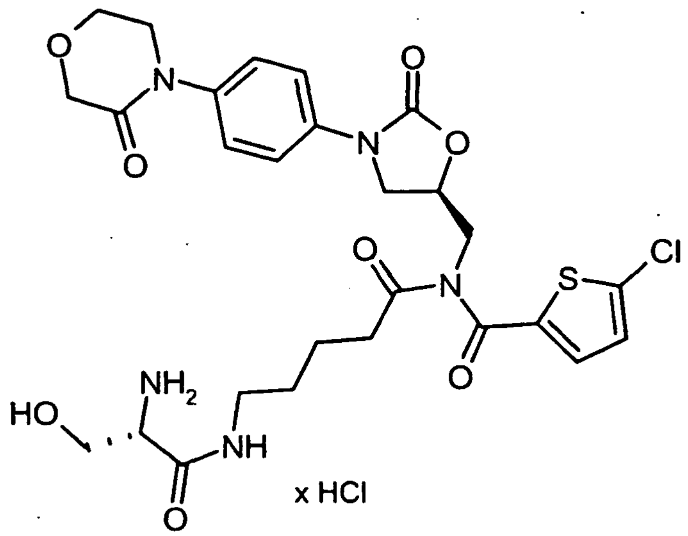

*Stufe a):*

350 mg (875 μmol) 2,5-Dioxopyrrolidin-1-yl N-(tert-butoxycarbonyl)-L-serinat werden zusammen mit 13.7 μl Ethyldiisopropylamin in 1 ml DMF vorgelegt. 50 mg (87 μmol) der Verbindung aus Beispiel 4A gelöst in 5 ml DMF werden tropfenweise über einen Zeitraum von 30 min zugesetzt. Nach 30 min Rühren bei RT wird eingeengt und der Rückstand durch Flash-Chromatographie zunächst mit Dichlormethan/Essigsäwethylester 3:1 und dann Dichlormethan/Essigsäurethylester/Methanol im Verhältnis von 150:50:5, dann 150:50:10 und schließlich im Verhältnis von 150:50:20 als Eluent gereinigt. Die entsprechenden Fraktionen, die noch verunreinigte Zielverbindung enthalten, werden vereinigt und im Vakuum eingeengt. Der Rückstand wird dann nochmals durch präparative HPLC (Methode 1a) aufgereinigt. Die entsprechenden Fraktionen werden eingeengt und im Hochvakuum getrocknet. Man erhält 33 mg (52% d. Th.) der geschützten Titelverbindung.
LC-MS (Methode 12): $R_t$ = 2.87 min; m/z = 722 $(M+H)^+$.

*Stufe b):*

33 mg (46 μmol) der geschützten Verbindung werden in Dichlormethan gelöst und mit 1.6 ml wasserfreier Trifluoressigsäure versetzt und 10 min bei RT gerührt. Dann wird im Vakuum bei 25°C oder darunter eingeengt und der Rückstand anschließend in 5 ml Salzsäure (pH 3) aufgenommen und lyophilisiert. Man erhält 24 mg (80% d. Th.) der Titelverbindung.
LC-MS (Methode 12): $R_t$ = 1.81 min; m/z = 622 $(M+H)^+$.
$^1$H-NMR (400MHz, DMSO-$d_6$): δ = 1.4 (m, 2H), 1.6 (m, 2H), 2.55 (m, 2H), 3.1 (dt, 2H), 3.6-3.8 (m, 5H), 3.85 (dd, 1H), 4.1-4.3 (m, 5H), 4.95 (m, 1H), 5.4 (m, 1H), 7.3 (d, 1H), 7.4 (d, 2H), 7.5 (d, 2H), 7.6 (d, 1H), 8.1 (m, 3H), 8.4 (t, 1H).

**Beispiel 22**

N-(5-{[(5-Chlor-2-thienyl)carbonyl]({(5S)-2-oxo-3-[4-(3-oxomorpholin-4-yl)phenyl]-1,3-oxazolidin-5-yl}methyl)amino}-5-oxopentyl)-L-leucinamid-Hydrochlorid

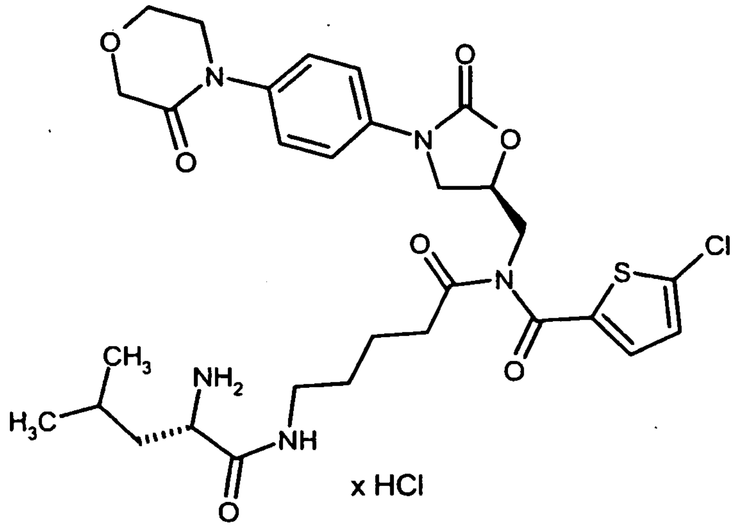

*Stufe a):*

287 mg (875 μmol) 2,5-Dioxopyrrolidin-1-yl-N-(tert-butoxycarbonyl)-L-leucinat werden zusammen mit 13.7 μl Ethyldiisopropylamin in 1 ml DMF vorgelegt. 50 mg (87 μmol) der Verbindung aus Beispiel 4A gelöst in 5 ml DMF werden tropfenweise über einen Zeitraum von 30 min zugesetzt. Nach 30 min Rühren bei RT wird eingeengt und der Rückstand durch Flash-Chromatographie zunächst mit Dichlormethan/Essigsäurethylester 3:1 und dann Dichlormethan/Essigsäurethylester/Methanol im Verhältnis von 150:50:5, dann 150:50:10 und schließlich im Verhältnis von 150:50:20 als Eluent gereinigt. Die entsprechenden Fraktionen, die noch verunreinigte Zielverbindung enthalten, werden vereinigt und im Vakuum eingeengt. Der Rückstand wird dann nochmals durch präparative HPLC (Methode 1a) aufgereinigt. Die entsprechenden Fraktionen werden eingeengt und im Hochvakuum getrocknet. Man erhält 10 mg (15% d. Th.) der geschützten Titelverbindung.
LC-MS (Methode 12): $R_t$ = 3.44 min; m/z = 748 $(M+H)^+$.

*Stufe b):*

10.2 mg (14 μmol) der geschützten Verbindung werden in Dichlormethan gelöst, mit 0.5 ml wasserfreier Trifluoressigsäure versetzt und dann 15 min bei RT gerührt. Dann wird im Vakuum bei 25°C oder darunter eingeengt und der Rückstand in 5 ml verdünnter Salzsäure (pH 3) aufgenommen und lyophilisiert. Man erhält 7 mg (73% d. Th.) der Titelverbindung.
LC-MS (Methode 12): R, = 2.25 min; m/z = 648 $(M+H)^+$.

**Beispiel 23**

N-(5-{[(5-Chlor-2-thienyl)carbonyl]({(5S)-2-oxo-3-[4-(3-oxomorpholin-4-yl)phenyl]-1,3-oxazolidin-5-yl}methyl)amino}-5-oxobutyl)-L-histidinamid-Hydrochlorid

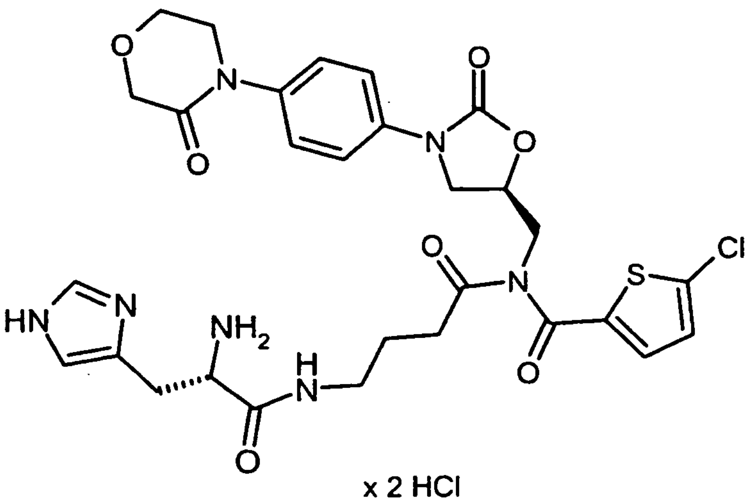

x 2 HCl

*Stufe a):*

195 mg (431 μmol) 2,5-Dioxopyrrolidin-1-yl-N,1-bis(tert-butoxycarbonyl)-L-histidinat werden zusammen mit 645 μl einer 0.1M Lösung von Ethyldiisopropylamin in DMF in 3 ml DMF vorgelegt. 40 mg (72 μmol) von Beispiel 3A gelöst in 17 ml DMF werden tropfenweise über einen Zeitraum von 1 h zugesetzt. Nach 30 min Rühren bei RT wird eingeengt und der Rückstand durch Flash-Chromatographie zunächst mit Acetonitril und später mit Acetonitril/Wasser 10:1 als Eluent gereinigt. Die entsprechenden Fraktionen, die noch verunreinigte Zielverbindung enthalten, werden vereinigt und im Vakuum eingeengt. Der Rückstand wird dann nochmals durch präparative HPLC (Methode 1a) aufgereinigt. Die entsprechenden Fraktionen, die ein Gemisch aus dem bis-Boc-geschützten und dem mono-Boc-geschützten Beispiel enthalten, werden eingeengt und im Hochvakuum getrocknet. Man erhält 30 mg (55% d. Th.) eines Gemischs aus der mono- und bis-Boc-geschützten Titelverbindung.
HPLC (Methode 2): $R_t$ = 4.47 min; 4.91 min
LC-MS (Methode 3): $R_t$ = 1.53 min; m/z = 758 $(M+H)^+$; $R_t$ = 2.45 min; m/z = 858 $(M+H)^+$.

*Stufe b):*

30 mg des Gemischs aus der bis-Boc-geschützten und der mono-Boc-geschützten Zwischenstufe werden in 2 ml wasserfreier Trifluoressigsäure aufgenommen und 10 min bei RT gerührt. Dann wird im Vakuum bei 25°C oder darunter eingeengt und der Rückstand anschließend zweimal aus Salzsäure pH 3 lyophilisiert. Man erhält 24 mg (83% d. Th.) der Titelverbindung.
HPLC (Methode 2): $R_t$ = 4.07 min;
LC-MS (Methode 13): $R_t$ = 2.41 min; m/z = 658 $(M+H)^+$.

**Beispiel 24**

N-(5-{[(5-Chlor-2-thienyl)carbonyl]({(5S)-2-oxo-3-[4-(3-oxomorpholin-4-yl)phenyl]-1,3-oxazolidin-5-yl}methyl)amino}-5-oxopentyl)-L-alpha-asparagin-Hydrochlorid

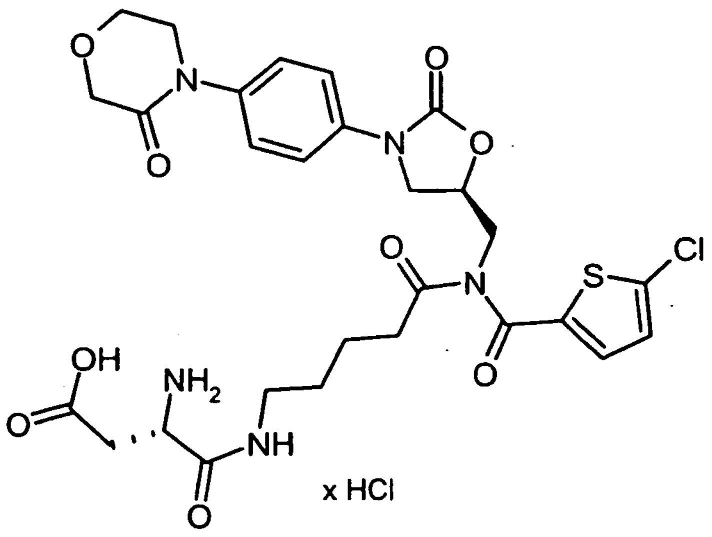

*Stufe a):*

338 mg (875 μmol) 5-tert-Butyl-1-(2,5-dioxopyrrolidin-1-yl)-N-(tert-butoxycarbonyl) -L-aspartat werden zusammen mit 13.7 μl Ethyldiisopropylamin in 1 ml DMF vorgelegt. 50 mg (87 μmol) der Verbindung aus Beispiel 4A gelöst in 5 ml DMF werden tropfenweise über einen Zeitraum von 30 min zugesetzt. Nach 30 min Rühren bei RT wird eingeengt und der Rückstand durch Flash-Chromatographie zunächst mit Dichlormethan/Essigsäurethylester 3:1 und dann Dichlormethan/Essigsäurethylester/Methanol im Verhältnis von 150:50:5, dann 150:50:10 und schließlich im Verhältnis von 150:50:20 als Eluent gereinigt. mit Dichlormethan/Essigsäwethylester/Methanol zunächst im Verhältnis von 150:50:5 als Eluent gereinigt. Die entsprechenden Fraktionen, die noch verunreinigte Zielverbindung enthalten, werden vereinigt und im Vakuum eingeengt. Der Rückstand wird dann nochmals durch präparative HPLC (Methode 1a) aufgereinigt. Die entsprechenden Fraktionen werden eingeengt und im Hochvakuum getrocknet. Man erhält 36 mg (51% d. Th.) der geschützten Titelverbindung.
HPLC (Methode 2a): $R_t$ = 5.4 min
LC-MS (Methode 12): $R_t$ = 3.52 min; m/z = 806 $(M+H)^+$.

*Stufe b):*

36 mg (45 μmol) der geschützten Verbindung werden in Dichlormethan gelöst und mit 1.5 ml wasserfreier Trifluoressigsäure versetzt und 2 h bei RT gerührt. Dann wird im Vakuum bei 25°C oder darunter eingeengt und der Rückstand anschließend in 5 ml Salzsäure (pH 3) aufgenommen und lyophilisiert. Man erhält 28 mg (91% d. Th.) der Titelverbindung.
$^1$H-NMR (400MHz, DMSO-$d_6$): δ = 1.4 (m, 2H), 1.6 (m, 2H), 2.7 - 2.9 (m, 4H), 3.1 (m, 2H), 3.7 (t, 2H), 3.8 (dd, 1H), 4.0 (t, 2H), 4.1-4.3 (m, 5H), 4.9 (m, 1H), 7.3 (d, 1H), 7.4 (d, 2H), 7.5 (d, 2H), 7.6 (d, 1H), 8.2 (m, 3H), 8.4 (t, 1H), 13.0 (m, 1H).

**B. <u>Bestimmune von Löslichkeit, Stabilität und Freisetzunesverhalten</u>**

a) <u>Bestimmung der Löslichkeit:</u>

Die Prüfsubstanz wird in Wasser oder verdünnter Salzsäure (pH 4) suspendiert. Diese Suspension wird 24 h bei Raumtemperatur geschüttelt. Nach Ultra-Zentrifugation bei 224000g für 30 min wird der Überstand mit DMSO verdünnt und per HPLC analysiert. Quantifiziert wird über eine ZweiPunkt-Kalibrationskurve der Testverbindung in DMSO.

HPLC Methode:

Agilent 1100 mit DAD (G1315A), quat. Pumpe (G1311A), Autosampler CTC HTS PAL, Degaser (G1322A) und Säulenthermostat (G1316A); Säule: Kromasil C18, 60 x 2.1 mm, 3.5 μm; Temperatur: 30°C; Eluent A: Wasser + 5 ml Perchlorsäure/Liter, Eluent B: Acetonitril; Flussrate: 0.75 ml/min; Gradient: 0-0.5 min 98% A, 2% B; Rampe 0.5-4.5 min 10% A, 90% B; 4.5-6 min 10% A, 90% B; Rampe 6.5-6.7 min 98% A, 2% B; 6.7-7.5 min 98% A, 2% B.

In Tabelle 1 sind die Löslichkeitswerte repräsentativer Ausführungsbeispiele in verdünnter Salzsäure (pH 4) dargestellt:

Tabelle 1

| Beispiel Nr. | Löslichkeit [mg/Liter] |
|---|---|
| 3 | > 3500 |
| 7 | > 500 |
| 12 | > 3000 |
| 14 | 1900 |

Eine Zersetzung der Beispielverbindungen in diesen Lösungen wird nicht beobachtet.

Die Löslichkeit der zu Grunde liegenden Wirksubstanz [Verbindung (A)] in verdünnter Salzsäure (pH 4) wird in diesem Test mit 8.1 mg/Liter bestimmt.

b) Stabilität in Puffer bei verschiedenen pH-Werten:

0.3 mg der Testsubstanz werden in einem 2 ml-HPLC-Vial eingewogen und mit 0.5 ml Acetonitril versetzt. Zum Lösen der Substanz wird das Probengefäß für ca. 10 Sekunden ins Ultraschallbad gegeben. Anschließend werden 0.5 ml der jeweiligen Pufferlösung zugefügt und die Probe erneut im Ultraschallbad behandelt.

Eingesetzte Pufferlösungen:

pH 4.0: 1 Liter Millipore-Wasser wird mit 1N Salzsäure auf pH 4.0 eingestellt;

pH 7.4: 90 g Natriumchlorid, 13.61 g Kaliumdihydrogenphosphat und 83.35 g 1 M Natronlauge werden auf 1 Liter mit Millipore-Wasser aufgefüllt und dann 1:10 verdünnt.

Über einen Zeitraum von 24 Stunden bei 25°C werden stündlich jeweils 5 μl der Probenlösung per HPLC auf ihren Gehalt an unveränderter Testsubstanz analysiert. Quantifiziert wird über die Flächenprozente der entsprechenden Peaks.

HPLC-Methode:

Agilent 1100 mit DAD (G1314A), binärer Pumpe (G1312A), Autosampler (G1329A), Säulenofen (G1316A), Thermostat (G1330A); Säule: Kromasil 100 C18, 60 mm x 2.1 mm, 3.5 μm; Säulentemperatur: 30°C; Eluent A: Wasser + 5 ml Perchlorsäure/Liter, Eluent B: Acetonitril; Gradient: 0-1.0 min 98% A, 2% B; 1.0-9.0 min 2% A, 98% B; 9.0-13.0 min 2% A, 98% B; 13.0-13.5 min 98% A, 2% B; 13.5-15.0 98% A, 2% B; Flussrate: 0.75 ml/min; UV-Detektion: 210 nm.

In Tabelle 2 sind zu repräsentativen Ausführungsbeispielen die Verhältnisse der Peakflächen (F) zu den jeweiligen Zeitpunkten im Verhältnis zu den Peakflächen beim Startzeitpunkt dargestellt:

Tabelle 2

| Beispiel Nr. | Puffer pH | % Testsubstanz nach 4 h [F(t=4h) x 100 / F(t=0h)] | % Testsubstanz nach 24 h [F(t=24h) x 100 / F(t=0h)] |
|---|---|---|---|
| 2 | 4 | 101 | 101 |
| 2 | 7.4 | 99 | 94 |
| 3 | 4 | 100 | 101 |
| 3 | 7.4 | 100 | 100 |
| 4 | 4 | 100 | 100 |
| 4 | 7.4 | 92 | 77 |

(fortgesetzt)

| Beispiel Nr. | Puffer pH | % Testsubstanz nach 4 h W(t=4h) x 100 / F(t=0h)] | % Testsubstanz nach 24 h [F(t=24h) x 100 / F(t=0h)] |
|---|---|---|---|
| 5 | 4 | 100 | 100 |
| 5 | 7.4 | 96 | 84 |
| 6 | 4 | 99 | 99 |
| 6 | 7.4 | 90 | 53 |
| 7 | 4 | 100 | 101 |
| 7 | 7.4 | 97 | 84 |
| 8 | 4 | 100 | 100 |
| 8 | 7.4 | 86 | 48 |
| 10 | 4 | 100 | 101 |
| 10 | 7.4 | 100 | 100 |
| 11 | 4 | 101 | 101 |
| 11 | 7.4 | 100 | 99 |
| 12 | 4 | 100 | 101 |
| 12 | 7.4 | 99 | 93 |
| 14 | 4 | 100 | 100 |
| 14 | 7.4 | 98 | 88 |

In diesem Test wird bei pH 7.4 gleichzeitig mit der Abnahme des Gehalts an Testsubstanz eine Zunahme der Wirkstoff-Verbindung (A) festgestellt.

c) *In vitro*-Stabilität in Ratten- und Humanplasma *):

Ein definiertes Plasmavolumen (z.B. 2.0 ml) wird in einem geschlossenen Reagenzglas im Wasserbad auf 37°C erwärmt. Nach Erreichen der Soll-Temperatur wird eine definierte Menge der Prüfsubstanz als Lösung zugegeben (Volumen des Lösungsmittels max. 2% des Plasmavolumens). Das Plasma wird geschüttelt und eine erste Probe (50-100 μl) sofort entnommen. Im Zeitraum bis 2 h nach Inkubationsbeginn werden anschließend 4-6 weitere Aliquote entnommen.

Die Plasmaproben werden zur Proteinfällung mit Acetonitril versetzt. Nach Zentrifugation werden Prüfsubstanz und gegebenenfalls bekannte Spaltprodukte der Prüfsubstanz im Überstand mit einer geeigneten LC/MS-MS-Methode quantitativ bestimmt.

*) Stabilitätsbestimmungen in heparinisiertem Blut (Ratten- oder Humanblut) werden - wie für Plasma beschrieben - durchgefiihrt

d) Bestimmung der metabolischen Stabilität in Hepatozyten:

Metabolische Stabilitäten neuer Testverbindungen gegenüber Hepatozyten werden bestimmt, indem die Verbindungen bei niedrigen Konzentrationen (bevorzugt unter 1uM) und bei niedrigen Zellzahlen (bevorzugt bei 110^6Zellen/ml) inkubiert werden, um möglichst lineare kinetische Bedingungen im Versuch sicherzustellen. Sieben Proben aus der Inkubationslösung werden in einem festgelegten Zeitraster für die LC-MS Analytik entnommen, um die Halbwertszeit (den Abbau) der Verbindung zu bestimmen. Aus dieser Halbwertszeit werden unterschiedliche "Clearance-Parameter (CL)" (s.u.) und "Fmax"- Werte (s.u.) berechnet.

Die CL und Fmax - Werte stellen ein Maß für den "Phase 1" und "Phase 2" Metabolismus der Verbindung in den Hepatozyten dar. Um den Einfluss des organischen Lösungsmittels auf die Enzyme in den Inkubationsansätzen möglichst klein zu halten, werden deren Konzentrationen im allgemeinen auf 1% (Acetonitril) bzw. auf 0.1% (DMSO) begrenzt.

Für alle Spezies und Rassen wird mit einer Hepatozytenzellzahl in der Leber von 1.1*10^8 Zellen / g Leber

gerechnet. CL Parameter deren Berechnung auf Halbwertszeiten beruhen, die über die Inkubationszeit hinausgehen (üblicherweise 90 Minuten), können nur als grobe Richtwerte angesehen werden.

Die berechneten Parameter und deren Bedeutung sind:
(QH = speziesspezifischerLeberblutfluss)

| | |
|---|---|
| **Fmax-well-stirred [%]:** | maximal mögliche Bioverfügbarkeit nach oraler Applikation |
| Berechnung: | $(1\text{-}CL_{blood}\text{ well-stirred }/QH)*100$ |
| **$CL_{blood}$ well-stirred [L/(h*kg)]:** | berechnete Blut Clearance (Well-stirred Modell) |
| Berechnung: | $(QH*\ CL'_{intrinsic}) / (QH+ CL'_{intrinsic})$ |
| **$CL'_{intrinsic}$ [ml/(min*kg)]:** | maximale Fähigkeit der Leber (der Hepatozyten) eine Verbindung zu metabolisieren, unter der Annahme, dass der Leberblutfluss nicht geschwindigkeitslimitierend ist |
| Berechnung: | $CL'_{intrinsic,apparent}$ * speziesspezifischer Hepatozytenzahl [1.1*10^8/g Leber]* speziesspezifischem Lebergewicht [g/kg] |
| **$CL'_{intrinsic,apparent}$ [ml/(min*mg)]:** | "normiert" die Eliminationskonstante, indem diese durch die eingesetzte Hepatozytenzellzahl x (x*10^6/ml) dividiert wird |
| Berechnung: | $k_{el}$ [1/min] / (Zellzahl [x*10^6] / Inkubationsvolumen [ml]) |

e) <u>i.v.-Pharmakokinetik in Wistar-Ratten:</u>

Am Tag vor der Substanzgabe wird den Versuchstieren (männliche Wistar-Ratten, Körpergewicht 200-250 g) unter Isofluran®-Narkose ein Katheter für die Blutgewinnung in die Vena Jugularis implantiert.

Am Versuchstag wird eine definierte Dosis der Prüfsubstanz als Lösung mit einer Hamilton®-Glasspritze in die Schwanzvene appliziert (Bolusgabe, Applikationsdauer <10 s). Innerhalb von 24 h nach Substanzgabe werden sequentiell Blutproben (8-12 Zeitpunkte) über den Katheter entnommen. Zur Plasmagewinnung werden die Proben in heparinisierten Röhrchen zentrifugiert. Pro Zeitpunkt wird ein definiertes Plasmavolumen zur Proteinfällung mit Acetonitril versetzt. Nach Zentrifugation werden Prüfsubstanz und gegebenenfalls bekannte Spaltprodukte der Prüfsubstanz im Überstand mit einer geeigneten LC/MS-MS-Methode quantitativ bestimmt.

Die Berechnung pharmakokinetischer Kenngrößen der Prüfsubstanz bzw. der daraus freigesetzten Wirkstoff-Verbindung (A) wie AUC, $C_{max}$, $T_{1/2}$ (Halbwertszeit) und CL (Clearance) erfolgt aus den gemessenen Plasmakonzentrationen.

f) <u>Bestimmung der antithrombotischen Wirkung in einem arteriovenösen Shunt-Modell in Ratten:</u>

Nüchterne männliche Ratten (Stamm: HSD CPB:WU) werden durch intraperitoneale Gabe einer Rompun/Ketavet-Lösung narkotisiert (12 mg/kg / 50 mg/kg). Die Thrombusbildung wird in einem arteriovenösen Shunt in Anlehnung an die von P.C. Wong et al. beschriebene Methode [Thrombosis Research 83 (2), 117-126 (1996)] ausgelöst. Dazu werden die linke Vena jugularis und die rechte Arteria carotis freipräpariert. In die Arterie wird ein 8 cm langer Polyethylenkatheter (PE60, Fa. Becton-Dickinson), gefolgt von einem 6 cm langen Tygonschlauch (R-3606, ID 3.2 mm, Fa. Kronlab), der zur Erzeugung einer thrombogenen Oberfläche einen aufgerauhten und zu einer Doppelschlinge gelegten Nylonfaden (60 x 0.26 mm, Fa. Berkley Trilene) enthält, eingebunden. In die Vena jugularis wird ein 2 cm langer Polyethylenkatheter (PE60, Fa. Becton-Dickinson) eingebunden und über einen 6 cm langen Polyethylenkatheter (PE160, Fa. Becton-Dickinson) mit dem Tygonschlauch verbunden. Die Schläuche werden mit physiologischer Kochsalzlösung vor Öffnung des Shuntes gefüllt. Der extrakorporale Kreislauf wird 15 min lang aufrechterhalten. Dann wird der Shunt entfernt und der Nylonfaden mit dem Thrombus sofort gewogen. Das Leergewicht des Nylonfadens ist vor Versuchsbeginn ermittelt worden. Die Prüfsubstanz (als Lösung in physiologischer Kochsalzlösung, die mit 0.1 N Salzsäure auf pH 4 eingestellt ist) wird vor Anlegung des extrakorporalen Kreislaufs als Bolus-Injektion verabreicht.

**C. <u>Ausführungsbeispiele für pharmazeutische Zusammensetzungen</u>**

Die erfindungsgemäßen Verbindungen können beispielsweise folgendermaßen in pharmazeutische Zubereitungen überführt werden:

**i.v.-Lösung:**

Die erfindungsgemäße Verbindung wird in einer Konzentration unterhalb der Sättigungslöslichkeit in einem physiologisch verträglichen Lösungsmittel (z.B. isotonische Kochsalzlösung, Glucoselösung 5% und/oder PEG 400-Lösung 30%, die jeweils auf einen pH-Wert von 3-5 eingestellt sind) gelöst. Die Lösung wird gegebenenfalls steril filtriert und/oder in sterile und pyrogenfreie Injektionsbehältnisse abgefüllt.

## Patentansprüche

1. Verbindung der Formel

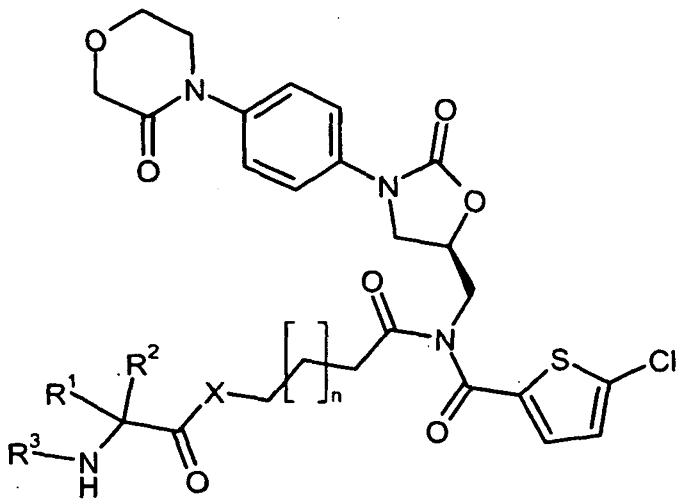

(I),

in welcher

n für die Zahl 1 oder 2 steht,
X für ein Sauerstoffatom, Schwefelatom oder NH steht,
$R^1$ für die Seitengruppe einer natürlichen $\alpha$-Aminosäure oder ihrer Homologe oder Isomere steht,
$R^2$ für Wasserstoff oder Methyl steht,
$R^3$ für Wasserstoff steht,
oder
$R^1$ und $R^3$ sind über eine $(CH_2)_3$- oder $(CH_2)_4$-Gruppe verknüpft und bilden zusammen mit dem Stickstoff- bzw. Kohlenstoffatom, an das sie gebunden sind einen 5- bzw. 6-Ring,

sowie ihre Salze, Solvate und Solvate der Salze.

2. Verbindung der Formel (I) nach Anspruch 1, in welcher

n für die Zahl 1 oder 2 steht,
X für ein Sauerstoffatom, Schwefelatom oder NH steht,
$R^1$ für Wasserstoff, Methyl, Propan-2-yl, Propan-1-yl, 2-Methylpropan-1-yl, Imidazol-4-ylmethyl, Hydroxymethyl, 1-Hydroxyethyl, Carboxymethyl, 2-Carboxyethyl, Carbamoylmethyl, 2-Carbamoylethyl, 4-Aminobutan-1-yl, 3-Aminopropan-1-yl, 3-Guanidinopropan-1-yl, Benzyl oder 4-Hydroxybenzyl steht,
$R^2$ für Wasserstoff oder Methyl steht,
$R^3$ für Wasserstoff steht,
oder
$R^1$ und $R^3$ sind über eine $(CH_2)_3$- oder $(CH_2)_4$-Gruppe verknüpft und bilden zusammen mit dem Stickstoff- bzw. Kohlenstoffatom, an das sie gebunden sind einen 5- bzw. 6-Ring,

sowie ihre Salze, Solvate und Solvate der Salze.

**3.** Verbindung der Formel (I) nach Anspruch 1 oder 2, in welcher

n für die Zahl 1 oder 2 steht,
X für NH steht,
$R^1$ für Wasserstoff, Methyl, Propan-2-yl, 2-Methylpropan-1-yl, Imidazol-4-ylmethyl, Hydroxymethyl, 1-Hydroxyethyl, Carboxymethyl, 2-Carboxyethyl, Carbamoylmethyl, 2-Carbamoylethyl, 4-Aminobutan-1-yl, Benzyl oder 4-Hydroxybenzyl steht,
$R^2$ für Wasserstoff steht,
$R^3$ für Wasserstoff steht,

sowie ihre Salze, Solvate und Solvate der Salze.

**4.** Verfahren zur Herstellung einer Verbindung der Formel (I) oder eines ihrer Salze, ihrer Solvate oder der Solvate ihrer Salze nach Anspruch 1, **dadurch gekennzeichnet, dass**

[A] eine Verbindung der Formel

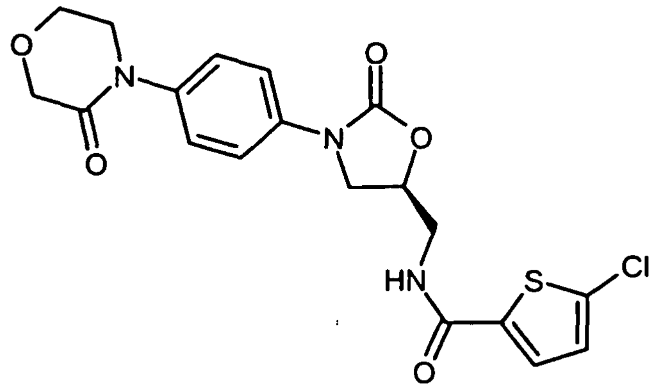

(A)

zunächst in einem inerten Lösungsmittel in Gegenwart einer Base mit einer Verbindung der Formel

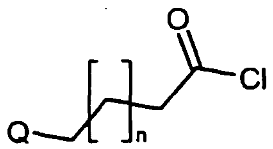

(II),

in welcher n die in Anspruch 1 angegebene Bedeutung hat,
und
Q für eine Abgangsgruppe wie beispielsweise Chlor, Brom oder Iod steht, in eine Verbindung der Formel

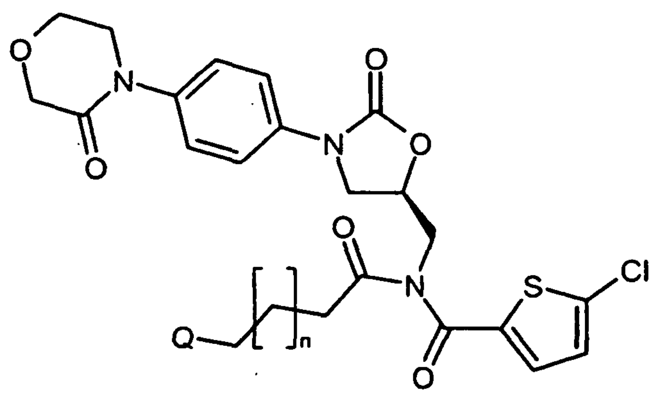

(III),

in welcher n die in Anspruch 1 angegebene Bedeutung hat, und
Q die in diesem Anspruch angegebene Bedeutung hat, überführt, diese dann nach Verfahren

[A1] in einem inerten Lösungsmittel mit dem Caesiumsalz einer α-Aminocarbonsäure oder einer α-Aminothiocarbonsäure der Formel

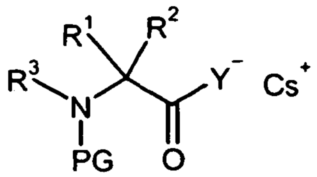

(IV),

in welcher $R^1$, $R^2$ und $R^3$ die in Anspruch 1 angegebene Bedeutung haben,

PG für eine Amino-Schutzgruppe wie beispielsweise tert.-Butoxycarbonyl (Boc) oder Benzyloxycarbonyl (Z) steht und
Y für O oder S steht,

zu einer Verbindung der Formel

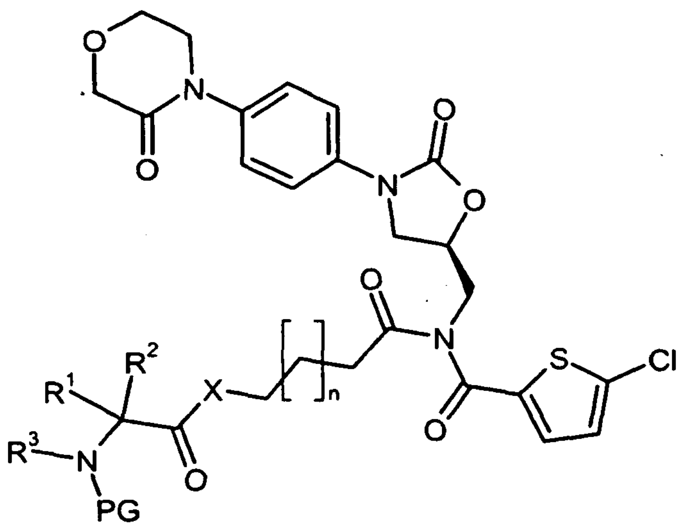

(V),

in welcher n, $R^1$, $R^2$ und $R^3$ die in Anspruch 1 angegebene Bedeutung haben, PG die in diesem Anspruch angegebene Bedeutung hat, und

X für O oder S steht,

umsetzt und nachfolgend die Schutzgruppe PG nach üblichen Methoden unter Erhalt einer Verbindung der Formel

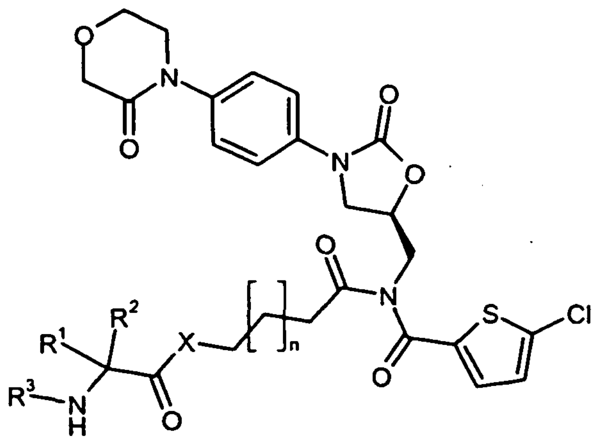

(I-A),

in welcher n, $R^1$, $R^2$ und $R^3$ die in Anspruch 1 angegebene Bedeutung haben, und

X für O oder S steht, entfernt, oder

[A2] in einem inerten Lösungsmittel in Gegenwart einer Base mit einer α-Aminothiocarbonsäure der Formel

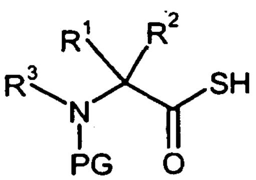

(VI),

in welcher $R^1$, $R^2$ und $R^3$ die in Anspruch 1 angegebene Bedeutung haben, PG für eine Amino-Schutzgruppe wie beispielsweise tert.-Butoxycarbonyl (Boc) oder Benzyloxycarbonyl (Z) steht, zu einer Verbindung der Formel

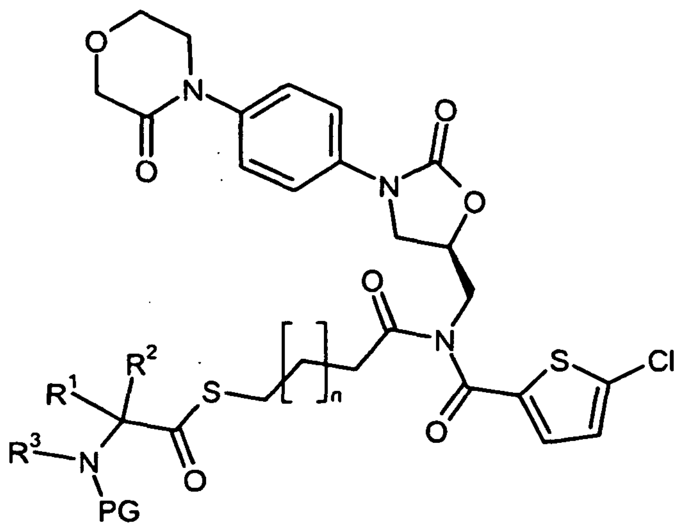

(V-A),

in welcher n, $R^1$, $R^2$ und $R^3$ die in Anspruch 1 angegebene Bedeutung haben, und PG die in diesem Anspruch angegebene Bedeutung hat, umsetzt und nachfolgend die Schutzgruppe PG nach üblichen Methoden unter Erhalt einer Verbindung der Formel

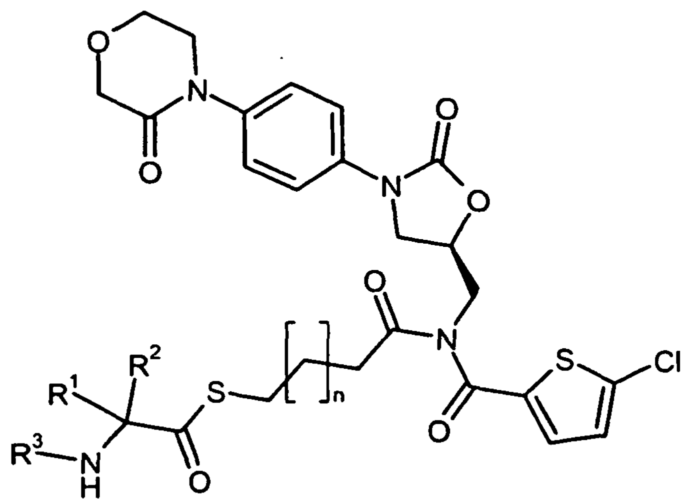

(I-A),

in welcher n, $R^1$, $R^2$ und $R^3$ die in Anspruch 1 angegebene Bedeutung haben, entfernt oder

[B] Verbindung (A) in einem inerten Lösungsmittel in Gegenwart einer Base mit einer Verbindung der Formel

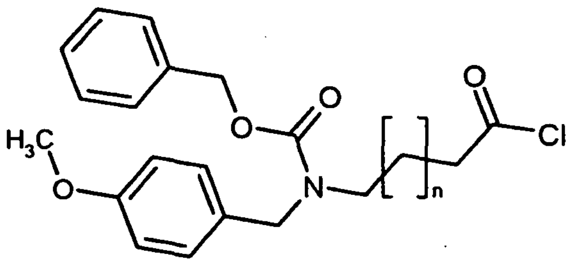

(VII),

in welcher n die in Anspruch 1 angegebene Bedeutung hat, zu einer Verbindung der Formeln

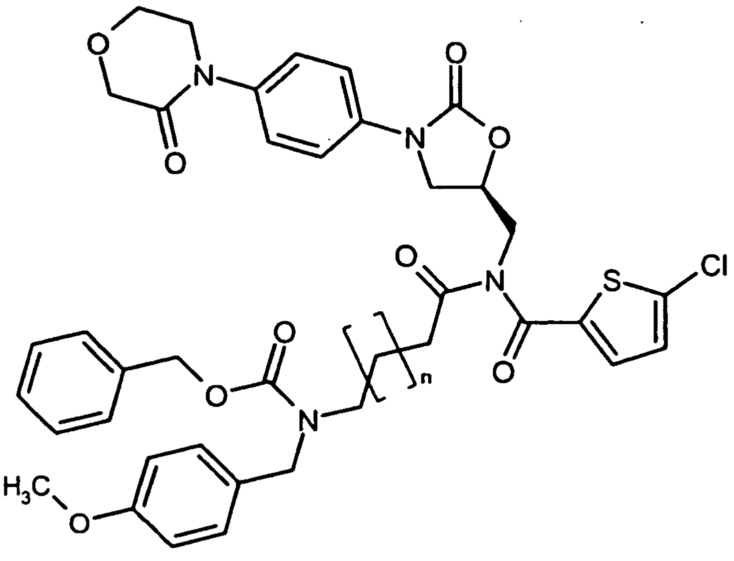

(VIII),

in welcher n die in Anspruch 1 angegebene Bedeutung hat,
umsetzt, anschließend die Schutzgruppen nach üblichen Methoden unter Erhalt einer Verbindung der Formel

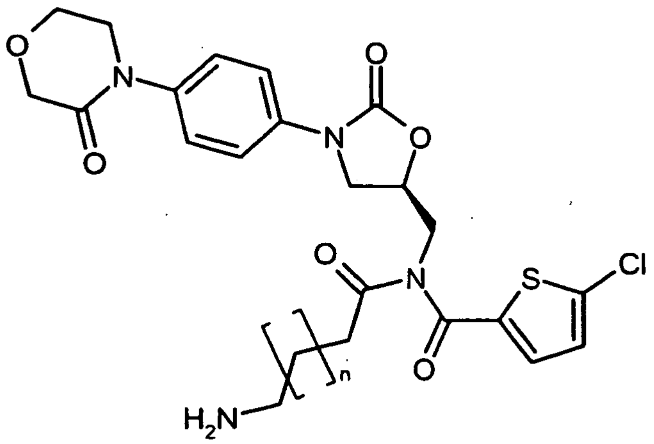

(IX),

in welcher n die in Anspruch 1 angegebene Bedeutung hat, entfernt und dann in Gegenwart einer Base mit einer Verbindung der Formel

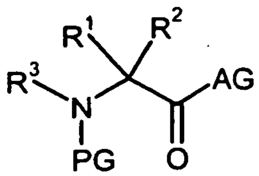

(X),

in welcher $R^1$, $R^2$ und $R^3$ die in Anspruch 1 angegebene Bedeutung haben,

AG für Hydroxy oder Halogen, bevorzugt Chlor oder Brom, steht oder zusammen mit der Carbonylgruppe einen Aktivester, bevorzugt einen N-Hydoxysuccinimidester, oder ein gemischtes Anhydrid, bevorzugt einen Kohlensäurealkylester, besonders bevorzugt einen Kohlensäureethylester, bildet, und
PG für eine Amino-Schutzgruppe wie beispielsweise tert.-Butoxycarbonyl (Boc) oder Benzyloxycarbonyl (Z) steht,

unter Erhalt einer Verbindung der Formel

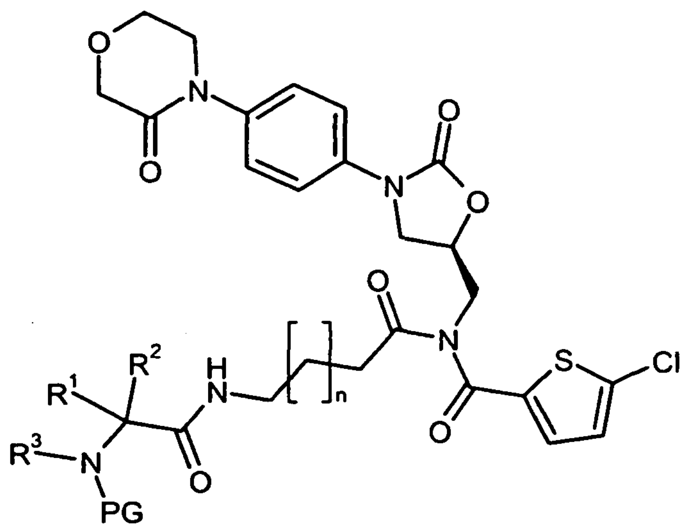

(XI)

in welcher n, $R^1$, $R^2$ und $R^3$ die in Anspruch 1 angegebene Bedeutung haben, und

PG die in diesem Anspruch angegebene Bedeutung hat, umsetzt, und anschließend die Schutzgruppe PG nach üblichen Methoden unter Erhalt einer Verbindung der Formel

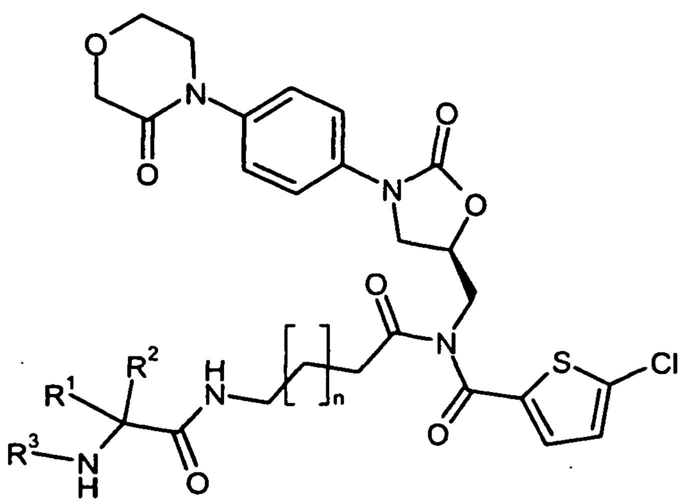

(I-B),

in welcher n, $R^1$, $R^2$ und $R^3$ die in Anspruch 1 angegebene Bedeutung haben, entfernt und die jeweils resultierenden Verbindungen der Formel (I-A) bzw. (I-B) gegebenenfalls mit den entsprechenden (i) Lösungsmitteln und/oder (ii) Säuren in ihre Solvate, Salze und/oder Solvate der Salze überführt.

**5.** Verbindung der Formel (I), wie in einem der Ansprüche 1 bis 3 definiert, zur Behandlung und/oder Prophylaxe von Krankheiten.

**6.** Verwendung einer Verbindung der Formel (I), wie in einem der Ansprüche 1 bis 3 definiert, zur Herstellung eines Arzneimittels zur Behandlung und/oder Prophylaxe von thromboembolischen Erkrankungen.

**7.** Arzneimittel enthaltend eine Verbindung der Formel (I), wie in einem der Ansprüche 1 bis 3 definiert, gegebenenfalls in Kombination mit einem inerten, nicht-toxischen, pharmazeutisch geeigneten Hilfsstoff.

**8.** Arzneimittel enthaltend eine Verbindung der Formel (I), wie in einem der Ansprüche 1 bis 3 definiert, in Kombination mit einem weiteren Wirkstoff.

**9.** Arzneimittel nach Anspruch 7 oder 8 zur Behandlung und/oder Prophylaxe von thromboembolischen Erkrankungen.

**10.** Arzneimittel nach einem der Ansprüche 7 bis 9 zur intravenösen Anwendung.

## Claims

**1.** Compound of the formula

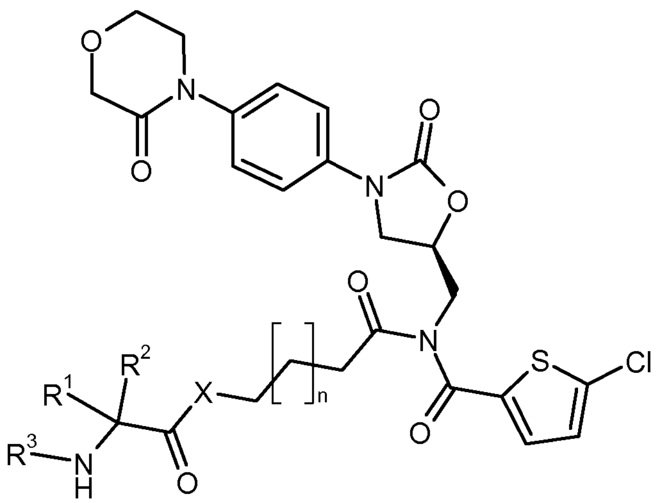

(I),

in which

n is 1 or 2,
X is an oxygen atom, a sulphur atom or NH,
$R^1$ is the side group of a natural $\alpha$-amino acid or of its homologs or isomers,
$R^2$ is hydrogen or methyl,
$R^3$ is hydrogen, or
$R^1$ and $R^3$ are linked via a $(CH_2)_3$ or $(CH_2)_4$ group and combine with the nitrogen or carbon atom to which they are attached to form a 5- or 6-membered ring, respectively,

and also its salts, solvates and solvates of the salts.

**2.** Compound of the formula (I) according to Claim 1, in which

n is 1 or 2,
X is an oxygen atom, a sulphur atom or NH,
$R^1$ is hydrogen, methyl, propan-2-yl, propan-1-yl, 2-methylpropan-1-yl, imidazol-4-ylmethyl, hydroxymethyl, 1-hydroxyethyl, carboxymethyl, 2-carboxyethyl, carbamoylmethyl, 2-carbamoylethyl, 4-aminobutan-1-yl, 3-aminopropan-1-yl, 3-guanidinopropan-1-yl, benzyl or 4-hydroxybenzyl,
$R^2$ is hydrogen or methyl,
$R^3$ is hydrogen, or
$R^1$ and $R^3$ are linked via a $(CH_2)_3$ or $(CH_2)_4$ group and combine with the nitrogen or carbon atom to which they are attached to form a 5- or 6-membered ring, respectively,

and also its salts, solvates and solvates of the salts.

**3.** Compound of the formula (I) according to Claim 1 or 2, in which

n is 1 or 2,
X is NH,
$R^1$ is hydrogen, methyl, propan-2-yl, 2-methylpropan-1-yl, imidazol-4-ylmethyl, hydroxymethyl, 1-hydroxyethyl, carboxymethyl, 2-carboxyethyl, carbamoylmethyl, 2-carbamoylethyl, 4-aminobutan-1-yl, benzyl or 4-hydroxybenzyl,
$R^2$ is hydrogen,
$R^3$ is hydrogen,

and also its salts, solvates and solvates of the salts.

**4.** Process for preparing a compound of the formula (I) or one of its salts, its solvates or the solvates of its salts according to Claim 1, **characterized in that**

[A] a compound of the formula

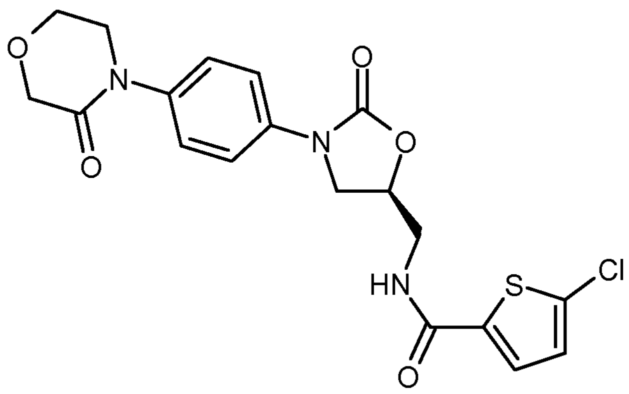

(A)

is initially converted in an inert solvent in the presence of a base with a compound of the formula

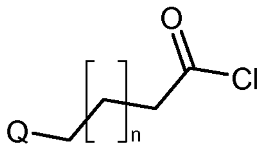

(II),

in which n has the meaning indicated in Claim 1, and

Q is a leaving group such as, for example, chlorine, bromine or iodine,

into a compound of the formula

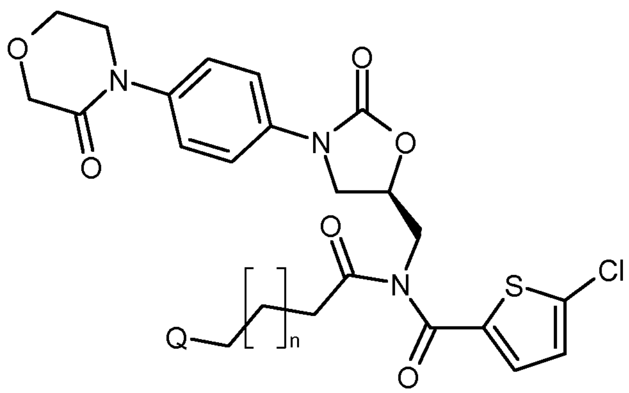

(III),

in which n has the meaning indicated in Claim 1, and Q has the meaning indicated in this claim, the latter is then reacted according to process

[A1] in an inert solvent with the caesium salt of an α-amino carboxylic acid or α-amino thiocarboxylic acid of the formula

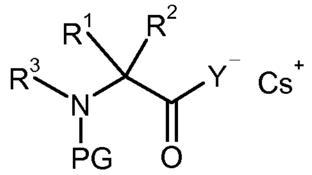

(IV),

in which $R^1$, $R^2$ and $R^3$ have the meaning indicated in Claim 1,

PG is an amino protective group such as, for example, *tert*-butoxycarbonyl (Boc) or benzyloxycarbonyl (Z), and
Y is O or S,

to give a compound of the formula

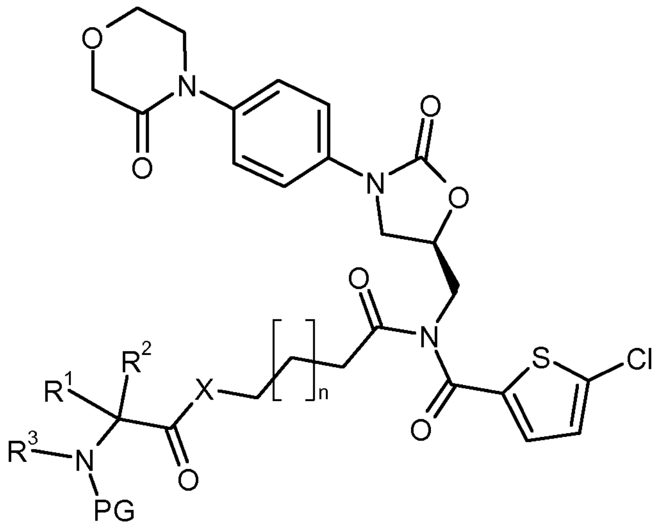

(V),

in which n, $R^1$, $R^2$ and $R^3$ have the meaning indicated in Claim 1, PG has the meaning indicated in this claim, and

X is O or S,

and subsequently the protective group PG is removed according to conventional methods to obtain a compound of the formula

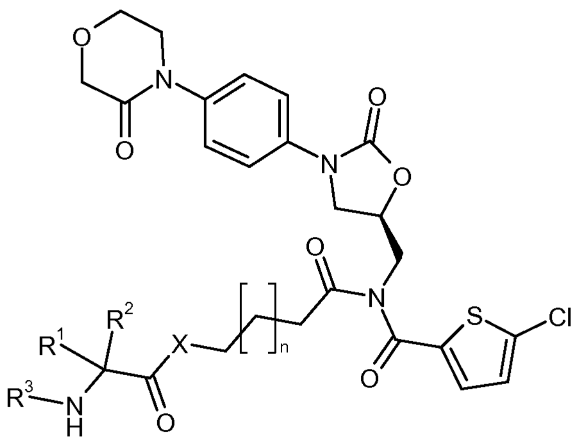

(I-A),

in which n, $R^1$, $R^2$ and $R^3$ have the meaning indicated in Claim 1, and

X is O or S, or

[A2] in an inert solvent in the presence of a base with an α-amino thiocarboxylic acid of the formula

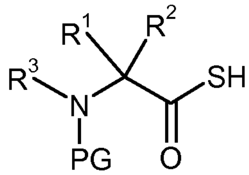

(VI),

in which $R^1$, $R^2$ and $R^3$ have the meaning indicated in Claim 1,

PG is an amino protective group such as, for example, *tert*-butoxycarbonyl (Boc) or benzyloxycarbonyl (Z), to give a compound of the formula

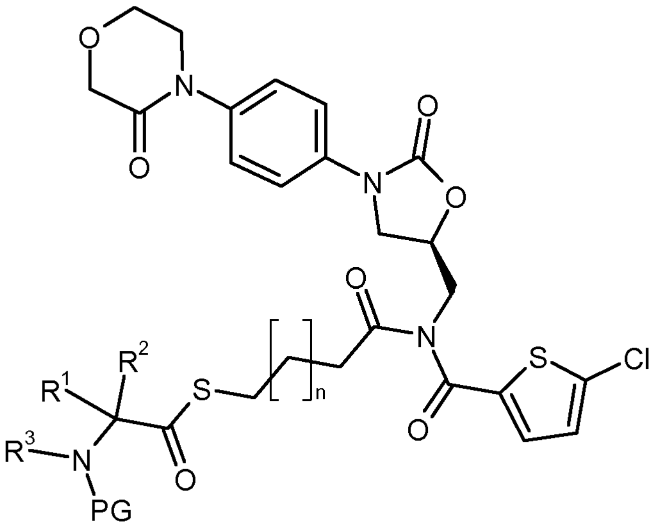

(V-A),

in which n, $R^1$, $R^2$ and $R^3$ have the meaning indicated in Claim 1, and PG has the meaning indicated in this claim, and subsequently the protective group PG is removed according to conventional methods to obtain a compound of the formula

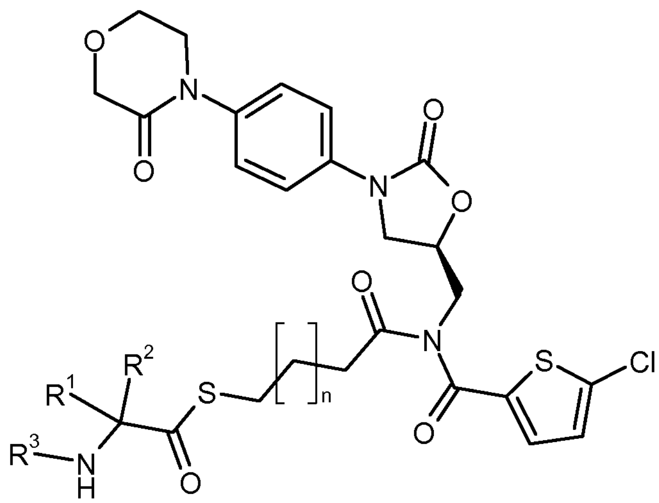

(I-A),

in which n, $R^1$, $R^2$ and $R^3$ have the meaning indicated in Claim 1, or

[B] compound (A) is reacted in an inert solvent in the presence of a base with a compound of the formula

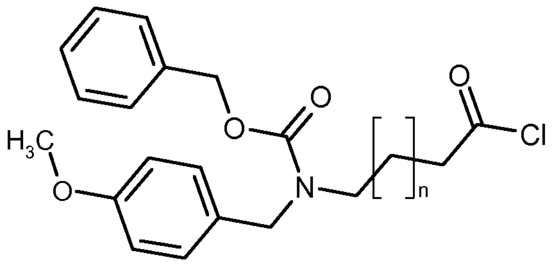

(VII),

in which n has the meaning indicated in Claim 1,
to give a compound of the formula

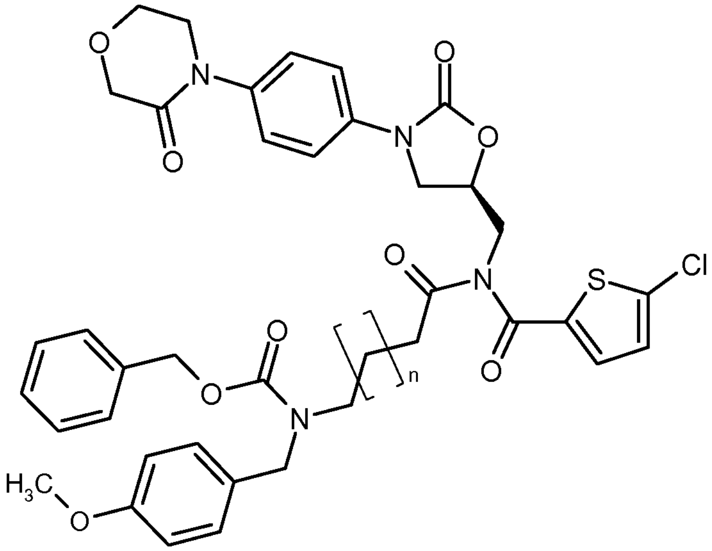

(VIII),

in which n has the meaning indicated in Claim 1,
subsequently the protective groups are removed according to conventional methods to obtain a compound of the formula

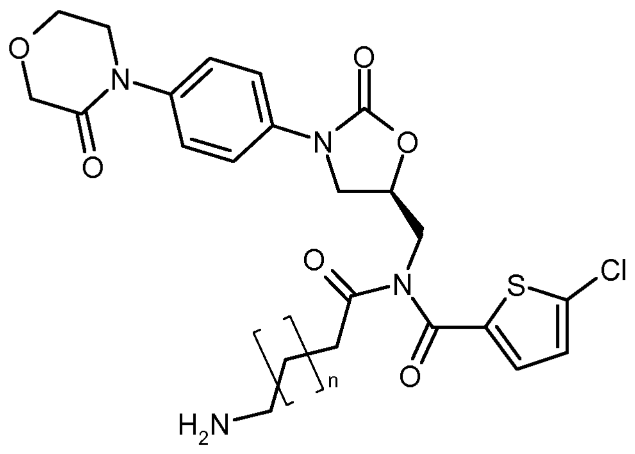

(IX),

in which n has the meaning indicated in Claim 1, and then in the presence of a base with a compound of the formula

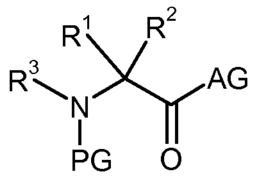

(X),

in which $R^1$, $R^2$ and $R^3$ have the meaning indicated in Claim 1,

AG is hydroxyl or halogen, preferably chlorine or bromine, or combines with the carbonyl group to form an active ester, preferably an N-hydoxysuccinimide ester, or a mixed anhydride, preferably an alkyl carbonate, more preferably an ethyl carbonate, and
PG is an amino protective group such as, for example, tert-butoxycarbonyl (Boc) or benzyloxycarbonyl (Z),

to obtain a compound of the formula

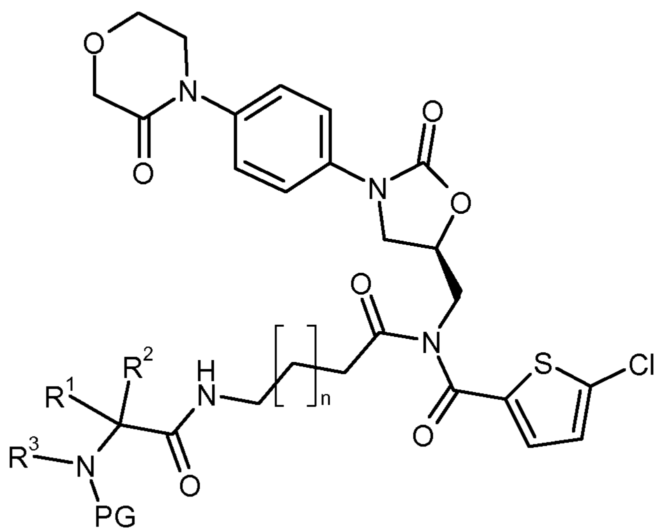

(XI)

in which n, $R^1$, $R^2$ and $R^3$ have the meaning indicated in Claim 1, and
PG has the meaning indicated in this claim,
and subsequently the protective group PG is removed according to conventional methods to obtain a compound of the formula

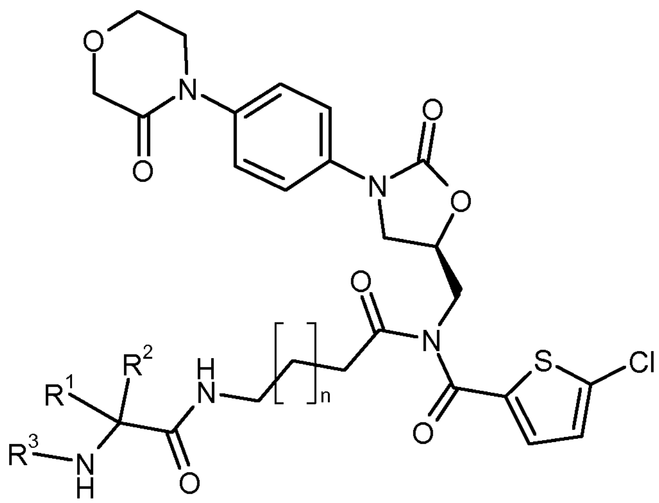

(I-B),

in which n, $R^1$, $R^2$ and $R^3$ have the meaning indicated in Claim 1 and the compounds of the formula (I-A) or (I-B) resulting in each case are converted where appropriate with the appropriate (i) solvents and/or (ii) acids into their solvates, salts and/or solvates of the salts.

**5.** Compound of the formula (I) as defined in any of Claims 1 to 3 for the treatment and/or prophylaxis of diseases.

**6.** Use of a compound of the formula (I) as defined in any of Claims 1 to 3 for the manufacture of a medicament for the treatment and/or prophylaxis of thromboembolic disorders.

**7.** Medicament comprising a compound of the formula (I) as defined in any of Claims 1 to 3, where appropriate in combination with an inert, non-toxic, pharmaceutically suitable excipient.

**8.** Medicament comprising a compound of the formula (I) as defined in any of Claims 1 to 3 in combination with a further active ingredient.

**9.** Medicament according to Claim 7 or 8 for the treatment and/or prophylaxis of thromboembolic disorders.

**10.** Medicament according to any of Claims 7 to 9 for intravenous use.

**Revendications**

**1.** Composé de formule

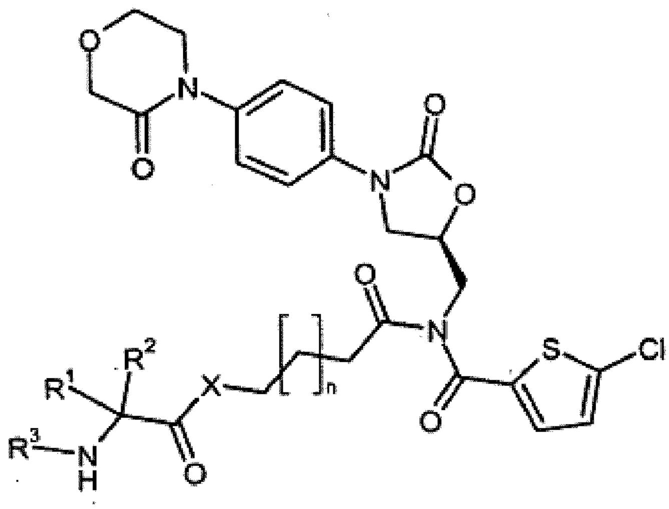

(I),

dans laquelle

n représente le nombre 1 ou 2,
X représente un atome d'oxygène, un atome de soufre ou NH,
$R^1$ représente le groupe latéral d'un $\alpha$-aminoacide naturel ou de ses homologues ou isomères,
$R^2$ représente un atome d'hydrogène ou le groupe méthyle,
$R^3$ représente un atome d'hydrogène, ou
$R^1$ et $R^3$ sont reliés par un groupe $(CH_2)_3$ ou $(CH_2)_4$ et forment ensemble avec l'atome d'azote ou respectivement de carbone auquel ils sont liés un cycle pentagonal ou hexagonal,

ainsi que ses sels, produits de solvatation et produits de solvatation des sels.

**2.** Composé de formule (I) selon la revendication 1, dans lequel

n représente le nombre 1 ou 2,
X représente un atome d'oxygène, un atome de soufre ou NH,
$R^1$ représente un atome d'hydrogène, le groupe méthyle, propan-2-yle, propan-1-yle, 2-méthyl-propan-1-yle, imidazol-4-ylméthyle, hydroxyméthyle, 1-hydroxyéthyle, carboxyméthyle, 2-carboxyéthyle, carbamoylméthyle, 2-carbamoyléthyle, 4-aminobutan-1-yle, 3-aminopropan-1-yle, 3-guanidinopropan-1-yle, benzyle ou 4-hydroxy-benzyle,
$R^2$ représente un atome d'hydrogène ou le groupe méthyle,
$R^3$ représente un atome d'hydrogène, ou
$R^1$ et $R^3$ sont reliés par un groupe $(CH_2)_3$ ou $(CH_2)_4$ et forment ensemble avec l'atome d'azote ou respectivement de carbone auquel ils sont liés un cycle pentagonal ou hexagonal,

ainsi que ses sels, produits de solvatation et produits de solvatation des sels.

**3.** Composé de formule (I) selon la revendication 1 ou 2, dans lequel

n représente le nombre 1 ou 2,
X représente NH,
$R^1$ représente un atome d'hydrogène, le groupe méthyle, propan-2-yle, 2-méthylpropan-1-yle, imidazol-4-ylméthyle, hydroxyméthyle, 1-hydroxyéthyle, carboxyméthyle, 2-carboxyéthyle, carbamoylméthyle, 2-carbamoyléthyle, 4-aminobutan-1-yle, benzyle ou 4-hydroxybenzyle,
$R^2$ représente un atome d'hydrogène,
$R^3$ représente un atome d'hydrogène,

ainsi que ses sels, produits de solvatation et produits de solvatation des sels.

**4.** Procédé pour la préparation d'un composé de formule (I) ou d'un de ses sels, de ses produits de solvatation ou des

produits de solvatation de ses sels selon la revendication 1, **caractérisé en ce que**

[A] d'abord on convertit un composé de formule

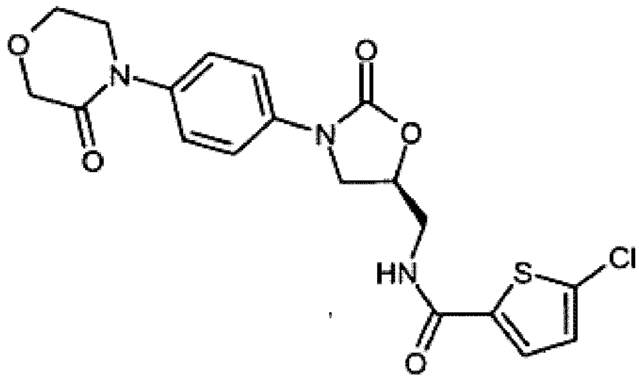

(A)

dans un solvant inerte, en présence d'une base, à l'aide d'un composé de formule

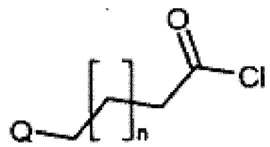

(II),

dans laquelle n a la signification indiquée dans la revendication 1,
et

Q représente un groupe partant, comme par exemple un atome de chlore, brome ou iode,

en un composé de formule

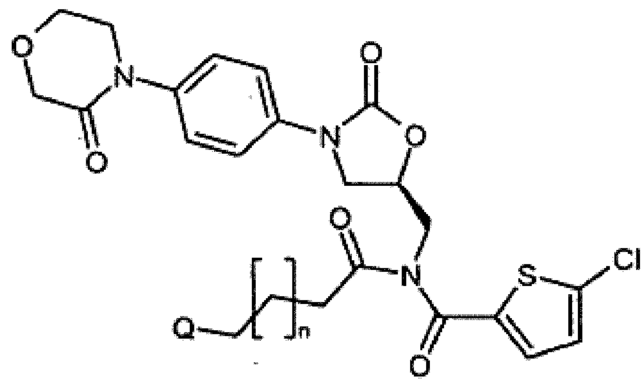

(III),

dans laquelle n a la signification indiquée dans la revendication 1, et

Q a la signification indiquée dans la présente revendication,

puis selon le procédé

[A1] on le fait réagir dans un solvant inerte, avec le
sel de césium d'un acide $\alpha$-aminocarboxylique ou d'un acide $\alpha$-aminothiocarboxylique de formule

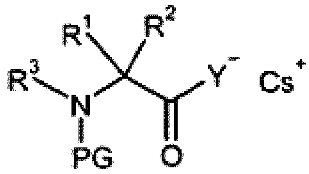 (IV),

dans laquelle $R^1$, $R^2$ et $R^3$ ont la signification indiquée dans la revendication 1,

PG représente un groupe protecteur de fonction amino, comme par exemple le groupe tert-butoxycarbonyle (Boc) ou benzyloxycarbonyle (Z) et
Y représente O ou S,

pour obtenir un composé de formule

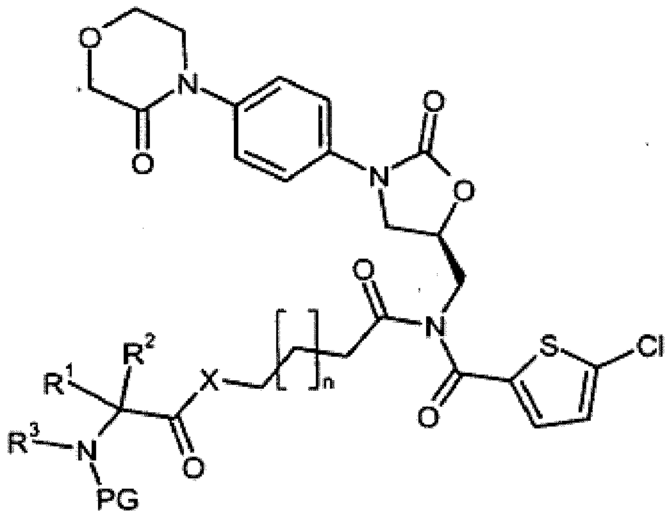 (V),

dans laquelle n, $R^1$, $R^2$ et $R^3$ ont la signification indiquée dans la revendication 1,

PG a la signification indiquée dans la présente revendication, et
X représente O ou S,

et ensuite on élimine selon des méthodes usuelles le groupe protecteur PG, pour obtenir un composé de formule

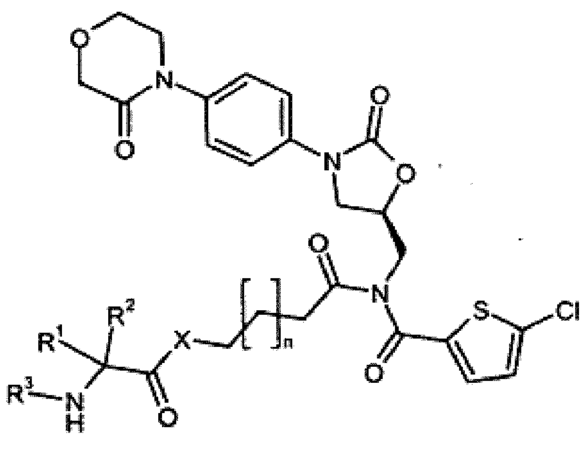 (I-A),

dans laquelle n, $R^1$, $R^2$ et $R^3$ ont la signification indiquée dans la revendication 1, et

X représente O ou S, ou

[A2] on le fait réagir dans un solvant inerte, en présence d'une base, avec un acide α-aminothiocarboxylique de formule

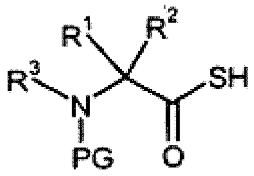

(VI),

dans laquelle $R^1$, $R^2$ et $R^3$ ont la signification indiquée dans la revendication 1,

PG représente un groupe protecteur de fonction amino, comme par exemple le groupe tert-butoxycarbonyle (Boc) ou benzyloxycarbonyle (Z), pour obtenir un composé de formule

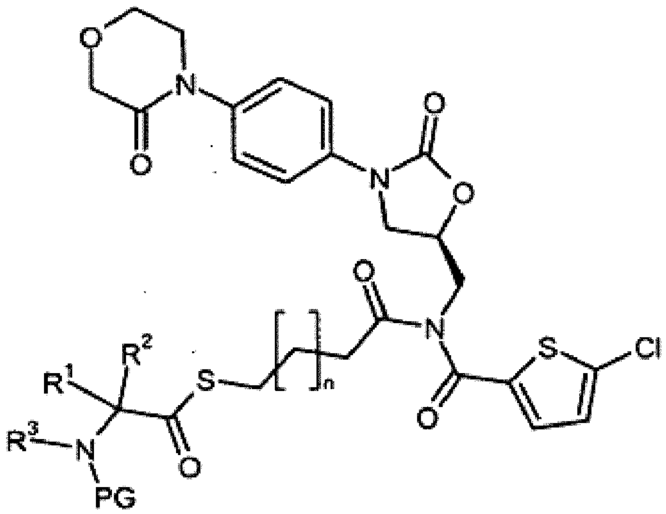

(V-A),

dans laquelle n, $R^1$, $R^2$ et $R^3$ ont la signification indiquée dans la revendication 1, et PG a la signification indiquée dans la présente revendication, et ensuite on élimine selon des méthodes usuelles le groupe protecteur PG, pour obtenir un composé de formule

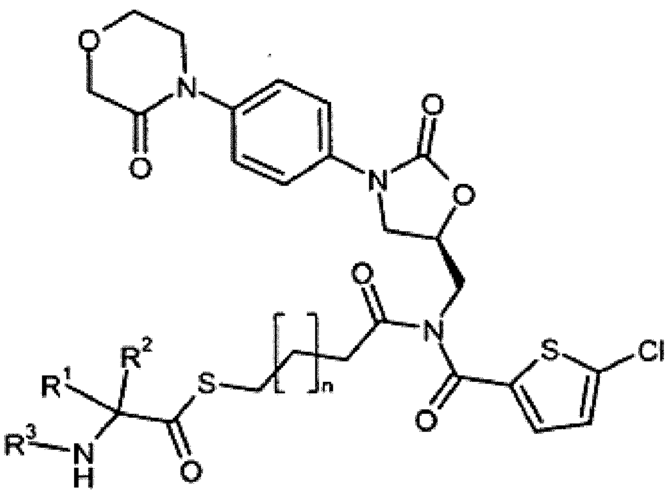

(I-A),

dans laquelle n, $R^1$, $R^2$ et $R^3$ ont la signification indiquée dans la revendication 1, ou

[B] on fait réagir le composé (A) dans un solvant inerte, en présence d'une base, avec un composé de formule

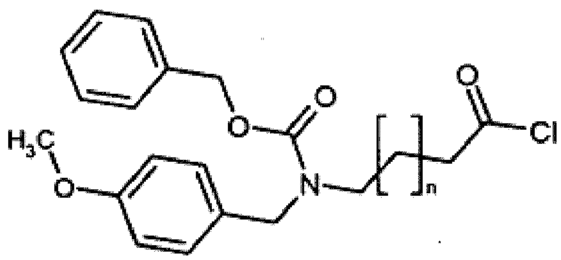

(VII),

dans laquelle n a la signification indiquée dans la revendication 1,
pour obtenir un composé de formule

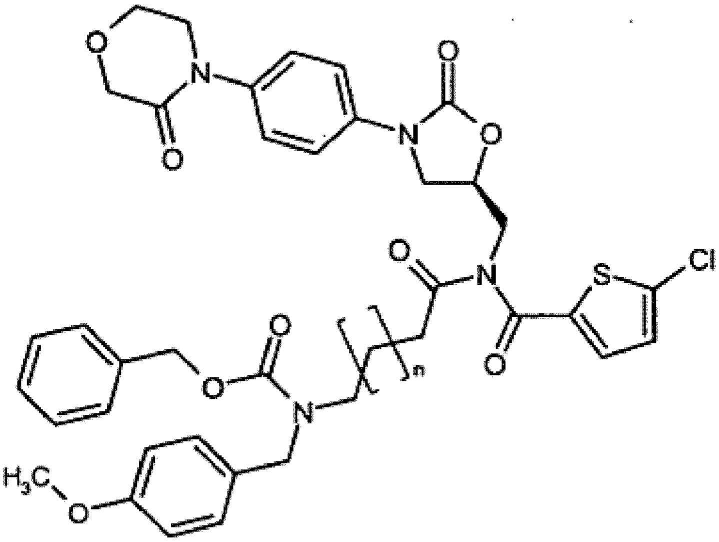

(VIII),

dans laquelle n a la signification indiquée dans la revendication 1,
ensuite on élimine selon des méthodes usuelles les groupes protecteurs pour obtenir un composé de formule

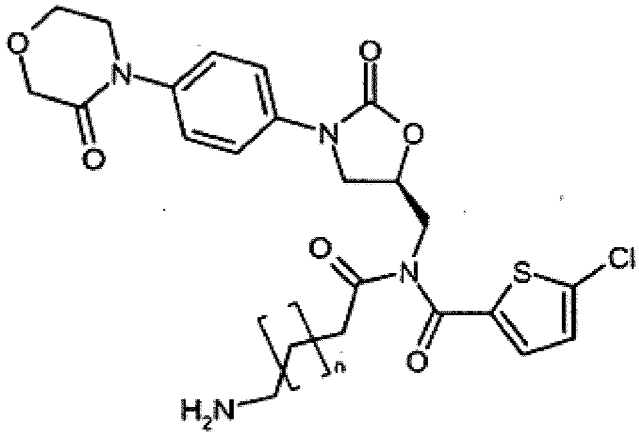

(IX),

dans laquelle n a la signification indiquée dans la revendication 1, et ensuite on le fait réagir, en présence d'une base, avec un composé de formule

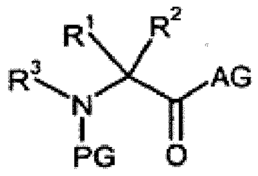

(X),

dans laquelle $R^1$, $R^2$ et $R^3$ ont la signification indiquée dans la revendication 1,

AG représente le groupe hydroxy ou un atome d'halogène, de préférence de chlore ou de brome, ou forme conjointement avec le groupe carbonyle un ester actif, de préférence un ester de N-hydroxy-succinimide, ou un anhydride mixte, de préférence un carbonate d'alkyle, de façon particulièrement préférée un carbonate d'éthyle, et

PG représente un groupe protecteur de fonction amino, comme par exemple le groupe tert-butoxycarbonyle (Boc) ou benzyloxycarbonyle (Z),

pour obtenir un composé de formule

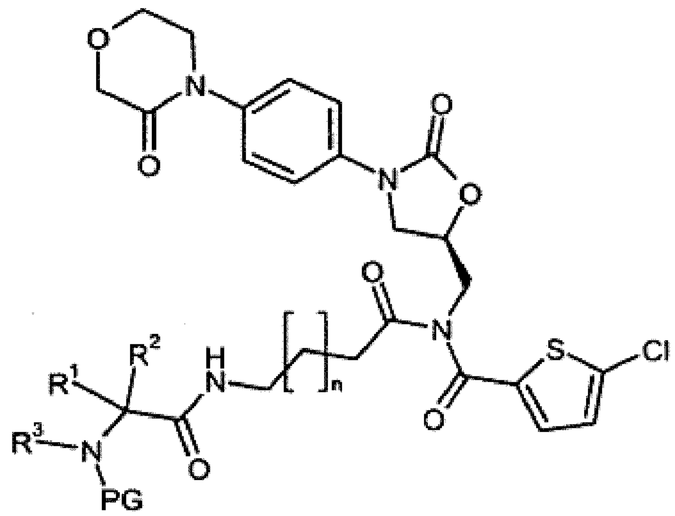

(XI)

dans laquelle n, $R^1$, $R^2$ et $R^3$ ont la signification indiquée dans la revendication 1 et

PG a la signification indiquée dans la présente revendication,

et ensuite on élimine selon des méthodes usuelles le groupe protecteur PG, pour obtenir un composé de formule

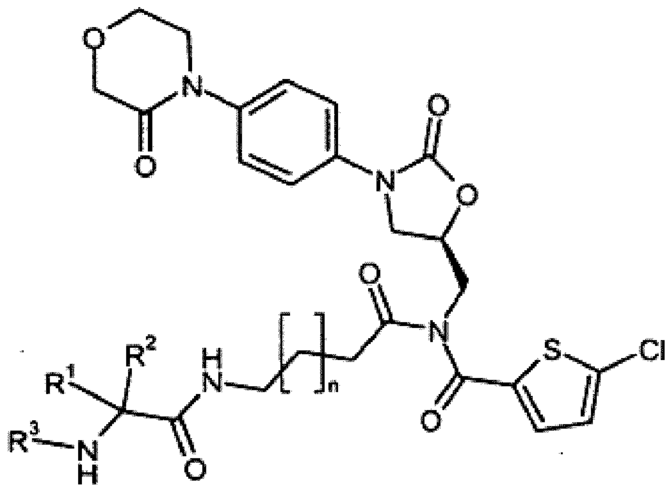

(I-B),

dans laquelle n, $R^1$, $R^2$ et $R^3$ ont la signification indiquée dans la revendication 1,
et on convertit les composés de formule (I-A) ou (I-B) qui respectivement en résultent, éventuellement avec (i) les solvants et/ou (ii) les acides correspondants, en leurs produits de solvatation, sels, et/ou produits de solvatation des sels.

**5.** Composé de formule (I), tel que défini dans l'une quelconque des revendications 1 à 3, pour le traitement et/ou la prophylaxie de maladies.

**6.** Utilisation d'un composé de formule (I), tel que défini dans l'une quelconque des revendications 1 à 3, pour la fabrication d'un médicament destiné au traitement et/ou à la prophylaxie de maladies thromboemboliques.

**7.** Médicament contenant un composé de formule (I), tel que défini dans l'une quelconque des revendications 1 à 3, éventuellement en association avec un adjuvant inerte, non toxique, pharmaceutiquement approprié.

**8.** Médicament contenant un composé de formule (I), tel que défini dans l'une quelconque des revendications 1 à 3, en association avec une autre substance active.

**9.** Médicament selon la revendication 7 ou 8, destiné au traitement ou à la prophylaxie de maladies thromboemboliques.

**10.** Médicament selon l'une quelconque des revendications 7 à 9, destiné à l'administration par voie intraveineuse.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 2005028473 A
- WO 0100622 A
- WO 03006440 A


### In der Beschreibung aufgeführte Nicht-Patentliteratur


- **P. ETTMAYER et al.** *J. Med. Chem.,* 2004, vol. 47, 2393
- Design of Prodrugs: Bioreversible derivatives for various functional groups and chemical entities. Elsevier Science Publishers B.V, 1985
- **S. ROEHRIG et al.** *J. Med. Chem.,* 2005, vol. 48, 5900
- **A. H. KAHNS et al.** *Int. J. Pharmaceutics,* 1991, vol. 71, 31-43
- **T.W. GREENE ; P.G.M. WUTS.** Protective Groups in Organic Synthesis. Wiley, 1999
- **M. BODANSZKY ; A. BODANSZKY.** The Practice of Peptide Synthesis. Springer-Verlag, 1984
- **S. ROECHRIG et al.** *J. Med. Chem,* 2005, vol. 48, 5900
- **R. MICHELOT et al.** *Bioorg. Med. Chem.,* 1996, vol. 4, 2201
- **M. JOHNSTON et al.** *J.Org.Chem.,* 1985, vol. 50, 2200
- **W.D. FULLER et al.** *J.Am.Chem.Soc.,* 1990, vol. 112, 7414
- **S. MOBASHERI et al.** *J.Org.Chem.,* 1992, vol. 57, 2755
- **P.C. WONG et al.** *Thrombosis Research,* 1996, vol. 83 (2), 117-126